# EUROPEAN PATENT APPLICATION

(11) **EP 3 363 795 A1**
(43) Date of publication of application: **22.08.2018**
(21) Application number: 16855146.3
(22) Date of filing: 11.07.2016
(51) Int. Cl.: C07D 333/54, C07D 333/56, C07D 333/64, C07D 333/70, C07D 409/04, C07D 409/10, C07D 409/12, C09K 19/12, C09K 19/14, C09K 19/18, C09K 19/20, C09K 19/30, C09K 19/32, C09K 19/34, C09K 19/54, G02F 1/13

(54) **LIQUID-CRYSTALLINE COMPOUND HAVING BENZOTHIOPHENE, LIQUID-CRYSTAL COMPOSITION, AND LIQUID-CRYSTAL DISPLAY ELEMENT**

(30) Priority: 13.10.2015 JP 2015201951
(71) Applicant: JNC Corporation, Chiyoda-ku Tokyo 100-8105 (JP); JNC Petrochemical Corporation, Tokyo 100-0004 (JP)
(72) Inventor: YANO Tomohiro, Ichihara-shi Chiba 290-8551 (JP)
(74) Representative: Thurston, Joanna
(86) International application number: PCT/JP2016/070418
(87) International publication number: WO 2017/064892

(57) **Abstract**

A compound, represented by formula (1):

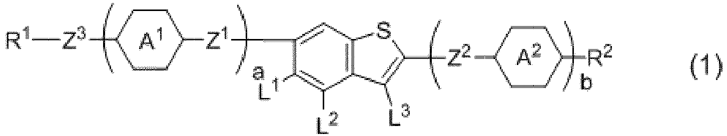

In formula (1), R¹ is hydrogen, alkyl or the like; ring A¹ and ring A² are independently 1,4-cyclohexylene, 1,4-phenylene or the like; Z¹, Z² and Z³ are independently a single bond, -C≡C- or the like; L¹, L² and L³ are independently hydrogen, chlorine or fluorine; and a and b are independently 0, 1, 2, 3 or 4, and a sum of a and b is 4 or less.

## Description

### Technical Field

The invention relates to a liquid crystal compound and a liquid crystal composition. More specifically, the invention relates to a compound having benzothiophene, a liquid crystal composition containing the compound and having a nematic phase, and a liquid crystal display device including the composition.

A liquid crystal display device has been widely utilized in a display of a personal computer, a television and so forth. The device utilizes optical anisotropy and dielectric anisotropy of a liquid crystal compound, and so forth. As an operating mode of the liquid crystal display device, such a mode exists as a phase change (PC) mode, a twisted nematic (TN) mode, a super twisted nematic (STN) mode, a bistable twisted nematic (BTN) mode, an electrically controlled birefringence (ECB) mode, an optically compensated bend (OCB) mode, an in-plane switching (IPS) mode, a vertical alignment (VA) mode, a fringe field switching (FFS) mode and a polymer sustained alignment (PSA) mode.

In such a liquid crystal display device, a liquid crystal composition having suitable physical properties is used. In order to further improve characteristics of the liquid crystal display device, the liquid crystal compound contained in the composition preferably has physical properties described in (1) to (8) below: (1) high stability to heat, light or the like, (2) a high clearing point, (3) low minimum temperature of a liquid crystal phase, (4) small viscosity (η), (5) suitable optical anisotropy (Δn), (6) large dielectric anisotropy (Δε), (7) a suitable elastic constant (K) and (8) excellent compatibility with other liquid crystal compounds.

An effect of the physical properties of the liquid crystal compound on the characteristics of the device is as described below. A compound having high stability to heat, light or the like as described in (1) increases a voltage holding ratio of the device. Thus, a service life of the device becomes longer. A compound having high clearing point as described in (2) extends a temperature range in which the device can be used. A compound having low minimum temperature of the liquid crystal phase such as a nematic phase and a smectic phase as described in (3), particular a compound having low minimum temperature of the nematic phase, also extends the temperature range in which the device can be used. A compound having small viscosity as described in (4) shortens a response time of the device.

A compound having large optical anisotropy as described in (5) improves a contrast of the device. According to a design of the device, a compound having large optical anisotropy or small optical anisotropy, more specifically, a compound having suitable optical anisotropy is required. When the response time is shortened by decreasing a cell gap of the device, the compound having large optical anisotropy is suitable. A compound having large dielectric anisotropy as described in (6) decreases threshold voltage of the device. Thus, electric power consumption of the device is reduced. On the other hand, a compound having small dielectric anisotropy shortens the response time of the device by decreasing viscosity of the composition.

With regard to (7), a compound having a large elastic constant shortens the response time of the device. A compound having a small elastic constant decreases the threshold voltage of the device. Therefore, the suitable elastic constant is required according to the characteristics to be desirably improved. A compound having excellent compatibility with other liquid crystal compounds as described in (8) is preferred. The reason is that the physical properties of the composition are adjusted by mixing liquid crystal compounds having different physical properties.

A variety of liquid crystal compounds having large dielectric anisotropy have been so far synthesized. A variety of liquid crystal compounds having large optical anisotropy have also been synthesized. The reason is that excellent physical properties that are not found in conventional compounds are expected from a new compound. The reason is that a suitable balance is expected to be obtained regarding at least two of physical properties in the composition by adding the new compound to a liquid crystal composition. In view of such a situation, with regard to the physical properties (1) to (8) described above, a compound having excellent physical properties and a suitable balance has been desired.

Several liquid crystal compounds having a benzothiophene skeleton have been so far prepared. In Patent literature No. 1 and Patent literature No. 2, compounds (A) to (D) and so forth are described. However, no characteristics are described on the compounds, and whether or not the compounds are suitable as the liquid crystal compound has been unknown.

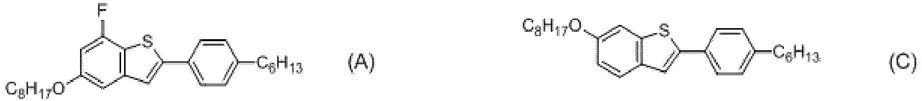

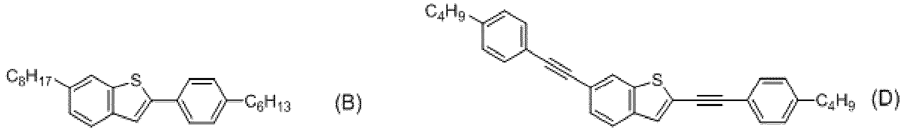

### Citation List

### Patent Literature

Patent literature No. 1: JP 2000-328062 A.
Patent literature No. 2: WO 2012-126570 A.

### Summary of Invention

### Technical Problem

A first object is to provide a liquid crystal compound satisfying at least one of physical properties such as high stability to heat or light, a high clearing point (or high maximum temperature of a nematic phase), low minimum temperature of a liquid crystal phase, small viscosity, suitable optical anisotropy, large positive dielectric anisotropy, a suitable elastic constant and excellent compatibility with other liquid crystal compounds. A second object is to provide a liquid crystal composition that contains the compound and satisfies at least one of physical properties such as high stability to heat or light, high maximum temperature of the nematic phase, low minimum temperature of the nematic phase, small viscosity, suitable optical anisotropy, large dielectric anisotropy, large specific resistance and a suitable elastic constant. The object is to provide a liquid crystal composition having a suitable balance regarding at least two of the physical properties. A third object is to provide a liquid crystal display device including the composition and having a wide temperature range in which the device can be used, a short response time, a large voltage holding ratio, low threshold voltage, a large contrast ratio and a long service life.

### Solution to Problem

The invention concerns a compound represented by formula (1), a liquid crystal composition containing the compound, and a liquid crystal display device including the composition: 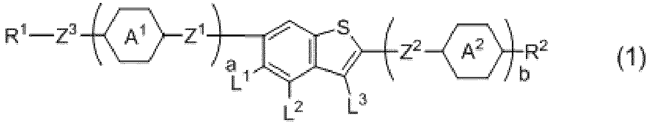 wherein, in formula (1),
R¹ and R² are independently hydrogen or alkyl having 1 to 20 carbons, and in the alkyl, at least one piece of -CH₂- may be replaced by -O- or -S-, and at least one piece of - (CH₂)₂- may be replaced by -CH=CH-, and in the groups, at least one hydrogen may be replaced by halogen;
ring A¹ and ring A² are independently 1,4-cyclohexylene, 1,4-cyclohexenylene, 1,4-phenylene, tetrahydropyran-2,5-diyl, 1,3-dioxane-2,5-diyl, pyrimidine-2,5-diyl, 2,6,7-trioxabicyclo[2.2.2]octane-1,4-diyl, naphthalene-2,6-diyl or pyridine-2,5-diyl, and at least one hydrogen on the rings may be replaced by halogen;
a and b are independently 0, 1, 2, 3 or 4, and a sum of a and b is 4 or less, and when a or b is 2 or more, two of ring A¹, two of ring A², two pieces of Z¹ or two pieces of Z² may be identical or different;
Z¹ and Z² are independently a single bond or alkylene having 1 to 4 carbons, and in the alkylene, at least one piece of -CH₂- may be replaced by -O- or -COO-, and at least one piece of -(CH₂)₂- may be replaced by -CH=CH- or -C=C-, and in the groups, at least one hydrogen may be replaced by halogen, and a case where Z¹ and Z² are simultaneously either -CH=CH- or -C=C- is excluded, and when a is 0 and b is 1, Z² is alkylene having 1 to 4 carbons, and in the alkylene, at least one piece of -CH₂- may be replaced by -O- or -COO-, at least one piece of - (CH₂)₂- may be replaced by -CH=CH- or -C≡C-, and at least one hydrogen may be replaced by halogen; and Z³ is -O- or a single bond; and
L¹, L² and L³ are independently hydrogen, fluorine or chlorine.

### Advantageous Effects of Invention

A first advantage is to provide a liquid crystal compound satisfying at least one of physical properties such as high stability to heat or light, a high clearing point (or high maximum temperature of a nematic phase), low minimum temperature of a liquid crystal phase, small viscosity, suitable optical anisotropy, large dielectric anisotropy, a suitable elastic constant and excellent compatibility with other liquid crystal compounds. A second advantage is to provide a liquid crystal composition that contains the compound and satisfies at least one of physical properties such as high stability to heat or light, high maximum temperature of the nematic phase, low minimum temperature of the nematic phase, small viscosity, suitable optical anisotropy, large dielectric anisotropy, large specific resistance and a suitable elastic constant. The advantage is to provide a liquid crystal composition having a suitable balance regarding at least two of the physical properties. A third advantage is to provide a liquid crystal display device including the composition and having a wide temperature range in which the device can be used, a short response time, a large voltage holding ratio, low threshold voltage, a large contrast ratio and a long service life.

### Description of Embodiments

Usage of terms herein is as described below. Terms "liquid crystal compound," "liquid crystal composition" and "liquid crystal display device" may be occasionally abbreviated as "compound," "composition" and "device," respectively. "Liquid crystal compound" is a generic term for a compound having a liquid crystal phase such as a nematic phase and a smectic phase, and a compound having no liquid crystal phase but to be added for the purpose of adjusting physical properties of a composition such as maximum temperature, minimum temperature, viscosity and dielectric anisotropy. The compound has a six-membered ring such as 1,4-cyclohexylene and 1,4-phenylene, and has rod-like molecular structure. "Liquid crystal display device" is a generic term for a liquid crystal display panel and a liquid crystal display module. "Polymerizable compound" is a compound to be added for the purpose of forming a polymer in the composition.

The liquid crystal composition is prepared by mixing a plurality of liquid crystal compounds. A proportion (content) of the liquid crystal compounds is expressed in terms of weight percent (% by weight) based on the weight of the liquid crystal composition. An additive such as the polymerizable compound, a polymerization initiator, a polymerization inhibitor, an optically active compound, an antioxidant, an ultraviolet light absorber, a light stabilizer, a heat stabilizer, a dye and an antifoaming agent is added to the composition when necessary. A proportion (amount of addition) of the additive is expressed in terms of weight percent (% by weight) based on the weight of the liquid crystal composition in a manner similar to the proportion of the liquid crystal compound. Weight parts per million (ppm) may be occasionally used. A proportion of the polymerization initiator and the polymerization inhibitor is exceptionally expressed based on the weight of the polymerizable compound.

"Clearing point" is a transition temperature between the liquid crystal phase and an isotropic phase in the liquid crystal compound. "Minimum temperature of the liquid crystal phase" is a transition temperature between a solid and the liquid crystal phase (the smectic phase, the nematic phase or the like) in the liquid crystal compound. "Maximum temperature of the nematic phase" is a transition temperature between the nematic phase and the isotropic phase in a mixture of the liquid crystal compound and a base liquid crystal or in the liquid crystal composition, and may be occasionally abbreviated as "maximum temperature." "Minimum temperature of the nematic phase" may be occasionally abbreviated as "minimum temperature." An expression "increase the dielectric anisotropy" means that a value of dielectric anisotropy positively increases in a composition having positive dielectric anisotropy, and the value of dielectric anisotropy negatively increases in a composition having negative dielectric anisotropy.

A compound represented by formula (1) may be occasionally abbreviated as compound (1). At least one compound selected from the group of compounds represented by formula (1) may be occasionally abbreviated as compound (1). "Compound (1)" means one compound, a mixture of two compounds or a mixture of three or more compounds represented by formula (1). A same rule applies also to any other compound represented by any other formula. In formulas (1) to (15), a symbol of A¹ or the like surrounded by a hexagonal shape corresponds to ring A¹, respectively. The hexagonal shape represents a six-membered ring such as cyclohexane or benzene. The hexagonal shape may occasionally represents a fused ring such as naphthalene or a bridged ring such as adamantane.

A symbol of terminal group R¹ is used in a plurality of compounds in chemical formulas of component compounds. In the compounds, two groups represented by two pieces of arbitrary R¹ may be identical or different. For example, in one case, R¹ of compound (1-1) is ethyl and R¹ of compound (1-2) is ethyl. In another case, R¹ of compound (1-1) is ethyl and R¹ of compound (1-2) is propyl. A same rule applies also to a symbol of R¹¹, Z¹¹ or the like. In compound (8), when i is 2, two of ring D¹ exists. In the compound, two groups represented by two of ring D¹ may be identical or different. A same rule applies also to two of arbitrary ring D¹ when i is larger than 2. A same rule applies also to other symbols.

An expression "at least one piece of 'A'" means that the number of 'A' is arbitrary. An expression "at least one piece of 'A' may be replaced by 'B'" means that, when the number of 'A' is 1, a position of 'A' is arbitrary, and also when the number of 'A' is 2 or more, positions thereof can be selected without restriction. A same rule applies also to an expression "at least one piece of 'A' is replaced by 'B'." An expression "at least one piece of A may be replaced by B, C or D" means including a case where arbitrary A is replaced by B, a case where arbitrary A is replaced by C, and a case where arbitrary A is replaced by D, and also a case where a plurality of pieces of A are replaced by at least two of B, C and D. For example, "alkyl in which at least one piece of -CH₂- may be replaced by -O- or -CH=CH-" includes alkyl, alkenyl, alkoxy, alkoxyalkyl, alkoxyalkenyl and alkenyloxyalkyl. In addition, a case where two pieces of consecutive -CH₂- are replaced by -O- to form -O-O- is not preferred.

Halogen is fluorine, chlorine, bromine and iodine. Preferred halogen is fluorine and chlorine. Further preferred halogen is fluorine. Alkyl is straight-chain alkyl or branched-chain alkyl, but includes no cyclic alkyl. In general, straight-chain alkyl is preferred to branched-chain alkyl. A same rule applies also to a terminal group such as alkoxy and alkenyl. With regard to a configuration of 1,4-cyclohexylene, trans is preferred to cis for increasing the maximum temperature. Then, 2-fluoro-1,4-phenylene means two divalent groups described below. In a chemical formula, fluorine may be leftward (L) or rightward (R). A same rule applies also to an asymmetrical divalent group formed by removing two hydrogens from a ring, such as tetrahydropyran-2,5-diyl.

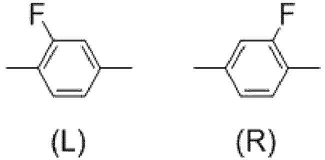

The invention includes items described below.
Item 1. A compound, represented by formula (1): 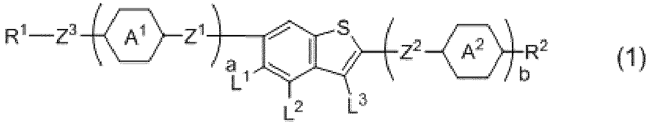 wherein, in formula (1),
   R¹ and R² are independently hydrogen or alkyl having 1 to 20 carbons, and in the alkyl, at least one piece of -CH₂- may be replaced by -O- or -S-, and at least one piece of - (CH₂)₂- may be replaced by -CH=CH-, and in the groups, at least one hydrogen may be replaced by halogen;
   ring A¹ and ring A² are independently 1,4-cyclohexylene, 1,4-cyclohexenylene, 1,4-phenylene, tetrahydropyran-2,5-diyl, 1,3-dioxane-2,5-diyl, pyrimidine-2,5-diyl, 2,6,7-trioxabicyclo[2.2.2]octane-1,4-diyl, naphthalene-2,6-diyl or pyridine-2,5-diyl, and at least one hydrogen on the rings may be replaced by halogen;
   a and b are independently 0, 1, 2, 3 or 4, and a sum of a and b is 4 or less, and when a or b is 2 or more, two of ring A¹, two of ring A², two pieces of Z¹ or two pieces of Z² may be identical or different;
   Z¹ and Z² are independently a single bond or alkylene having 1 to 4 carbons, and in the alkylene, at least one piece of -CH₂- may be replaced by -O- or -COO-, and at least one piece of -(CH₂)₂- may be replaced by -CH=CH- or -C=C-, and in the groups, at least one hydrogen may be replaced by halogen, and a case where Z¹ and Z² are simultaneously either -CH=CH- or -C=C- is excluded, and when a is 0 and b is 1, Z² is alkylene having 1 to 4 carbons, and in the alkylene, at least one piece of -CH₂- may be replaced by -O- or -COO-, at least one piece of - (CH₂)₂- may be replaced by -CH=CH- or -C≡C-, and at least one hydrogen may be replaced by halogen; and Z³ is -O- or a single bond; and
   L¹, L² and L³ are independently hydrogen, fluorine or chlorine.
Item 2. The compound according to item 1, wherein, in formula (1),
   R¹ and R² are independently hydrogen, alkyl having 1 to 10 carbons, alkoxy having 1 to 9 carbons, alkenyl having 2 to 10 carbons, or alkyl having 1 to 10 carbons in which at least one hydrogen is replaced by fluorine;
   ring A¹ and ring A² are independently 1,4-cyclohexylene, 1,4-cyclohexenylene, 1,4-phenylene in which at least one hydrogen may be replaced by halogen, or tetrahydropyran-2,5-diyl;
   Z¹ and Z² are independently a single bond, -(CH₂)₂-, -CH=CH-, -CF=CF-, -C≡C-, -COO-, -OCO-, -CF₂O-, -OCF₂-, -CH₂O-, -OCH₂-, - (CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or -(CH₂)₂-CH=CH-, and when a is 0 and b is 1, Z² is -(CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or - (CH₂)₂-CH=CH-;
   a and b are independently 0, 1, 2, 3 or 4, and a sum of a and b is 4 or less; and
   L¹, L² and L³ are independently hydrogen, fluorine or chlorine.
Item 3. The compound according to item 1 or 2, wherein, in formula (1),
   a sum of a and b is 0, 1, 2 or 3;
   R¹ and R² are independently hydrogen, alkyl having 1 to 10 carbons, alkoxy having 1 to 9 carbons, alkenyl having 2 to 10 carbons or fluorinated alkyl having 1 to 10 carbons;
   ring A¹ and ring A² are independently 1,4-cyclohexylene, 1,4-cyclohexenylene, 1,4-phenylene in which at least one hydrogen may be replaced by halogen, or tetrahydropyran-2,5-diyl;
   Z¹ and Z² are independently a single bond, -(CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or - (CH₂)₂-CH=CH-, and when a is 0 and b is 1, Z² is -(CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or - (CH₂)₂-CH=CH-; and
   L¹, L² and L³ are independently hydrogen, fluorine or chlorine.
Item 4. The compound according to any one of items 1 to 3, represented by any one of formulas (1-1) to (1-10): 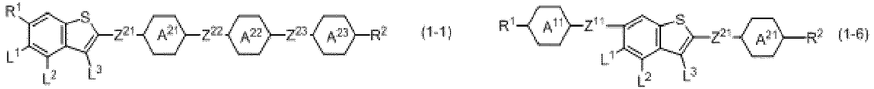 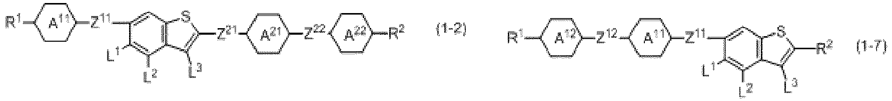 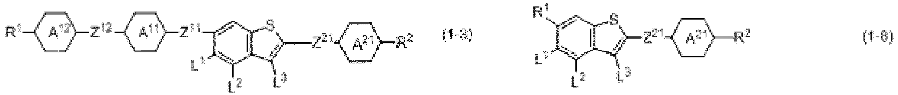 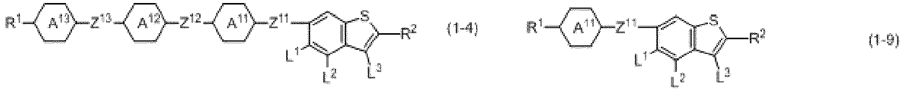 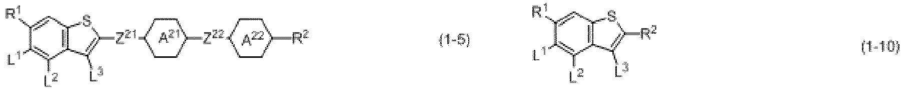 wherein, in formulas (1-1) to (1-7) and (1-9) to (1-10),
   R¹ and R² are independently hydrogen, alkyl having 1 to 10 carbons, alkoxy having 1 to 9 carbons, alkenyl having 2 to 10 carbons, or alkyl having 1 to 10 carbons in which at least one hydrogen is replaced by fluorine;
   ring A¹¹, ring A¹², ring A¹³, ring A²¹, ring A²² and ring A²³ are independently1,4-cyclohexylene, 1,4-cyclohexenylene, 1,4-phenylene, 1,4-phenylene in which at least one hydrogen is replaced by halogen, or tetrahydropyran-2,5-diyl;
   Z¹¹, Z¹², Z¹³, Z²¹, Z²² and Z²³ are independently a single bond, -(CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or -(CH₂)₂-CH=CH-; and
   L¹, L² and L³ are independently hydrogen, fluorine or chlorine; and in formula (1-8),
   R¹ and R² are independently hydrogen, alkyl having 1 to 10 carbons, alkoxy having 1 to 9 carbons, alkenyl having 2 to 10 carbons, or alkyl having 1 to 10 carbons in which at least one hydrogen is replaced by fluorine;
   ring A²¹ is 1,4-cyclohexylene, 1,4-cyclohexenylene, 1,4-phenylene, 1,4-phenylene in which at least one hydrogen is replaced by halogen, or tetrahydropyran-2,5-diyl;
   Z²¹ is independently -(CH₂)₂-, -CH=CH-, -CF=CF-, -C=C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or -(CH₂)₂-CH=CH-; and
   L¹, L² and L³ are independently hydrogen, fluorine or chlorine.
Item 5. The compound according to item 4, represented by at least one of formulas (1-1) to (1-10), wherein, in formulas (1-1) to (1-7) and (1-9) to (1-10),
   R¹ and R² are independently hydrogen, fluorine, alkyl having 1 to 10 carbons, alkoxy having 1 to 9 carbons, alkenyl having 2 to 10 carbons or fluorinated alkyl having 1 to 10 carbons;
   ring A¹¹, ring A¹², ring A¹³, ring A²¹, ring A²² and ring A²³ are independently 1,4-cyclohexylene, 1,4-cyclohexenylene, 1,4-phenylene in which at least one hydrogen may be replaced by halogen, or tetrahydropyran-2,5-diyl;
   Z¹¹, Z¹², Z¹³, Z²¹, Z²² and Z²³ are independently a single bond, -(CH₂)₂-, -CH=CH-, -C=C-, -CF₂O-, -OCF₂-, -CH₂O- or -OCH₂-; and
   L¹, L² and L³ are independently hydrogen, fluorine or chlorine; and in formula (1-8),
   R¹ and R² are independently hydrogen, fluorine, alkyl having 1 to 10 carbons, alkoxy having 1 to 9 carbons, alkenyl having 2 to 10 carbons or fluorinated alkyl having 1 to 10 carbons;
   ring A¹¹, ring A¹², ring A¹³, ring A²¹, ring A²² and ring A²³ are independently 1,4-cyclohexylene, 1,4-cyclohexenylene, 1,4-phenylene in which at least one hydrogen may be replaced by halogen, or tetrahydropyran-2,5-diyl;
   Z¹¹, Z¹², Z¹³, Z²¹, Z²² and Z²³ are independently -(CH₂)₂-, -CH=CH-, -C≡C-, -CF₂O-, -OCF₂-, -CH₂O- or -OCH₂-; and
   L¹, L² and L³ are independently hydrogen, fluorine or chlorine.
Item 6. The compound according to item 6, represented by any one of formulas (1-1), (1-5) and (1-10) : 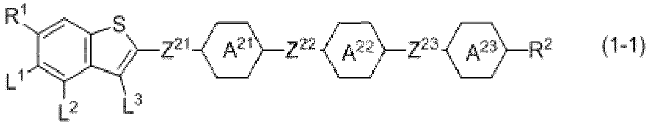 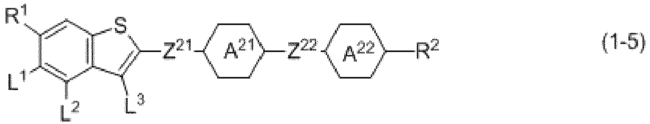 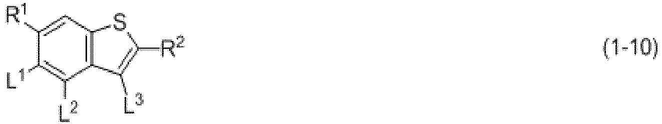 wherein, in formulas (1-1), (1-5) and (1-10),
   R¹ and R² are independently hydrogen, fluorine, alkyl having 1 to 10 carbons, alkoxy having 1 to 9 carbons, alkenyl having 2 to 10 carbons, or alkyl having 1 to 10 carbons in which at least one hydrogen is replaced by fluorine;
   ring A²¹, ring A²² and ring A²³ are independently 1, 4-cyclohexylene, or 1,4-phenylene in which at least one hydrogen may be replaced by halogen;
   Z²¹, Z²² and Z²³ are independently a single bond, -(CH₂)₂-, -CH=CH-, -C≡C-, -CH₂O- or -OCH₂-; and
   L¹, L² and L³ are independently hydrogen or fluorine.
Item 7. The compound according to item 5, represented by formula (1-8) : 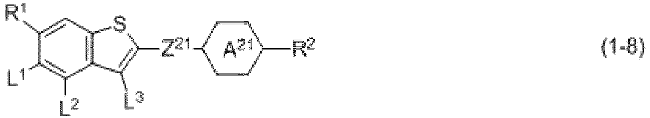 wherein, in formula (1-8),
   R¹ and R² are independently hydrogen, fluorine, alkyl having 1 to 10 carbons, alkoxy having 1 to 9 carbons, alkenyl having 2 to 10 carbons, or alkyl having 1 to 10 carbons in which at least one hydrogen is replaced by fluorine;
   ring A²¹ is 1,4-cyclohexylene, or 1,4-phenylene in which at least one hydrogen may be replaced by halogen;
   Z²¹ is a single bond, -(CH₂)₂-, -CH=CH-, -C≡C-, -CH₂O- or -OCH₂-; and
   L¹, L² and L³ are independently hydrogen or fluorine.
Item 8. The compound according to item 5, represented by any one of formulas (1-4), (1-7) and (1-9) : 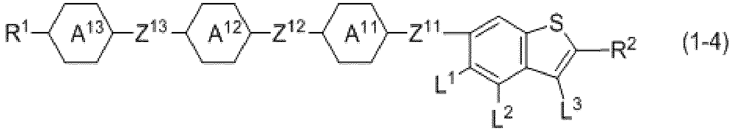 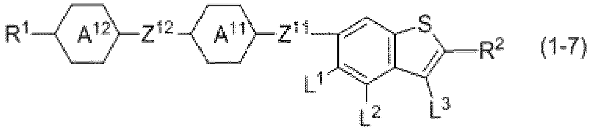 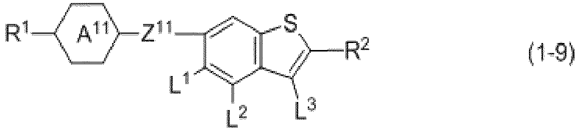 wherein, in formulas (1-4), (1-7) and (1-9),
   R¹ and R² are independently hydrogen, fluorine, alkyl having 1 to 10 carbons, alkoxy having 1 to 9 carbons, alkenyl having 2 to 10 carbons, or alkyl having 1 to 10 carbons in which at least one hydrogen is replaced by fluorine;
   ring A¹¹, ring A¹² and ring A¹³ are independently 1, 4-cyclohexylene, or 1,4-phenylene in which at least one hydrogen may be replaced by halogen;
   Z¹¹, Z¹² and Z¹³ are independently a single bond, -(CH₂)₂-, -CH=CH-, -C≡C-, -CH₂O- or -OCH₂-; and
   L¹, L² and L³ are independently hydrogen or fluorine.
Item 9. A liquid crystal composition, containing at least one compound according to any one of items 1 to 8.
Item 10. The liquid crystal composition according to item 9, further containing at least one compound selected from the group of compounds represented by formulas (2) to (4): 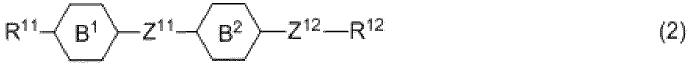 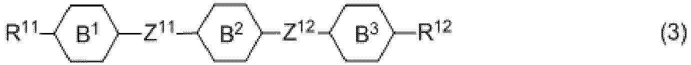 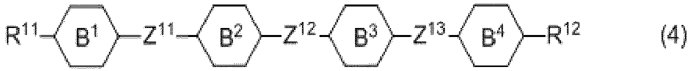 wherein, in formulas (2) to (4),
   R¹¹ and R¹² are independently alkyl having 1 to 10 carbons or alkenyl having 2 to 10 carbons, and in the alkyl or the alkenyl, at least one piece of -CH₂- may be replaced by -O-, and at least one hydrogen may be replaced by fluorine;
   ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
   Z¹¹, Z¹² and Z¹³ are independently a single bond, -CH₂CH₂-, -CH=CH-, -C≡C- or -COO-.
Item 11. The liquid crystal composition according to item 9 or 10, further containing at least one compound selected from the group of compounds represented by formulas (5) to (7) : 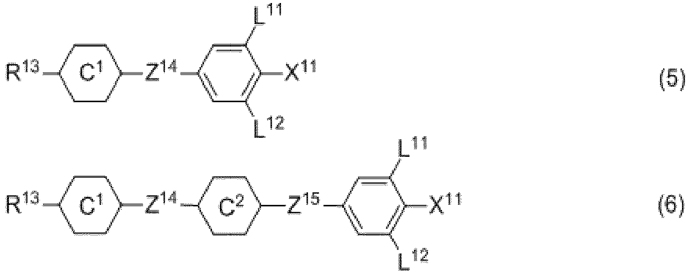 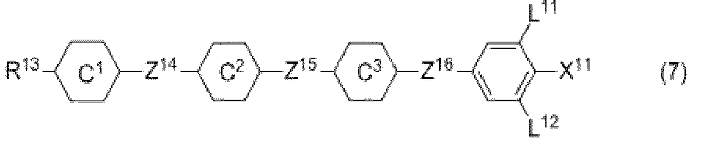 wherein, in formulas (5) to (7),
   R¹³ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine;
   X¹¹ is fluorine, chlorine, -OCF₃, -OCHF₂, -CF₃, -CHF₂, -CH₂F, -OCF₂CHF₂ or -OCF₂CHFCF₃;
   ring C¹, ring C² and ring C³ are independently 1,4-cyclohexylene, 1, 4-phenylene in which at least one hydrogen may be replaced by fluorine, tetrahydropyran-2,5-diyl, 1,3-dioxane-2,5-diyl, or pyrimidine-2,5-diyl;
   Z¹⁴, Z¹⁵ and Z¹⁶ are independently a single bond, -CH₂CH₂-, -CH=CH-, -C=C-, -COO-, -CF₂O-, -OCF₂-, -CH₂O- or -(CH₂)₄-; and
   L¹¹ and L¹² are independently hydrogen or fluorine.
Item 12. The liquid crystal composition according to any one of items 9 to 11, further containing at least one compound selected from the group of compounds represented by formula (8): 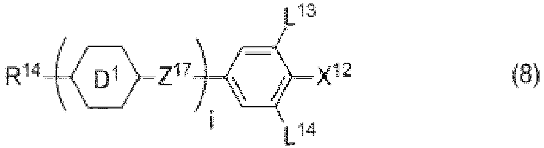 wherein, in formula (8),
   R¹⁴ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine;
   X¹² is -C=N or -C=C-C=N;
   ring D¹ is 1,4-cyclohexylene, 1,4-phenylene in which at least one hydrogen may be replaced by fluorine, tetrahydropyran-2,5-diyl, 1,3-dioxane-2,5-diyl, or pyrimidine-2,5-diyl;
   Z¹⁷ is a single bond, -CH₂CH₂-, -C≡C-, -COO-, -CF₂O-, -OCF₂- or -CH₂O- ;
   L¹³ and L¹⁴ are independently hydrogen or fluorine; and
   i is 1, 2, 3 or 4.
Item 13. The liquid crystal composition according to any one of items 9 to 12, further containing at least one compound selected from the group of compounds represented by formulas (9) to (15): 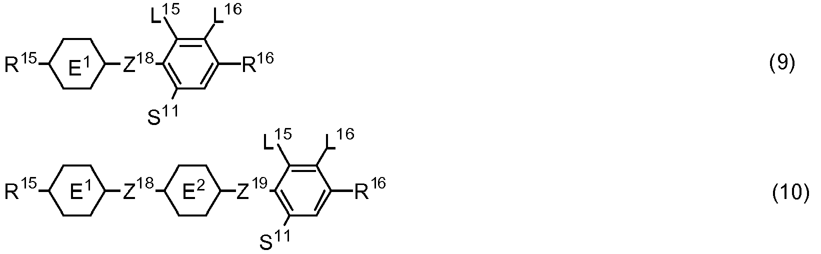 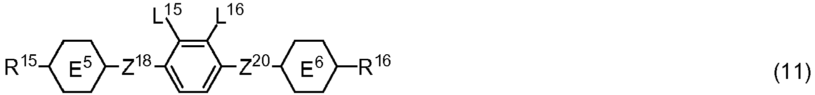 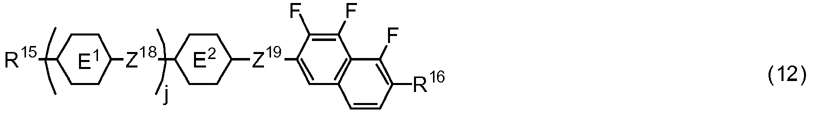 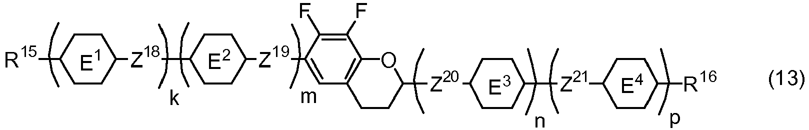 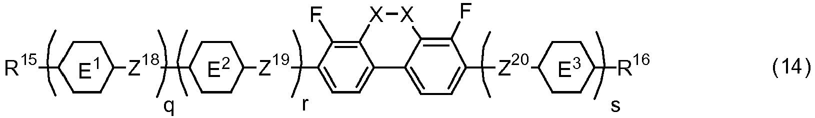 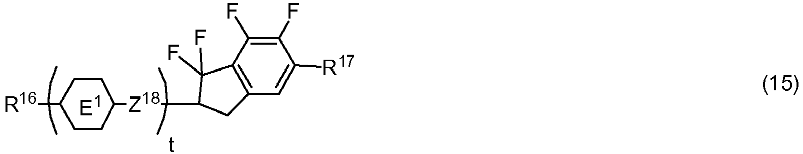 wherein, in formulas (9) to (15),
   R¹⁵ and R¹⁶ are independently alkyl having 1 to 10 carbons or alkenyl having 2 to 10 carbons, and in the alkyl and the alkenyl, at least one piece of -CH₂- may be replaced by -O-, and at least one hydrogen may be replaced by fluorine;
   R¹⁷ is hydrogen, fluorine, alkyl having 1 to 10 carbons or alkenyl having 2 to 10 carbons, and in the alkyl and the alkenyl, at least one piece of -CH₂- may be replaced by -O-, and at least one hydrogen may be replaced by fluorine;
   ring E¹, ring E², ring E³ and ring E⁴ are independently 1,4-cyclohexylene, 1,4-cyclohexenylene, 1,4-phenylene in which at least one hydrogen may be replaced by fluorine, tetrahydropyran-2,5-diyl, or decahydronaphthalene-2,6-diyl; and ring E⁵ and ring E⁶ are independently 1,4-cyclohexylene, 1,4-cyclohexenylene, 1,4-phenylene, tetrahydropyran-2,5-diyl or decahydronaphthalene-2,6-diyl;
   Z¹⁸, Z¹⁹, Z²⁰ and Z²¹ are independently a single bond, -CH₂CH₂-, -COO-, -CH₂O-, -OCF₂- or -OCF₂CH₂CH₂-;
   L¹⁵ and L¹⁶ are independently fluorine or chlorine;
   S¹¹ is hydrogen or methyl;
   X is -CHF- or -CF₂-; and
   j, k, m, n, p, q, r and s are independently 0 or 1, a sum of k, m, n and p is 1 or 2, a sum of q, r and s is 0, 1, 2 or 3, and t is 1, 2 or 3.
Item 14. The liquid crystal composition according to any one of items 9 to 13, further containing at least one of a polymerizable compound, a polymerization initiator, a polymerization inhibitor, an optically active compound, an antioxidant, an ultraviolet light absorber, a light stabilizer, a heat stabilizer, a dye and an antifoaming agent.
Item 15. A liquid crystal display device, including the liquid crystal composition according to any one of items 9 to 14.
Item 16. The liquid crystal display device according to item 15, wherein the liquid crystal composition according to any one of items 9 to 14 is encapsulated.
Item 17. The liquid crystal display device according to item 15, wherein the liquid crystal composition according to any one of items 9 to 14 is used for switching between 2D and 3D.

### 1. Aspect of compound (1)

Compound (1) of the invention has 1-benzothiophene ring structure. Preferred examples of compound (1) will be described. Preferred examples of a terminal group, ring structure, a bonding group and a substituent in compound (1) apply also to a subordinate formula of formula (1) for compound (1) such as compound (1-1) and compound (1-2) . In compound (1), physical properties can be arbitrarily adjusted by suitably combining kinds of the groups. Compound (1) may contain a larger amount of isotope such as ²H (deuterium) and ¹³C than the amount of natural abundance because no significant difference exists in the physical properties of the compound. In addition, symbols in compound (1) are defined according to item 1.

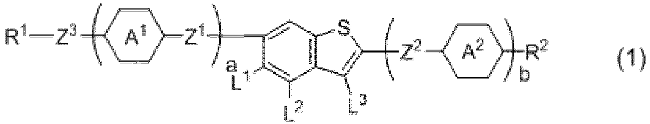

R¹ is hydrogen, fluorine or alkyl having 1 to 20 carbons, and in the alkyl, at least one piece of -CH₂- may be replaced by -O- or -S-, and at least one piece of -CH₂CH₂- may be replaced by -CH=CH-, and in the groups, at least one hydrogen may be replaced by fluorine.

Examples of R¹ include hydrogen, alkyl, alkoxy, alkoxyalkyl, alkoxyalkoxy, alkenyl, alkenyloxy, alkenyloxyalkyl, alkoxyalkenyl, alkylthio, alkylthioalkyl, alkenylthio, alkenylthioalkyl and alkylthioalkenyl. Preferred R¹ is alkyl, alkoxy, alkoxyalkyl, alkoxyalkoxy, alkenyl, alkenyloxy, alkenyloxyalkyl or alkoxyalkenyl. Further preferred R¹ is alkyl, alkoxy, alkoxyalkyl, alkenyl or alkenyloxy. Particularly preferred R¹ is alkyl or alkenyl. Most preferred R¹ is alkyl.

Preferred alkyl is -CH₃, -C₂H₅, -C₃H₇, -C₄H₉, -C₅H₁₁, -C₆H₁₃ or -C₇H₁₅.

Preferred alkoxy is -OCH₃, -OC₂H₅, -OC₃H₇, -OC₄H₉, -OC₅H₁₁, -OC₆H₁₃ or -OC₇H₁₅.

Preferred alkoxyalkyl is -CH₂OCH₃, -CH₂OC₂H₅, -CH₂OC₃H₇, -(CH₂)₂-OCH₃, -(CH₂)₂-OC₂H₅, -(CH₂)₂-OC₃H₇, -(CH₂)₃-OCH₃, -(CH₂)₄-OCH₃ or -(CH₂)₅-OCH₃.

Preferred alkenyl is -CH=CH₂, -CH=CHCH₃, -CH₂CH=CH₂, -CH=CHC₂H₅, -CH₂CH=CHCH₃, -(CH₂)₂-CH=CH₂, -CH=CHC₃H₇, -CH₂CH=CHC₂H₅, -(CH₂)₂-CH=CHCH₃ or -(CH₂)₃-CH=CH₂.

Preferred alkenyloxy is -OCH₂CH=CH₂, -OCH₂CH=CHCH₃ or -OCH₂CH=CHC₂H₅.

□ Preferred R¹ is hydrogen, -CH₃, -C₂H₅, -C₃H₇, -C₄H₉, -C₅H₁₁, -C₆H₁₃, -OCH₃, -OC₂H₅, -OC₃H₇, -OC₄H₉, -OC₅H₁₁, -CH₂OCH₃, -CH=CH₂, -CH=CHCH₃, -(CH₂)₂-CH=CH₂, -CH₂CH=CHC₂H₅, -(CH₂)₂-CH=CHCH₃, -OCH₂CH=CH₂, -OCH₂CH=CHCH₃ or -OCH₂CH=CHC₂H₅. Further preferred R¹ is -C₃H₇, -C₄H₉, -C₅H₁₁, -C₆H₁₃, -(CH₂)₂-CH=CH₂ or -(CH₂)₂-CH=CHCH₃.

When R¹ has the straight chain, a temperature range of the liquid crystal phase is wide and the viscosity is small. When R¹ has the branched chain, compatibility with other liquid crystal compounds is good. A compound in which R¹ is optically active is useful as a chiral dopant. A reverse twisted domain to be generated in the liquid crystal display device can be prevented by adding the compound to the composition. A compound in which R¹ is not optically active is useful as a component of the composition. When R¹ is alkenyl, a preferred configuration depends on a position of a double bond. An alkenyl compound having the preferred configuration has small viscosity, high maximum temperature or the wide temperature range of the liquid crystal phase.

A preferred configuration of -CH=CH- in the alkenyl depends on a position of a double bond. A trans configuration is preferred in alkenyl having the double bond in an odd-numbered position, such as -CH=CHCH₃, -CH=CHC₂H₅, -CH=CHC₃H₇, -CH=CHC₄H₉, -C₂H₄CH=CHCH₃ and -C₂H₄CH=CHC₂H₅. A cis configuration is preferred in alkenyl having the double bond in an even-numbered position, such as -CH₂CH=CHCH₃, -CH₂CH=CHC₂H₅ and -CH₂CH=CHC₃H₇. The alkenyl compound having the preferred configuration has a high clearing point or the wide temperature range of the liquid crystal phase. A detailed description is found in Mol. Cryst. Liq. Cryst., 1985, 131, 109 and Mol. Cryst. Liq. Cryst., 1985, 131, 327.

In formula (1), ring A¹ and ring A² are independently 1,4-cyclohexylene, 1,4-cyclohexenylene, 1,4-phenylene, tetrahydropyran-2,5-diyl, 1,3-dioxane-2,5-diyl, pyrimidine-2,5-diyl, 2,6,7-trioxabicyclo[2.2.2]octane-1,4-diyl, naphthalene-2,6-diyl or pyridine-2,5-diyl, and at least one hydrogen on the rings may be replaced by halogen.

Preferred ring A¹ or ring A² is 1,4-cyclohexylene, 1,4-phenylene, 2-fluoro-1,4-phenylene, 2,3-difluoro-1,4-phenylene, 2,6-difluoro-1,4-phenylene, 2,3,5-trifluoro-1,4-phenylene, tetrahydropyran-2,5-diyl, 1,3-dioxane-2,5-diyl or pyrimidine-2,5-diyl. Further preferred ring A¹ or ring A² is 1,4-cyclohexylene, 1,4-phenylene, 2-fluoro-1,4-phenylene, 2,6-difluoro-1,4-phenylene or tetrahydropyran-2,5-diyl. Particularly preferred ring A¹ or ring A² is 1,4-cyclohexylene, 1,4-phenylene, 2-fluoro-1,4-phenylene or 2,6-difluoro-1,4-phenylene.

When ring A¹ or ring A² is 1,4-cyclohexylene, the clearing point is high and the viscosity is small. When ring A¹ or ring A² is 1,4-phenylene, or 1,4-phenylene in which at least one hydrogen is replaced by fluorine, the optical anisotropy is large and an orientational order parameter is comparatively large. When ring A¹ or ring A² is 1,4-phenylene in which at least one hydrogen is replaced by fluorine, the dielectric anisotropy is large.

Z¹ and Z² are independently a single bond or alkylene having 1 to 4 carbons, and in the alkylene, at least one piece of -CH₂- may be replaced by -O- or -COO-, and at least one piece of -(CH₂)₂- may be replaced by -CH=CH- or -C=C-, and in the groups, at least one hydrogen may be replaced by halogen.

Preferred Z¹ or Z² is a single bond, -(CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH- (CH₂)₂- or -(CH₂)₂-CH=CH-. Further preferred Z¹ or Z² is a single bond, -(CH₂)₂-, -CH=CH-, -CF₂O-, -OCF₂-, -CH₂O-, -C≡C-, -(CH₂)₂OCF₂- or -OCH₂-.

When Z¹ or Z² is a single bond, chemical stability is high and the viscosity is small. When Z¹ or Z² is -C≡C-, the optical anisotropy is large.

In formula (1), Z³ is -O- or a single bond. A compound in which Z³ is -O- has the high clearing point and the large optical anisotropy, and therefore is preferred. A compound in which Z³ is a single bond has the small viscosity, and therefore is preferred.

In formula (1), L¹, L² and L³ are independently hydrogen, fluorine or chlorine. Preferred L¹, L² and L³ are a combination of hydrogen and fluorine. Preferred L¹, L² and L³ are a combination of fluorine and fluorine.

In formula (1), a and b are independently 0, 1, 2, 3 or 4, and a sum of a and b is 4 or less. A preferred sum of a and b is 0, 1 or 2.

When the sum of a and b is 0, compound (1) has the small viscosity. When the sum of a and b is 1 or 2, compound (1) has the high clearing point.

Examples of preferred compound (1) include compounds (1-1) to (1-10) described in item 5.

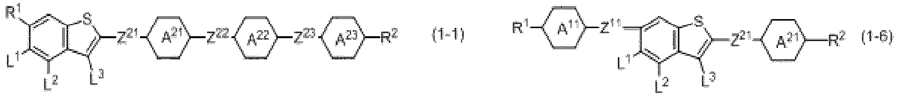

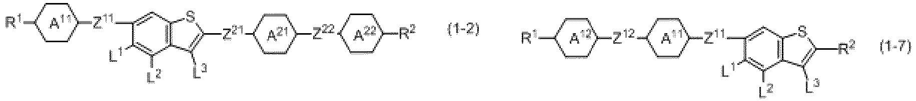

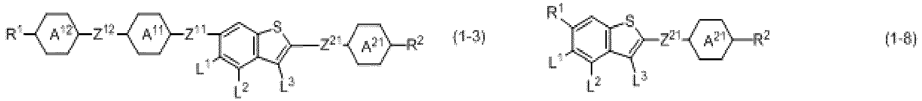

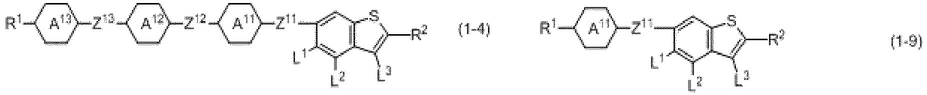

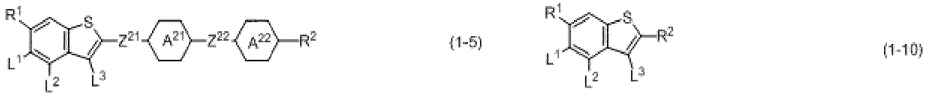

Preferred examples of ring A¹¹, ring A¹², ring A¹³, ring A²¹, ring A²² and ring A²³ in compounds (1-1) to (1-10), and effects of the groups on the physical properties are similar to a case in ring A¹ and ring A² in formula (1), and moreover, preferred examples of Z¹¹, Z¹², Z¹³, Z²¹, Z²² and Z²³ in compounds (1-1) to (1-10), and effects of the groups on the physical properties are similar to a case in Z¹ and Z² in formula (1) .

Specific examples of a preferred compound include compounds (No. 1) to (No. 200) described in Example 5.

### 2. Synthesis of compound (1)

A synthetic method of compound (1) will be described. Compound (1) can be prepared by suitably combining methods in organic synthetic chemistry. A method for introducing an objective terminal group, ring and bonding group into a starting material is described in books such as "Organic Syntheses" (John Wiley & Sons, Inc.), "Organic Reactions" (John Wiley & Sons, Inc.), "Comprehensive Organic Synthesis" (Pergamon Press) and "New Experimental Chemistry Course (Shin Jikken Kagaku Koza in Japanese)" (Maruzen Co., Ltd.).

### 2-1. Formation of bonding group Z

First, a scheme is shown with regard to a method for forming bonding groups Z¹ to Z². Next, reactions described in the scheme will be described in section (1) to section (11). In the scheme, MSG¹ (or MSG²) is a monovalent organic group having at least one ring. The monovalent organic groups represented by a plurality of MSG¹ (or MSG²) used in the scheme may be identical or different. Compounds (1A) to (1J) correspond to compound (1).

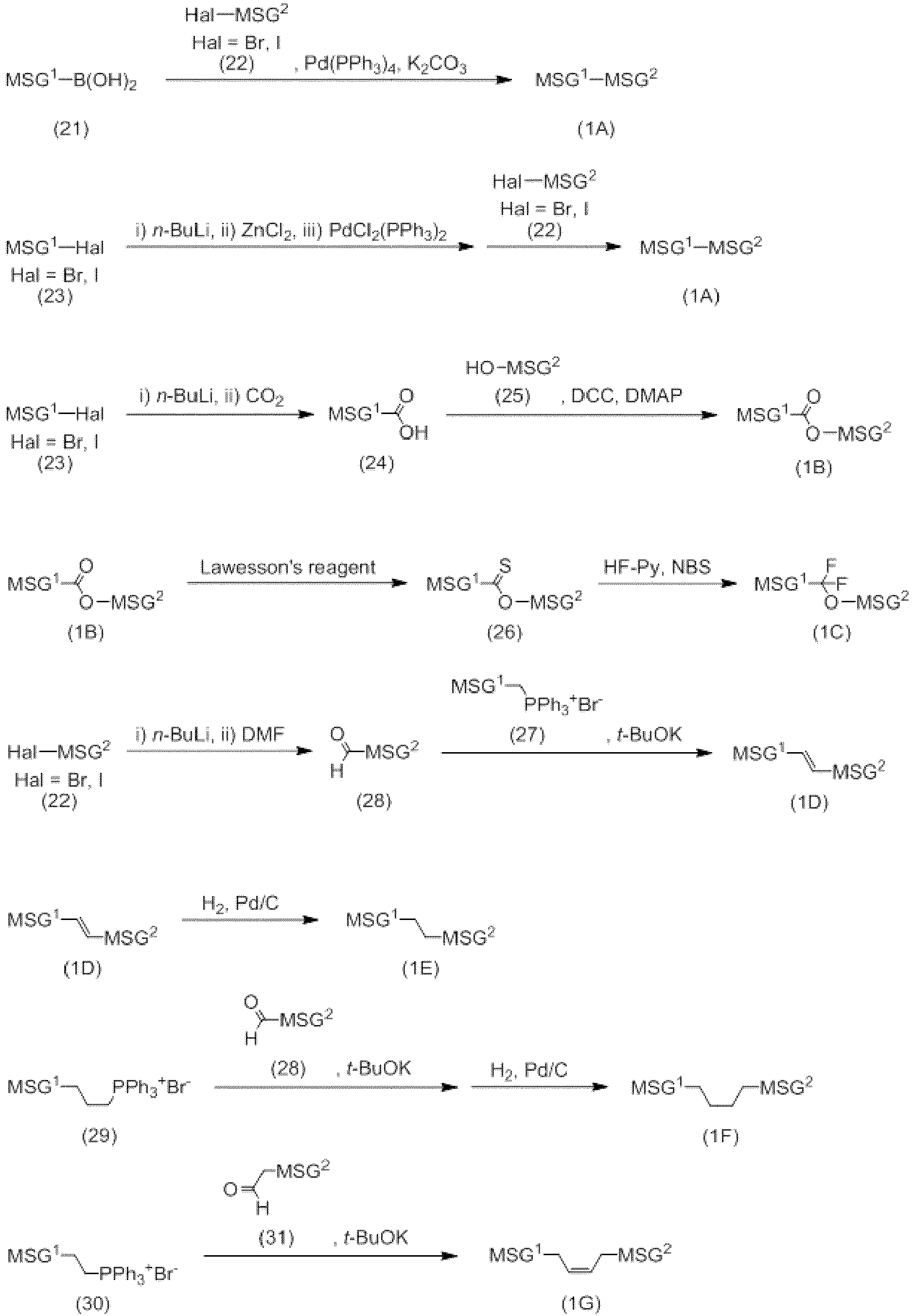

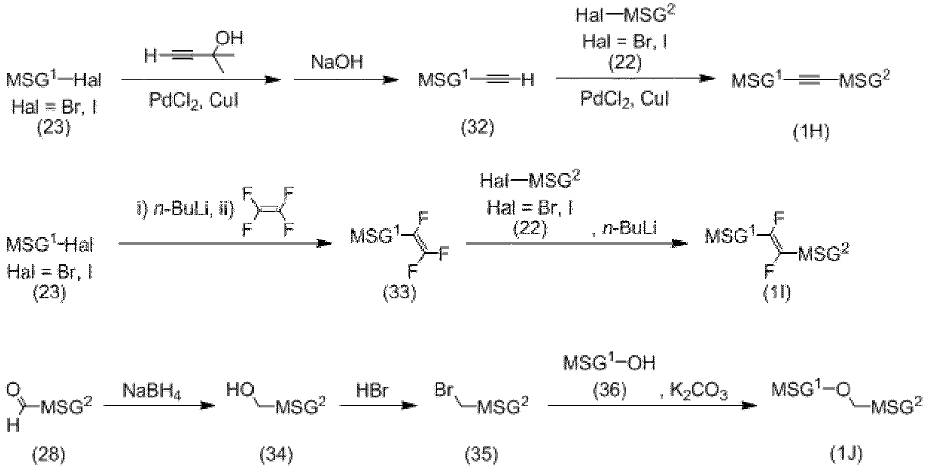

### (1) Formation of a single bond

Compound (1A) is prepared by allowing aryl boronic acid (21) prepared according to a publicly known method to react with halide (22) in the presence of a carbonate and a catalyst such as tetrakis(triphenylphosphine)palladium. Compound (1A) is also prepared by allowing halide (23) prepared according to a publicly known method to react with n-butyllithium and subsequently with zinc chloride, and further with halide (22) in the presence of a catalyst such as dichlorobis(triphenylphosphine)palladium.

### (2) Formation of -COO-

Carboxylic acid (24) is obtained by allowing halide (23) to react with n-butyllithium and subsequently with carbon dioxide. Compound (1B) is prepared by dehydration of compound (25) prepared according to a publicly known method and carboxylic acid (24) in the presence of 1,3-dicyclohexylcarbodiimide (DCC) and 4-dimethylaminopyridine (DMAP).

### (3) Formation of -CF₂O-

Thionoester (26) is obtained by treating compound (1B) with a thiation reagent such as Lawesson's reagent. Compound (1C) is prepared by fluorinating thionoester (26) with a hydrogen fluoride-pyridine complex and N-bromosuccinimide (NBS). Refer to M. Kuroboshi et al., Chem. Lett., 1992, 827. Compound (1C) is also prepared by fluorinating thionoester (26) with (diethylamino) sulfur trifluoride (DAST) . Refer to W. H. Bunnelle et al., J. Org. Chem. 1990, 55, 768. The bonding groups can also be formed according to a method described in Peer. Kirsch et al., Angew. Chem. Int. Ed. 2001, 40, 1480.

### (4) Formation of -CH=CH-

Aldehyde (28) is obtained by treating halide (22) with n-butyllithium and then allowing the treated halide to react with formamide such as N,N-dimethylformamide (DMF). Compound (1D) is prepared by allowing phosphorus ylide generated by treating phosphonium salt (27) prepared according to a publicly known method with a base such as potassium t-butoxide to react with aldehyde (28) . A cis isomer may be generated depending on reaction conditions, and therefore the cis isomer is isomerized into a trans isomer according to a publicly known method when necessary.

### (5) Formation of -(CH₂)₂-

Compound (1E) is prepared by hydrogenating compound (1D) in the presence of a catalyst such as palladium on carbon.

### (6) Formation of -(CH₂)₄-

A compound having -(CH₂)₂-CH=CH- is obtained by using phosphonium salt (29) in place of phosphonium salt (27) according to the method in section (4). Compound (1F) is prepared by performing catalytic hydrogenation of the compound obtained.

### (7) Formation of -CH₂CH=CHCH₂-

Compound (1G) is prepared by using phosphonium salt (30) in place of phosphonium salt (27) and aldehyde (31) in place of aldehyde (28) according to the method in section (4). A trans isomer may be generated depending on reaction conditions, and therefore the trans isomer is isomerized to a cis isomer according to a publicly known method when necessary.

### (8) Formation of -C=C-

Compound (32) is obtained by allowing halide (23) to react with 2-methyl-3-butyn-2-ol in the presence of a catalyst of dichloropalladium and copper halide, and then performing deprotection under basic conditions. Compound (1H) is prepared by allowing compound (32) to react with halide (22) in the presence of the catalyst of dichloropalladium and copper halide.

### (9) Formation of -CF=CF-

Compound (33) is obtained by treating halide (23) with n-butyllithium, and then allowing the treated halide to react with tetrafluoroethylene. Compound (1I) is prepared by treating halide (22) with n-butyllithium, and then allowing the treated halide to react with compound (33).

### (10) Formation of -OCH₂-

Compound (34) is obtained by reducing aldehyde (28) with a reducing agent such as sodium borohydride. Bromide (35) is obtained by brominating compound (34) with hydrobromic acid or the like. Compound (1J) is prepared by allowing bromide (35) to react with compound (36) in the presence of a base such as potassium carbonate.

### (11) Formation of -(CF₂)₂-

A compound having -(CF₂)₂- is obtained by fluorinating diketone (-COCO-) with sulfur tetrafluoride, in the presence of a hydrogen fluoride catalyst, according to a method described in J. Am. Chem. Soc., 2001, 123, 5414.

### 2-2. Formation of ring A¹ and ring A²

A starting material is commercially available or a synthetic method is well known with regard to a ring such as 1,4-cyclohexylene, 1,4-phenylene, 2-fluoro-1,4-phenylene, 2,6-difluoro-1,4-phenylene, tetrahydropyran-2,5-diyl, 1,3-dioxane-2,5-diyl, pyrimidine-2,5-diyl and pyridine-2,5-diyl.

### 2-3. Method for preparing compound (1)

An example of a method for preparing compound (1) is as described below. In the compounds, R¹, R², ring A¹, ring A², Z¹, Z², Z³, L¹, L², L³, a and b are defined in a manner identical to the definitions in item 1 described above. A Grignard reagent is prepared by allowing compound (b-1) prepared according to a publicly known method to react with Mg and allowing the prepared Grignard reagent to react with S and bromoacetaldehyde diethyl acetal to obtain (b-2). The resulting material is allowed to react with polyphosphoric acid in toluene or chlorobenzene to obtain (b-3). Then, (b-3) is subjected to lithiation with LDA into (b-4) and allowing various reagents to react therewith to obtain various intermediates. Compound (1) is derived therefrom by using various intermediates, according to a publicly known method.

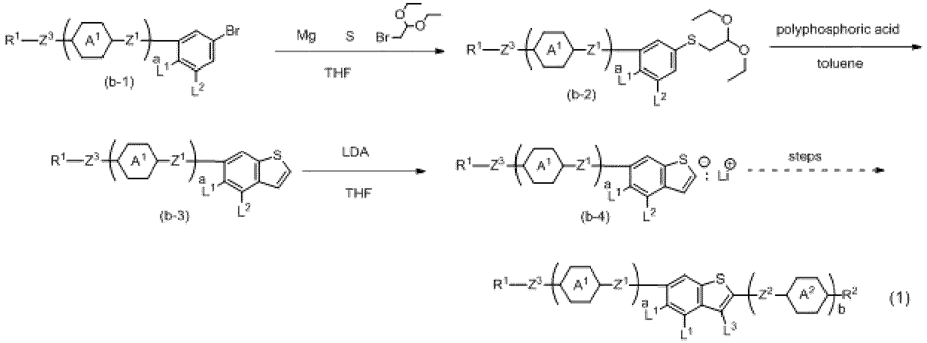

### 3. Liquid crystal composition

### 3-1. Component compound

A liquid crystal composition of the invention will be described. The composition contains at least one compound (1) as component A. Compound (1) is useful for increasing a maximum temperature of the composition. The composition may contain two, three or more compounds (1). A component in the composition may be only compound (1). In order to develop excellent physical properties, the composition preferably contains at least one of compounds (1) in the range of 1% by weight to 99% by weight. In a composition having positive dielectric anisotropy, a preferred content of compound (1) is in the range of 5% by weight to 60% by weight. In a composition having negative dielectric anisotropy, a preferred content of compound (1) is 30% by weight or less. The composition may also contain compound (1) and liquid crystal compounds that are not described herein.

The composition contains compound (1) as component A, and preferably further contains a liquid crystal compound selected from components B, C, D and E described below. Component B includes compounds (2) to (4). Component C includes compounds (5) to (7) . Component D includes compound (8). Component E includes compounds (9) to (15). The composition may contain any other liquid crystal compound different from compounds (2) to (15). When the composition is prepared, components B, C, D and E are preferably selected by taking into account the positive or negative dielectric anisotropy, magnitude of the dielectric anisotropy, or the like. A composition in which the components are suitably selected has high stability to heat or light, high maximum temperature, low minimum temperature, small viscosity, suitable optical anisotropy (more specifically, large optical anisotropy or small optical anisotropy), large dielectric anisotropy, large specific resistance and a suitable elastic constant (more specifically, a large elastic constant or a small elastic constant).

Component B includes a compound in which two terminal groups are alkyl or the like. Specific examples of preferred component B include compounds (2-1) to (2-11), compounds (3-1) to (3-19) and compounds (4-1) to (4-7) . In a compound of component B, R¹¹ and R¹² are independently alkyl having 1 to 10 carbons or alkenyl having 2 to 10 carbons, and in the alkyl or the alkenyl, at least one piece of -CH₂- may be replaced by -O-, and at least one hydrogen may be replaced by fluorine.

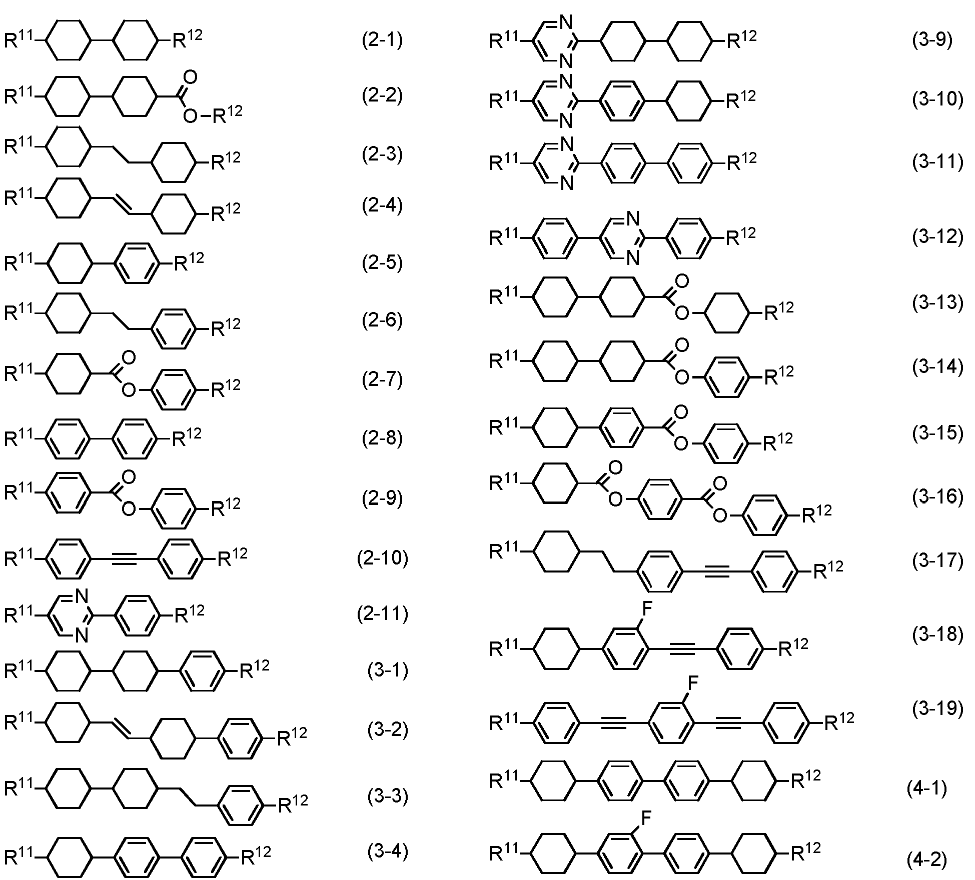

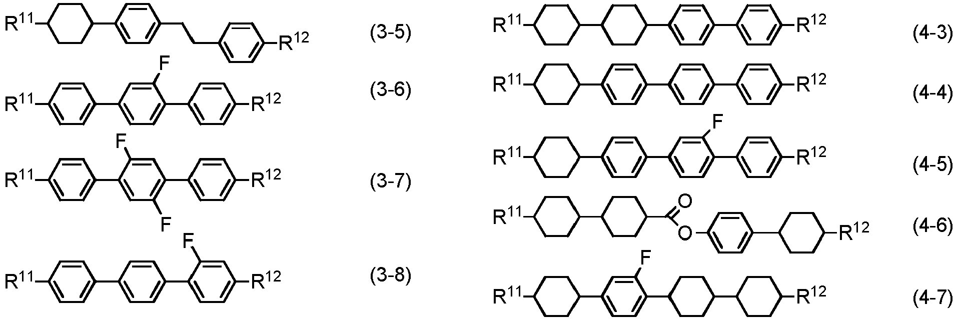

Component B has a small absolute value of dielectric anisotropy, and therefore is a compound close to neutrality. Compound (2) is mainly effective in decreasing the viscosity or adjusting the optical anisotropy. Compounds (3) and (4) are effective in extending a temperature range of a nematic phase by increasing the maximum temperature, or in adjusting the optical anisotropy.

As a content of component B is increased, the viscosity of the composition is decreased, but the dielectric anisotropy is decreased. Thus, as long as a desired value of a threshold voltage of the device is met, the content is preferably as large as possible. When a composition for the IPS mode, the VA mode or the like is prepared, the content of component B is preferably 30% by weight or more, and further preferably 40% by weight or more, based on the weight of the liquid crystal composition.

Component C is a compound having a halogen-containing group or a fluorine-containing group at a right terminal. Specific examples of preferred component C include compounds (5-1) to (5-16), compounds (6-1) to (6-113) and compounds (7-1) to (7-57). In a compound of component C, R¹³ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and X¹¹ is fluorine, chlorine, -OCF₃, -OCHF₂, -CF₃, -CHF₂, -CH₂F, -OCF₂CHF₂ or -OCF₂CHFCF₃.

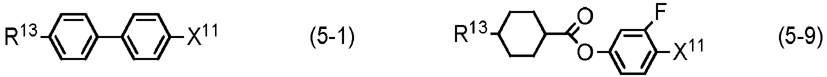

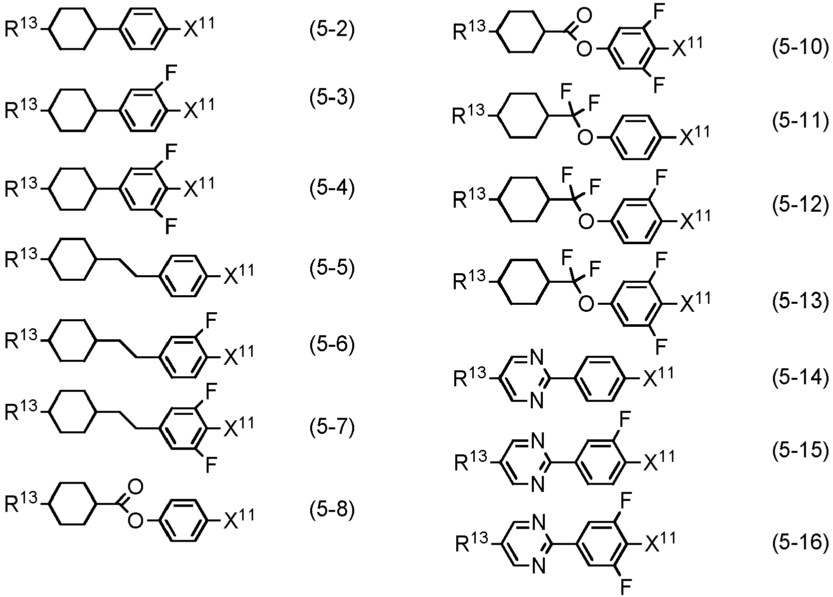

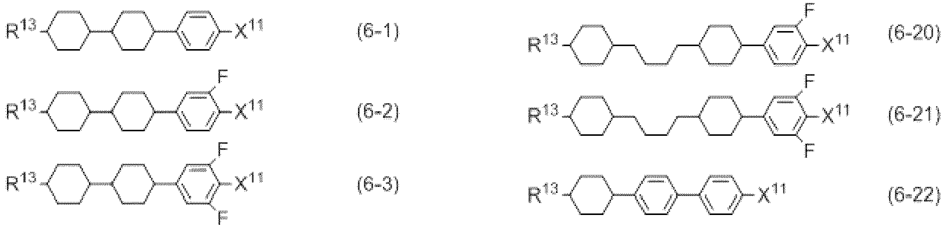

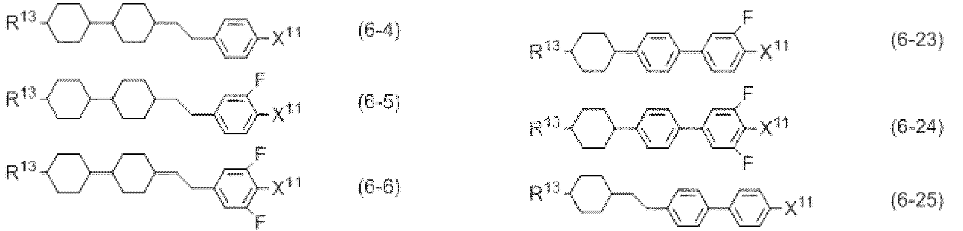

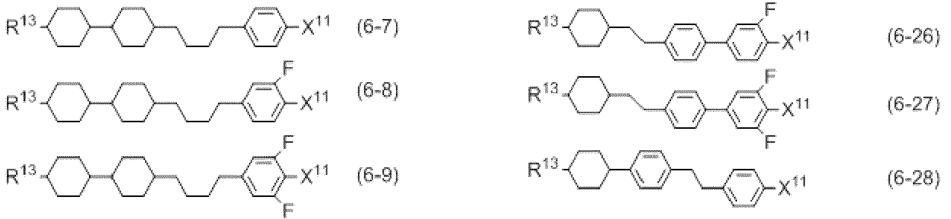

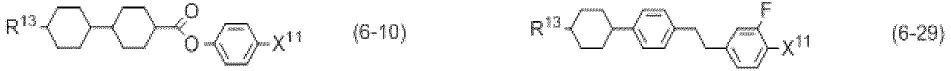

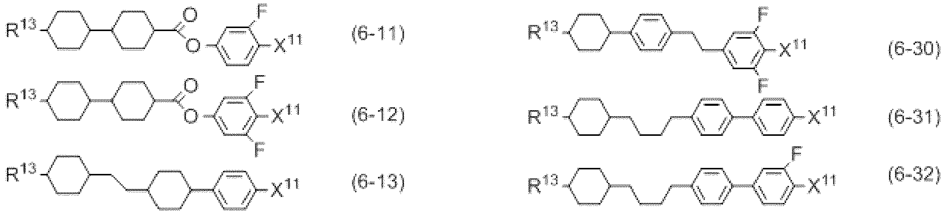

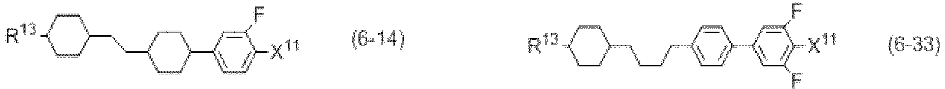

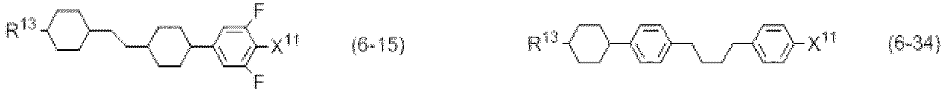

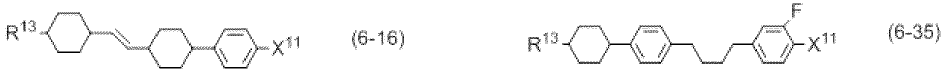

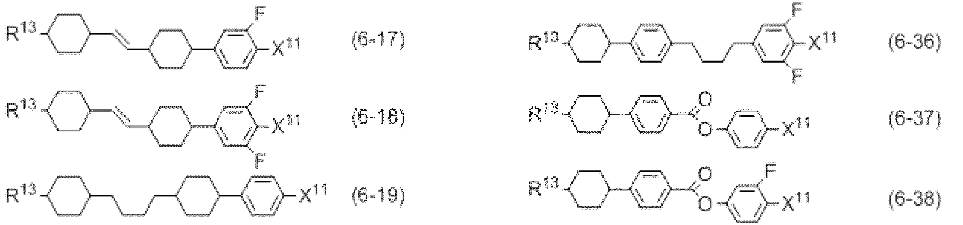

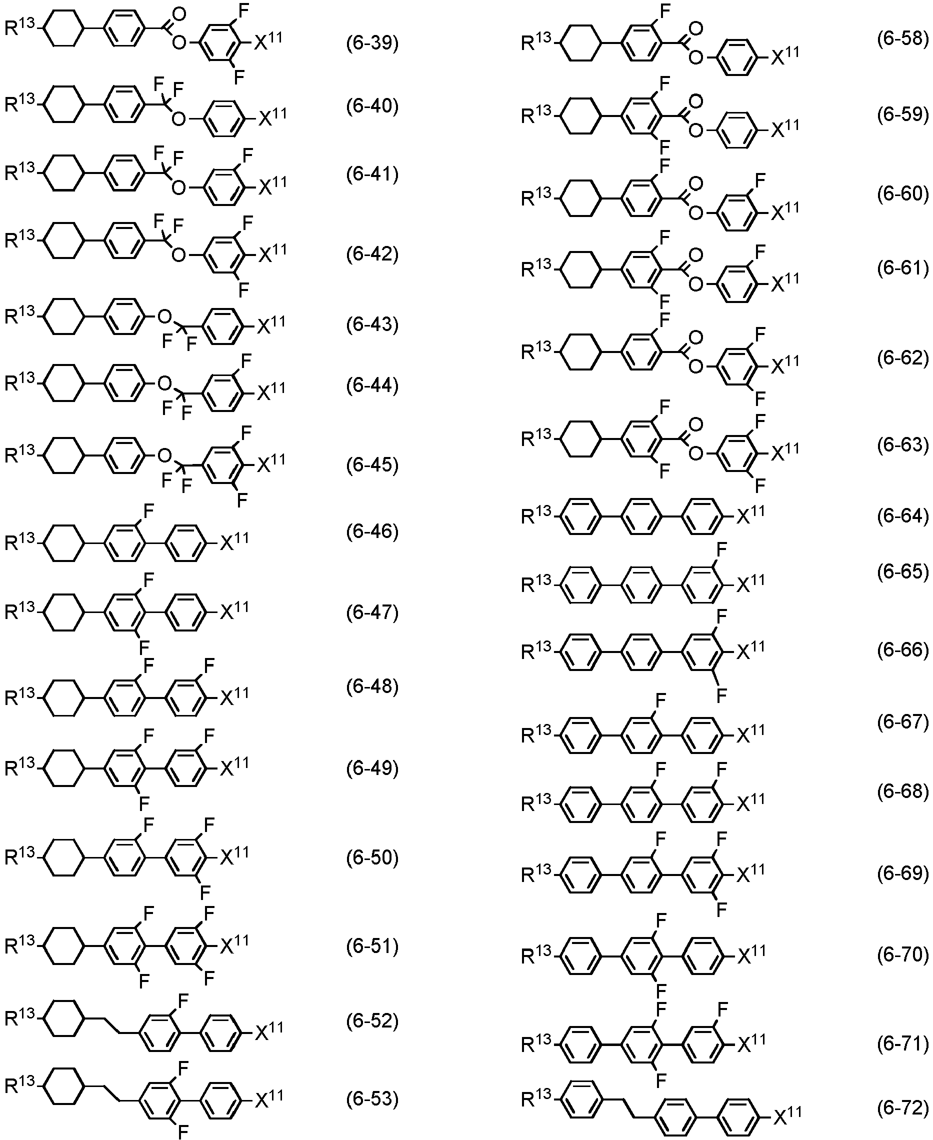

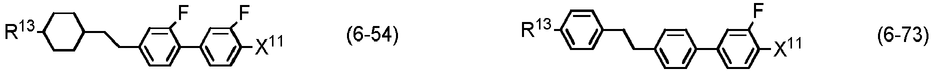

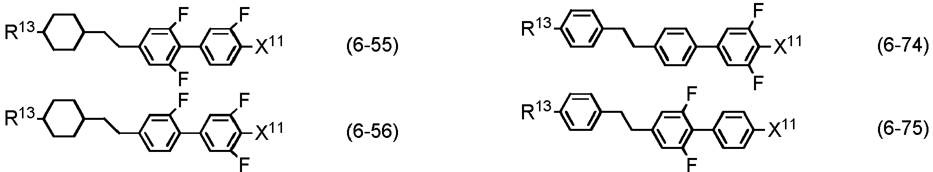

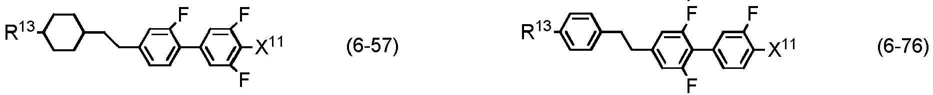

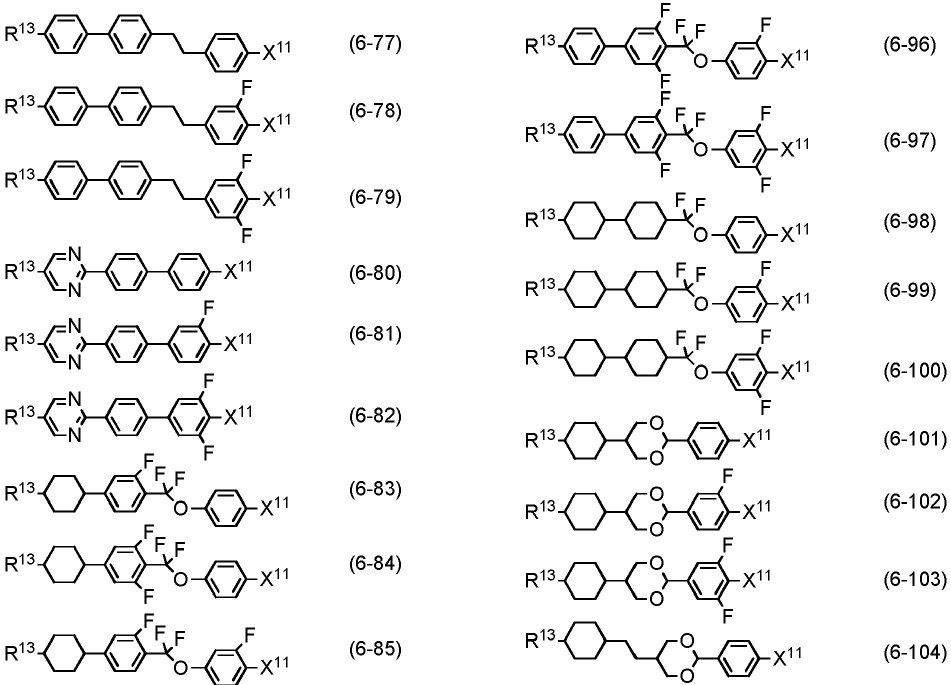

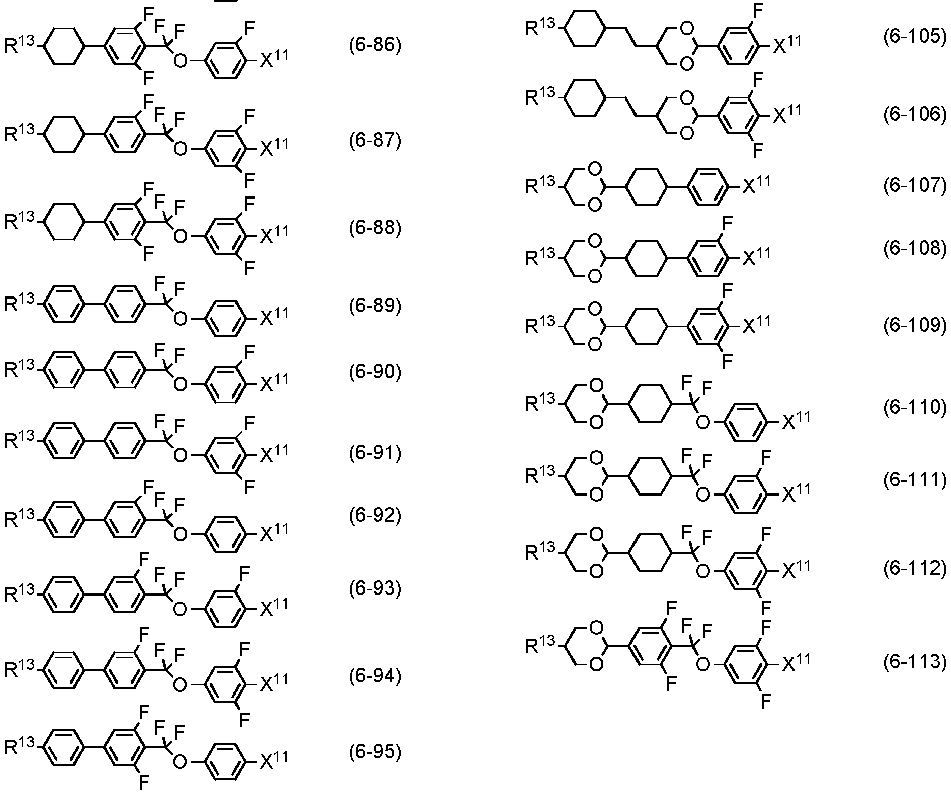

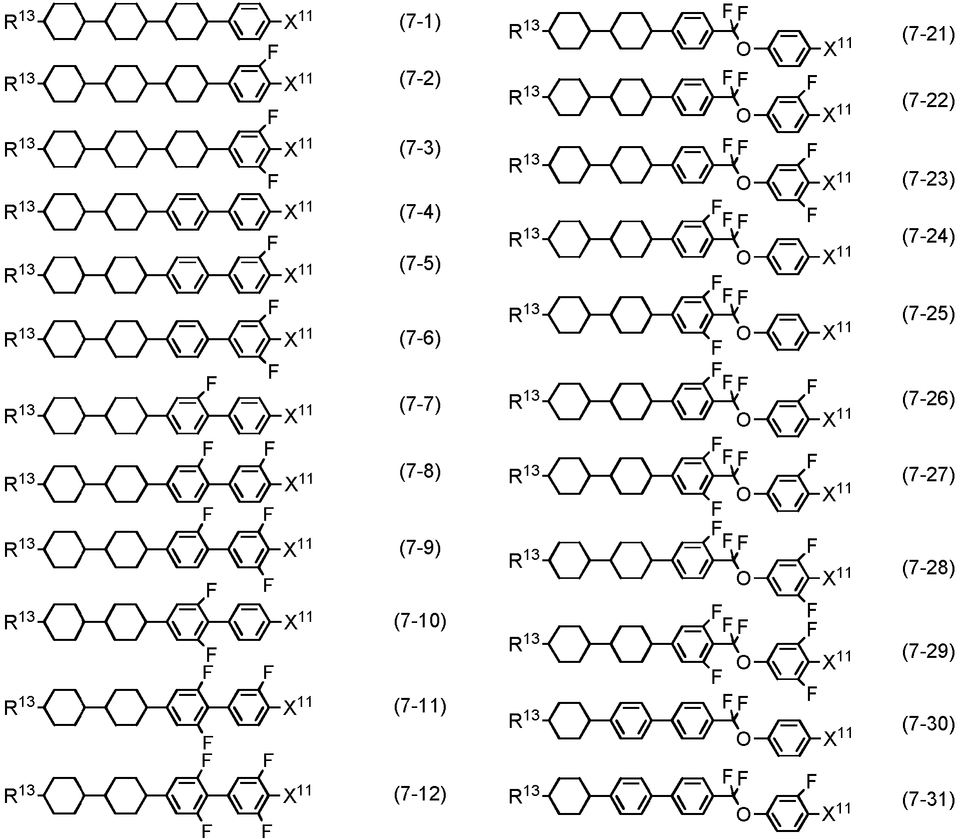

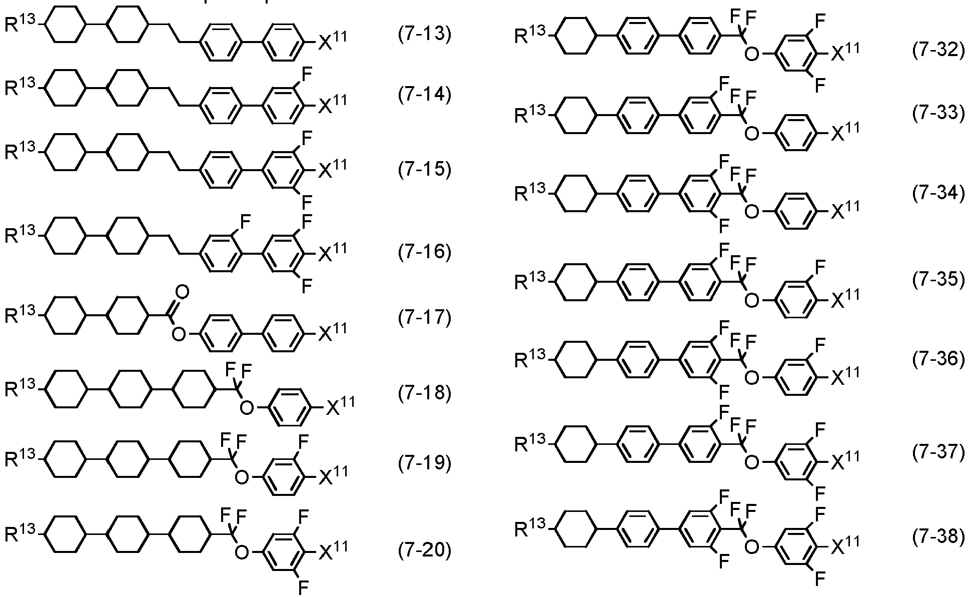

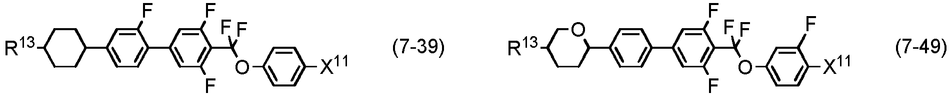

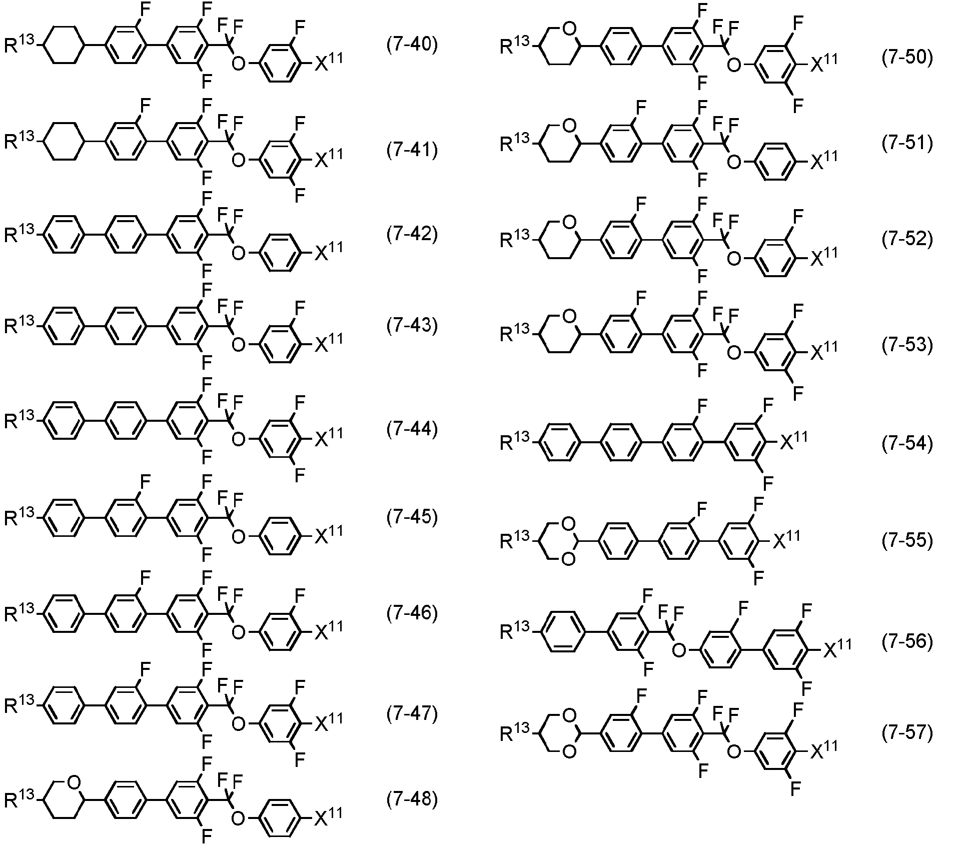

Component C has positive dielectric anisotropy, and superb stability to heat, light or the like, and therefore is used when a composition for the IPS mode, the FFS mode, the OCB mode or the like is prepared. A content of component C is suitably in the range of 1% by weight to 99% by weight, preferably in the range of 10% by weight to 97% by weight, and further preferably in the range of 40% by weight to 95% by weight, based on the weight of the liquid crystal composition. When component C is added to a composition having negative dielectric anisotropy, the content of component C is preferably 30% by weight or less based thereon. Addition of component C allows adjustment of the elastic constant of the composition and adjustment of a voltage-transmittance curve of the device.

Component D is compound (8) in which a right-terminal group is -C≡N or -C≡C-C≡N. Specific examples of preferred component D include compounds (8-1) to (8-64) . In a compound of component D, R¹⁴ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and X¹² is -C≡N or -C≡C-C≡N.

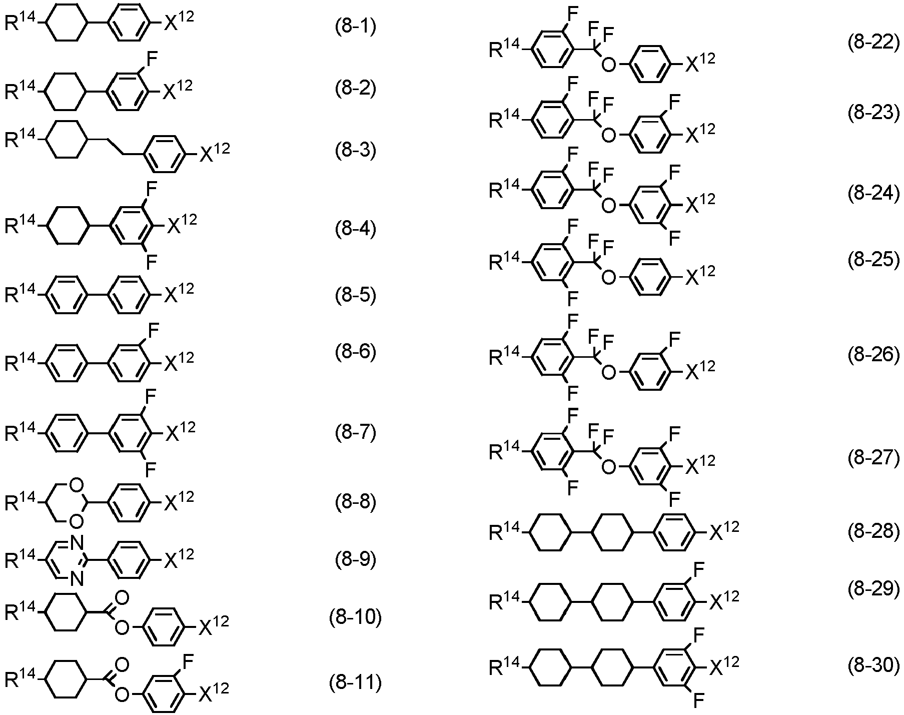

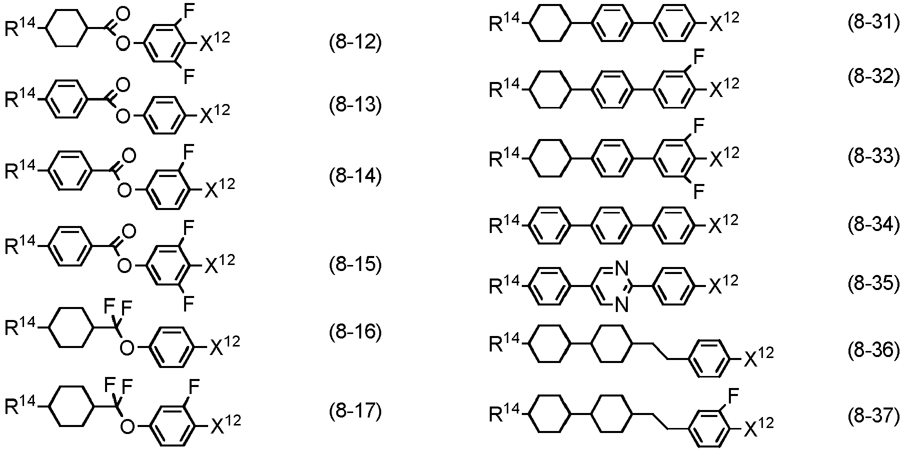

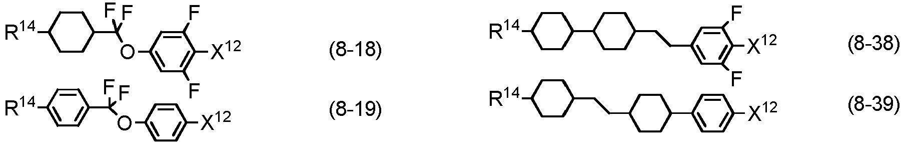

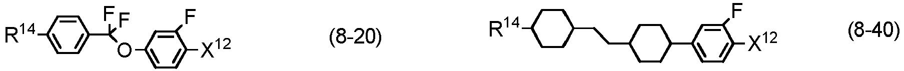

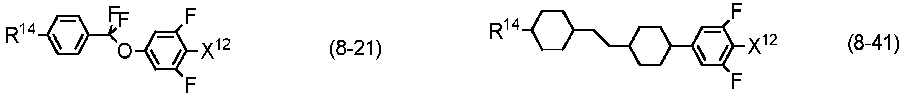

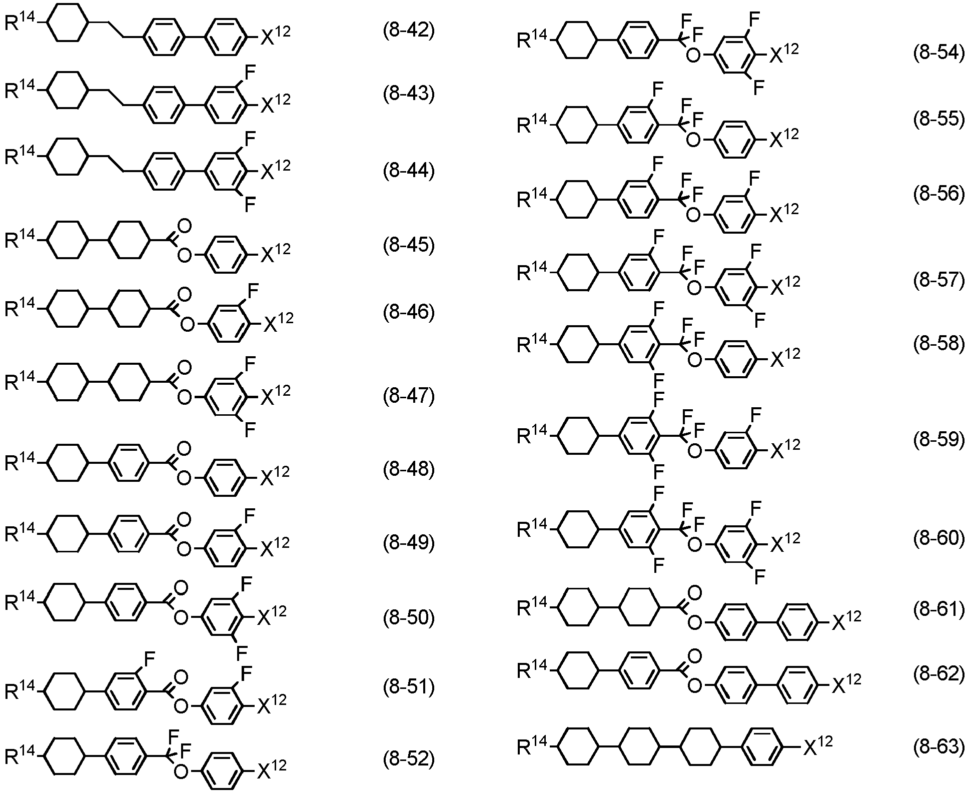

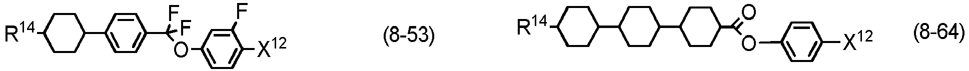

Component D has positive dielectric anisotropy and a value thereof is large, and therefore is mainly used when a composition for the TN mode or the like is prepared. Addition of component D can increase the dielectric anisotropy of the composition. Component D is effective in extending the temperature range of the liquid crystal phase, adjusting the viscosity or adjusting the optical anisotropy. Component D is also useful for adjustment of the voltage-transmittance curve of the device.

When the composition for the TN mode or the like is prepared, a content of component D is suitably in the range of 1% by weight to 99% by weight, preferably in the range of 10% by weight to 97% by weight, and further preferably in the range of 40% by weight to 95% by weight, based on the weight of the liquid crystal composition. When component D is added to a composition having negative dielectric anisotropy, the content of component D is preferably 30% by weight or less based thereon. Addition of component D allows adjustment of the elastic constant of the composition and adjustment of the voltage-transmittance curve of the device.

Component E includes compounds (9) to (15). The compounds have phenylene in which hydrogen in lateral positions are replaced by two halogens, such as 2,3-difluoro-1,4-phenylene. Specific examples of preferred component E include compounds (9-1) to (9-8), compounds (10-1) to (10-17), compound (11-1), compounds (12-1) to (12-3), compounds (13-1) to (13-11), compounds (14-1) to (14-3) and compounds (15-1) to (15-3) . In the compounds, R¹⁵ and R¹⁶ are independently alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and R¹⁷ is hydrogen, fluorine, alkyl having 1 to 10 carbons or alkenyl having 2 to 10 carbons, and in the alkyl and the alkenyl, at least one piece of -CH₂- may be replaced by -O-, and at least one hydrogen may be replaced by fluorine.

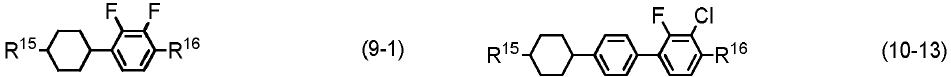

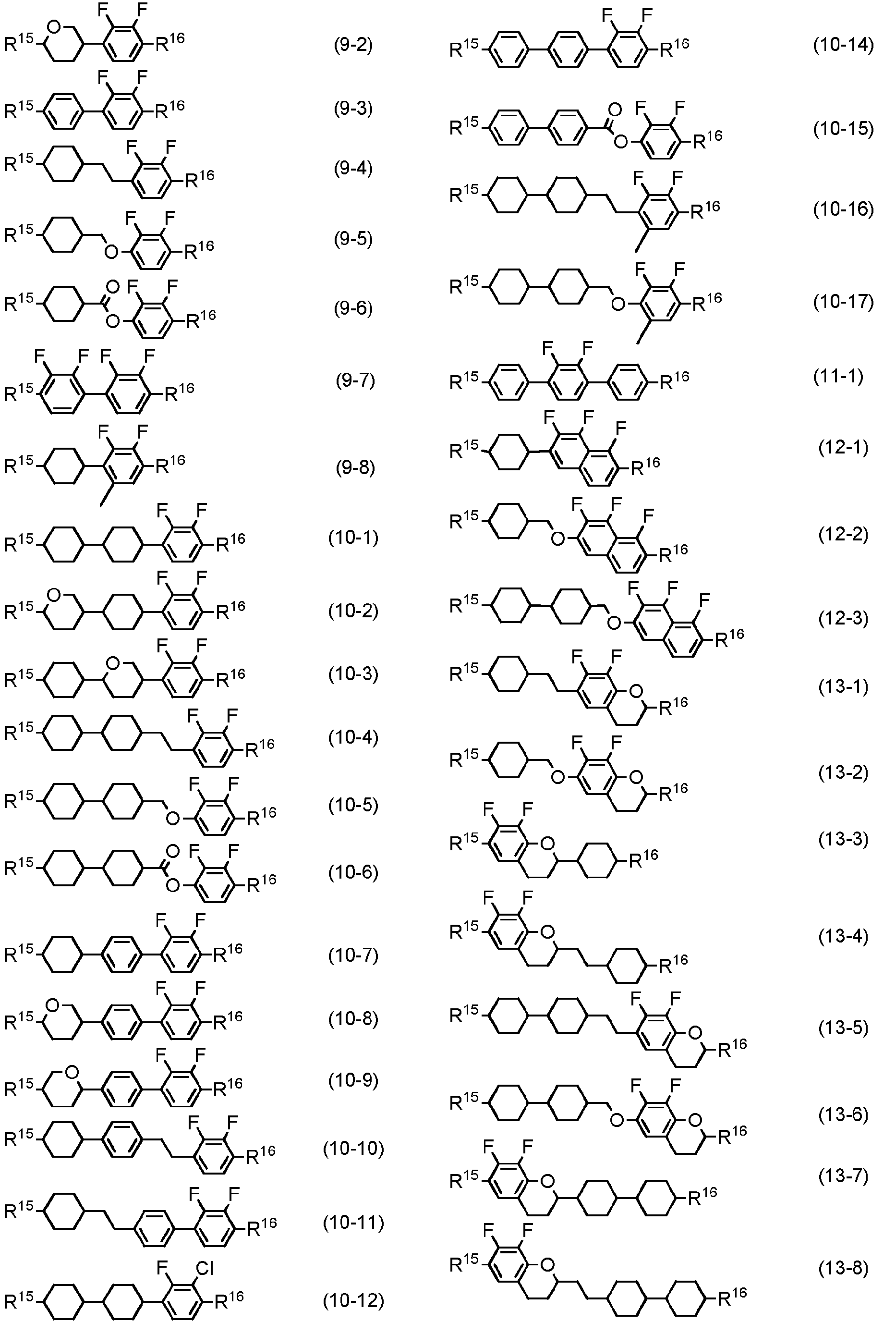

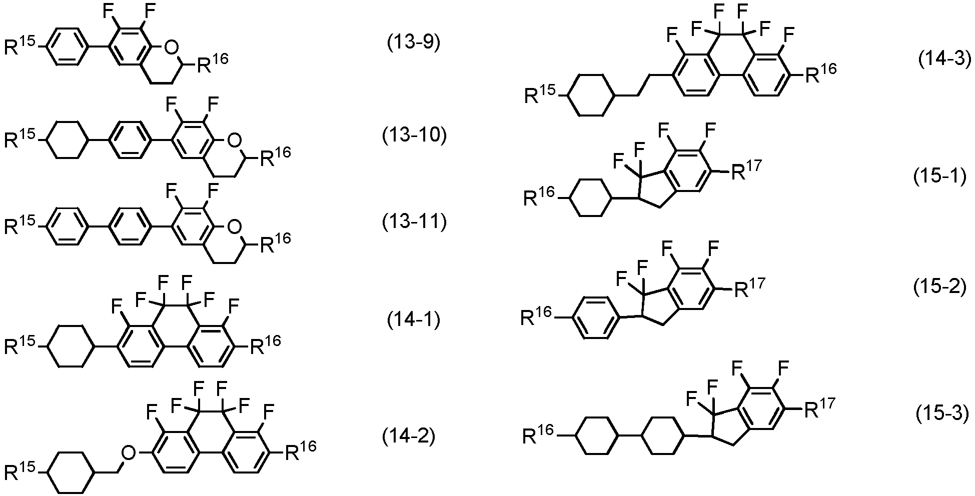

Component E has negatively large dielectric anisotropy. Component E is used when a composition for the IPS mode, the VA mode, the PSA mode or the like is prepared. As a content of component E is increased, the dielectric anisotropy of the composition is negatively increased, but the viscosity is increased. Thus, as long as the desired value of threshold voltage of the device is met, the content is preferably as small as possible. When the dielectric anisotropy at a degree of -5 is taken into account, the content is preferably 40% by weight or more in order to allow a sufficient voltage driving.

Among types of component E, compound (9) is a bicyclic compound, and therefore is mainly effective in decreasing the viscosity, adjusting the optical anisotropy or increasing the dielectric anisotropy. Compounds (10) and (11) are a tricyclic compound, and therefore are effective in increasing the maximum temperature, the optical anisotropy or the dielectric anisotropy. Compounds (12) to (15) are effective in increasing the dielectric anisotropy.

When a composition for the IPS mode, the VA mode, the PSA mode or the like is prepared, the content of component E is preferably 40% by weight or more, and further preferably in the range of 50% by weight to 95% by weight, based on the weight of the liquid crystal composition. When component E is added to a composition having positive dielectric anisotropy, the content of the compounds is preferably 30% by weight or less based thereon. Addition of component E allows adjustment of the elastic constant of the composition and adjustment of the voltage-transmittance curve of the device.

A liquid crystal composition satisfying at least one of physical properties such as high stability to heat or light, high maximum temperature, low minimum temperature, small viscosity, suitable optical anisotropy, large dielectric anisotropy, large specific resistance, large specific resistance and a suitable elastic constant can be prepared by suitably combining components B, C, D and E described above. A liquid crystal compound different from components B, C, D and E may be added when necessary.

### 3-2. Additive

A liquid crystal composition is prepared according to a publicly known method. For example, the component compounds are mixed and dissolved in each other by heating. According to an application, an additive may be added to the composition. Examples of the additive include the polymerizable compound, the polymerization initiator, the polymerization inhibitor, the optically active compound, the antioxidant, the ultraviolet light absorber, the light stabilizer, the heat stabilizer, the dye and the antifoaming agent. Such an additive is well known to those skilled in the art, and described in literature.

In a liquid crystal display device having the polymer sustained alignment (PSA) mode, the composition contains a polymer. The polymerizable compound is added for the purpose of forming the polymer in the composition. The polymerizable compound is polymerized by irradiation with ultraviolet light while voltage is applied between electrodes, and thus the polymer is formed in the composition. A suitable pretilt is obtained by the method, and therefore the device in which a response time is shortened and the image persistence is improved is obtained.

Preferred examples of the polymerizable compound include acrylate, methacrylate, a vinyl compound, a vinyloxy compound, propenyl ether, an epoxy compound (oxirane, oxetane) and vinyl ketone. Further preferred examples include a compound having at least one acryloyloxy, and a compound having at least one methacryloyloxy. Still further preferred examples also include a compound having both acryloyloxy and methacryloyloxy.

Still further preferred examples include compounds (M-1) to (M-17) . In the compounds, R²⁵ to R³¹ are independently hydrogen or methyl; s, v and x are independently 0 or 1; and t and u are independently an integer from 1 to 10. L²¹ to L²⁶ are independently hydrogen or fluorine; and L²⁷ and L²⁸ are independently hydrogen, fluorine or methyl.

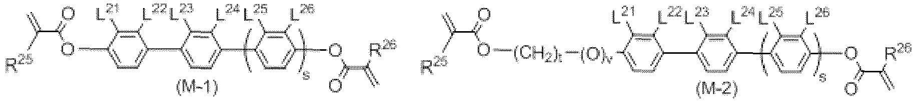

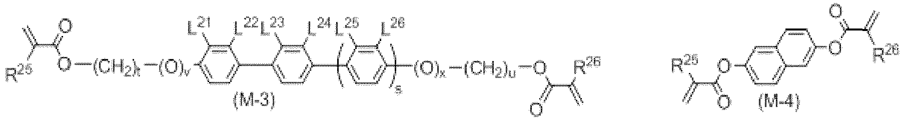

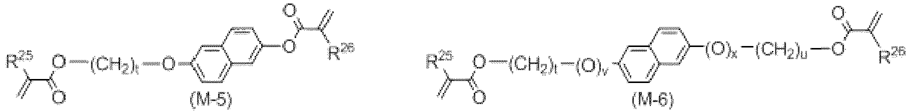

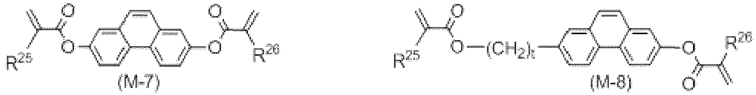

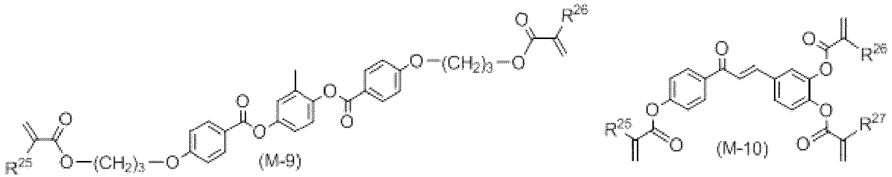

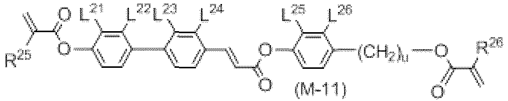

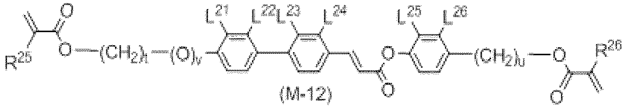

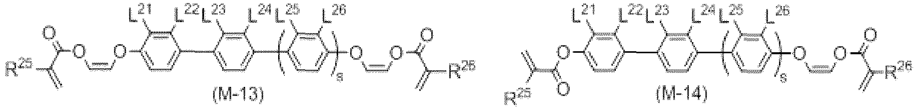

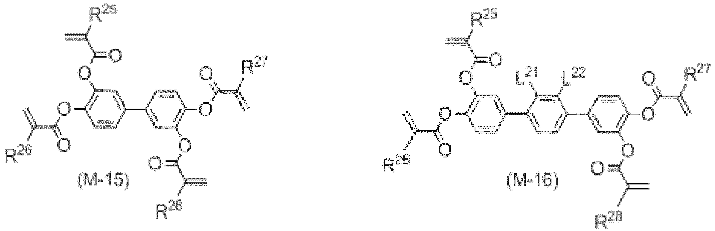

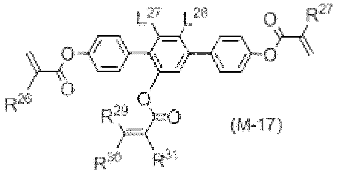

The polymerizable compound can be rapidly polymerized by adding the polymerization initiator. An amount of a remaining polymerizable compound can be decreased by optimizing a reaction temperature. Examples of a photoradical polymerization initiator include TPO, 1173 and 4265 from Darocur series of BASF SE, and 184, 369, 500, 651, 784, 819, 907, 1300, 1700, 1800, 1850 and 2959 from Irgacure series thereof.

Additional examples of the photoradical polymerization initiator include 4-methoxyphenyl-2,4-bis(1,3,5-trichloromethyl)triazine, 2-(4-butoxystyryl)-5-trichloromethyl-1,3,4-oxadiazole, 9-phenylacridine, 9,10-benzphenazine, a benzophenone-Michler's ketone mixture, a hexaarylbiimidazole-mercaptobenzimidazole mixture, 1-(4-isopropylphenyl)-2-hydroxy-2-methylpropane-1-one, benzyl dimethyl ketal, 2-methyl-1-[4-(methylthio)phenyl]-2-morpholinopropane-1-one, a mixture of 2,4-diethylxanthone and methyl p-dimethylaminobenzoate, and a mixture of benzophenone and methyltriethanolamine.

After the photoradical polymerization initiator is added to the liquid crystal composition, polymerization can be performed by irradiation with ultraviolet light while an electric field is applied. However, an unreacted polymerization initiator or a decomposition product of the polymerization initiator may cause poor display such as image persistence in the device. In order to prevent such an event, photopolymerization may be performed with no addition of the polymerization initiator. A preferred wavelength of irradiation light is in the range of 150 nanometers to 500 nanometers. A further preferred wavelength is in the range of 250 nanometers to 450 nanometers, and a most preferred wavelength is in the range of 300 nanometers to 400 nanometers.

Upon storing the polymerizable compound, the polymerization inhibitor may be added thereto for preventing polymerization. The polymerizable compound is ordinarily added to the composition without removing the polymerization inhibitor. Examples of the polymerization inhibitor include hydroquinone, a hydroquinone derivative such as methylhydroquinone, 4-t-butylcatechol, 4-methoxyphenol and phenothiazine.

The optically active compound is effective in inducing helical structure in liquid crystal molecules to give a required twist angle, and thereby preventing a reverse twist. A helical pitch can be adjusted by adding the optically active compound thereto. Two or more optically active compounds may be added for the purpose of adjusting temperature dependence of the helical pitch. Specific examples of a preferred optically active compound include compounds (Op-1) to (Op-18) described below. In compound (Op-18), ring J is 1,4-cyclohexylene or 1,4-phenylene, and R²⁸ is alkyl having 1 to 10 carbons.

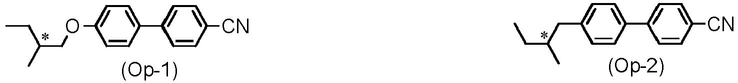

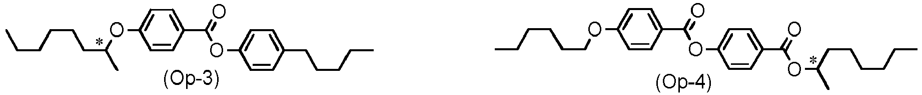

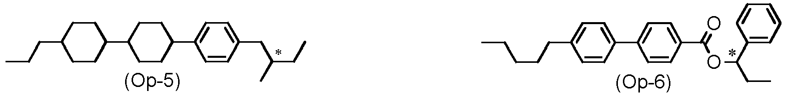

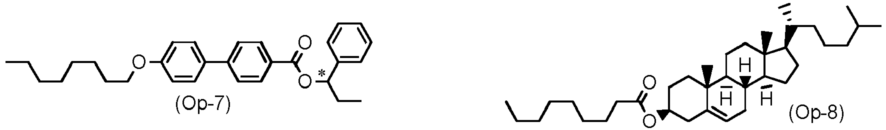

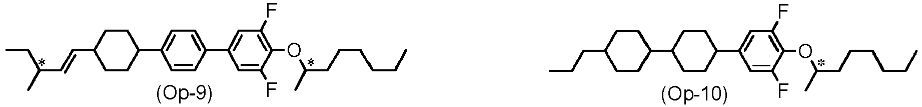

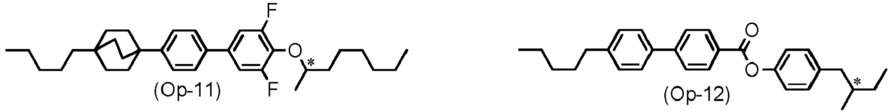

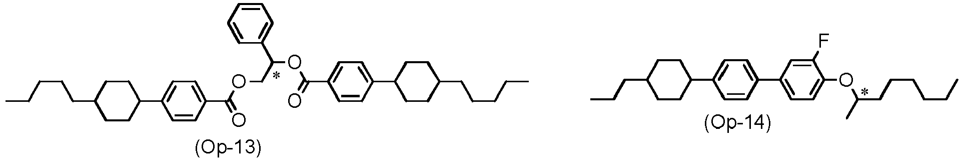

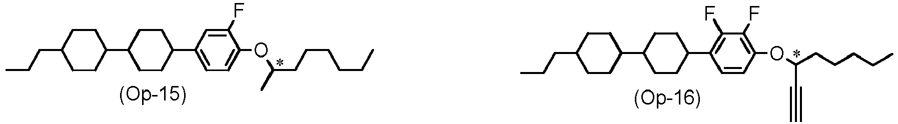

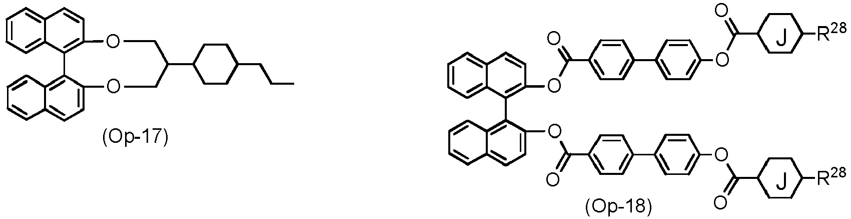

The antioxidant is effective for maintaining a large voltage holding ratio. Specific examples of a preferred antioxidant include compounds (AO-1) and (AO-2) described below; and IRGANOX 415, IRGANOX 565, IRGANOX 1010, IRGANOX 1035, IRGANOX 3114 and IRGANOX 1098 (trade names; BASF SE). The ultraviolet light absorber is effective for preventing a decrease of the maximum temperature. Preferred examples of the ultraviolet light absorber include a benzophenone derivative, a benzoate derivative and a triazole derivative, and specific examples include compounds (AO-3) and (AO-4) described below; TINUVIN 329, TINUVIN P, TINUVIN 326, TINUVIN 234, TINUVIN 213, TINUVIN 400, TINUVIN 328 and TINUVIN 99-2 (trade names; BASF SE); and 1,4-diazabicyclo[2.2.2]octane (DABCO).

The light stabilizer such as an amine having steric hindrance is preferred for maintaining the large voltage holding ratio. Specific examples of a preferred light stabilizer include compounds (AO-5) and (AO-6) described below; TINUVIN 144, TINUVIN 765 and TINUVIN 770DF (trade names; BASF SE). The heat stabilizer is also effective for maintaining the large voltage holding ratio, and specific preferred examples include IRGAFOS 168 (trade name; BASF SE). A dichroic dye such as an azo dye or an anthraquinone dye is added to the composition to be adapted for a device having a guest host (GH) mode. The antifoaming agent is effective for preventing foam formation. Preferred examples of the antifoaming agent include dimethyl silicone oil and methylphenyl silicone oil.

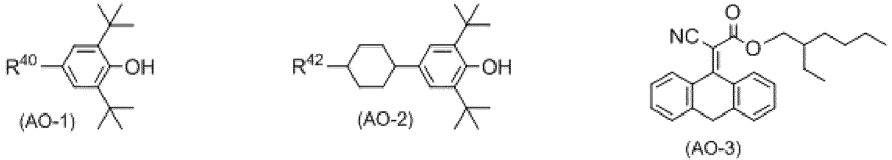

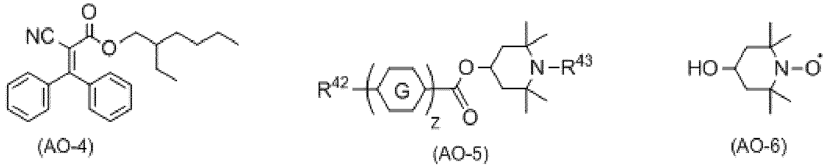

In compound (AO-1), R⁴⁰ is alkyl having 1 to 20 carbons, alkoxy having 1 to 20 carbons, -COOR⁴¹ or -CH₂CH₂COOR⁴¹, in which R⁴¹ is alkyl having 1 to 20 carbons. In compounds (AO-2) and (AO-5), R⁴² is alkyl having 1 to 20 carbons. In compound (AO-5), R⁴³ is hydrogen, methyl or O (oxygen radical); ring G is 1,4-cyclohexylene or 1,4-phenylene; and z is 1, 2 or 3.

### 4. Liquid crystal display device

The liquid crystal composition can be used in a liquid crystal display device having an operating mode such as the PC mode, the TN mode, the STN mode, the OCB mode and the PSA mode, and driven by an active matrix mode. The composition can also be used in a liquid crystal display device having the operating mode such as the PC mode, the TN mode, the STN mode, the OCB mode, the VA mode and the IPS mode, and driven by a passive matrix mode. The devices can be applied to any of a reflective type, a transmissive type and a transflective type.

The composition is also suitable for a nematic curvilinear aligned phase (NCAP) device, and the composition is microencapsulated herein. The composition can also be used in a polymer dispersed liquid crystal display device (PDLCD) or a polymer network liquid crystal display device (PNLCD). In the compositions, a large amount of liquid crystal compound is added. On the other hand, when an amount of adding the polymerizable compound is about 10% by weight or less based on the weight of the liquid crystal composition, the liquid crystal display device having the PSA mode is prepared. A preferred proportion is in the range of about 0.1% by weight to about 2% by weight based thereon. A further preferred proportion is in the range of about 0.2% by weight to about 1.0% by weight based thereon. The device having the PSA mode can be driven by a driving mode such as the active matrix mode and the passive matrix mode. Such a device can also be applied to any of the reflective type, the transmissive type and the transflective type.

### Examples

The invention will be described in greater detail by way of Examples (including Synthesis Examples and Use Examples). However, the invention is not limited by the Examples. The invention includes a mixture of a composition in Use Example 1 and a composition in Use Example 2. The invention also includes a composition prepared by mixing at least two of compositions in Use Examples.

### 1. Example of compound (1)

Compound (1) was prepared according to procedures described below. The thus prepared compound was identified by methods such as an NMR analysis. Physical properties of the compound and the composition, and characteristics of a device were measured by methods described below.

NMR analysis: For measurement, DRX-500 made by Bruker BioSpin Corporation was used. In ¹H-NMR measurement, a sample was dissolved in a deuterated solvent such as CDCl₃, and measurement was carried out under conditions of room temperature, 500 MHz and 16 times of accumulation. Tetramethylsilane was used as an internal standard. In ¹⁹F-NMR measurement, CFCl₃ was used as an internal standard, and measurement was carried out under conditions of 24 times of accumulation. In explaining nuclear magnetic resonance spectra obtained, s, d, t, q, quin, sex and m stand for a singlet, a doublet, a triplet, a quartet, a quintet, a sextet and a multiplet, and br being broad, respectively.

Gas chromatographic analysis: For measurement, GC-2010 Gas Chromatograph made by Shimadzu Corporation was used. As a column, a capillary column DB-1 (length 60 m, bore 0.25 mm, film thickness 0.25 µm) made by Agilent Technologies, Inc. was used. As a carrier gas, helium (1 mL/minute) was used. A temperature of a sample vaporizing chamber and a temperature of a detector (FID) part were set to 300°C and 300°C, respectively. A sample was dissolved in acetone and prepared to be a 1 weight % solution, and then 1 microliter of the solution obtained was injected into the sample vaporizing chamber. As a recorder, GC Solution System made by Shimadzu Corporation or the like was used.

HPLC Analysis: For measurement, Prominence (LC-20AD; SPD-20A) made by Shimadzu Corporation was used. As a column, YMC-Pack ODS-A (length 150 mm, bore 4.6 mm, particle diameter 5 µm) made by YMC Co., Ltd. was used. As an eluate, acetonitrile and water were appropriately mixed and used. As a detector, a UV detector, an RI detector, a CORONA detector or the like was appropriately used. When the UV detector was used, a detection wavelength was set to 254 nanometers. A sample was dissolved in acetonitrile and prepared to be a 0.1 weight % solution, and then 1 microliter of the solution was injected into a sample chamber. As a recorder, C-R7Aplus made by Shimadzu Corporation was used.

Ultraviolet-Visible spectrophotometry: For measurement, PharmaSpec UV-1700 made by Shimadzu Corporation was used. A detection wavelength was adjusted in the range of 190 nanometers to 700 nanometers. A sample was dissolved in acetonitrile and prepared to be a 0.01 mmol/L solution, and measurement was carried out by putting the solution in a quartz cell (optical path length: 1 cm).

Sample for measurement: Upon measuring phase structure and a transition temperature (a clearing point, a melting point, a polymerization starting temperature or the like), the compound itself was used as a sample. Upon measuring physical properties such as maximum temperature of a nematic phase, viscosity, optical anisotropy and dielectric anisotropy, a mixture of the compound and a base liquid crystal was used as a sample.

When the sample prepared by mixing the compound with the base liquid crystal was used, an extrapolated value was calculated according to the following formula and the calculated value was described: $\left[extrapolated value\right] = \left(100 \times \left[measured value of a sample\right] - \left[% by weight of a base liquid crystal\right] \times \left[measured value of the base liquid crystal\right]\right) / \left[% by weight of a compound\right] .$

Base liquid crystal (A): As the base liquid crystal, base liquid crystal (A) described below was used. A proportion of each component was expressed in terms of weight percent (% by weight).

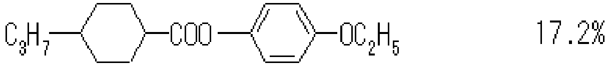

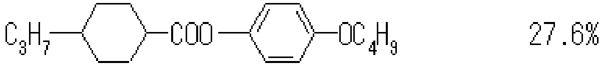

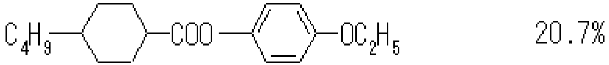

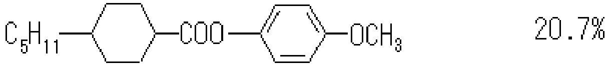

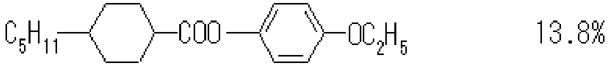

A ratio of the compound to base liquid crystal (A) was adjusted to (15% by weight : 85% by weight) . When crystals (or a smectic phase) precipitated at 25°C at the ratio, a ratio of the compound to base liquid crystal (A) was changed in the order of (10% by weight : 90% by weight), (5% by weight : 95% by weight) and (1% by weight : 99% by weight), and characteristics of the sample were measured at a ratio at which no crystal (or no smectic phase) precipitated at 25°C. In addition, unless otherwise noted, the ratio of the compound to base liquid crystal (A) was (15% by weight : 85% by weight).

Measuring method: Physical properties were measured according to methods described below. Most of the measuring methods are applied as described in the Standard of Japan Electronics and Information Technology Industries Association (JEITA) (JEITA ED-2521B) discussed and established by JEITA, or modified thereon. No thin film transistor (TFT) was attached to a TN device used for measurement.

### (1) Phase structure

A sample was placed on a hot plate in a melting point apparatus (FP-52 Hot Stage made by Mettler-Toledo International Inc., or 10083L Cooling and Heating Hot Stage made by Linkam Scientific Instruments Ltd.) equipped with a polarizing microscope. A state of phase and a change thereof were observed with the polarizing microscope while the sample was heated at a rate of 3°C per minute, and a kind of the phase was specified.

### (2) Transition temperature (°C)

For measurement, a differential scanning calorimeter, Diamond DSC System, made by PerkinElmer, Inc., or a high sensitivity differential scanning calorimeter, X-DSC7000, made by SII NanoTechnology Inc. was used. A sample was heated and then cooled at a rate of 3°C per minute, and a starting point of an endothermic peak or an exothermic peak caused by a phase change of the sample was determined by extrapolation, and thus a transition temperature was determined. A melting point and a polymerization starting temperature of a compound were also measured using the apparatus. Temperature at which a compound undergoes transition from a solid to a liquid crystal phase such as the smectic phase and the nematic phase may be occasionally abbreviated as "minimum temperature of the liquid crystal phase." Temperature at which the compound undergoes transition from the liquid crystal phase to liquid may be occasionally abbreviated as "clearing point."

A crystal was expressed as C. When kinds of the crystals were distinguishable, each of the crystals was expressed as C₁ or C₂. The smectic phase or the nematic phase was expressed as S or N. When smectic A phase, smectic B phase, smectic C phase or smectic F phase was distinguishable among the smectic phases, the phases were expressed as S_{A}, S_{B}, S_{C} or S_{F}, respectively. A liquid (isotropic) was expressed as I. A transition temperature was expressed as "C 50.0 N 100.0 I," for example. The expression indicates that a transition temperature from the crystals to the nematic phase is 50.0°C, and a transition temperature from the nematic phase to the liquid is 100.0°C.

### (3) Compatibility at low temperature

Samples in which the base liquid crystal and the compound were mixed for proportions of the compounds to be 20% by weight, 15% by weight, 10% by weight, 5% by weight, 3% by weight and 1% by weight were prepared, and put in glass vials. After the glass vials were kept in freezers at -10°C or -20°C for a predetermined period of time, whether or not crystals or a smectic phase precipitated was observed.

### (4) Maximum temperature of nematic phase (T_{NI} or NI; °C)

A sample was placed on a hot plate in a melting point apparatus equipped with a polarizing microscope, and heated at a rate of 1°C per minute. Temperature when part of the sample began to change from a nematic phase to an isotropic liquid was measured. When the sample was a mixture of compound (1) and the base liquid crystal, the maximum temperature was expressed in terms of a symbol T_{NI}. When the sample was a mixture of compound (1) and a compound such as components B, C and D, the maximum temperature was expressed in terms of a symbol NI. A maximum temperature of the nematic phase may be occasionally abbreviated as "maximum temperature."

### (5) Minimum temperature of nematic phase (T_{C}; °C)

Samples each having a nematic phase were put in glass vials and kept in freezers at temperatures of 0°C, -10°C, -20°C, -30°C and -40°C for 10 days, and then liquid crystal phases were observed. For example, when the sample was maintained in the nematic phase at -20 °C and changed to crystals or a smectic phase at -30°C, T_{C} was expressed as T_{C} < -20°C. A minimum temperature of the nematic phase may be occasionally abbreviated as "minimum temperature."

### (6) Viscosity (bulk viscosity; η; measured at 20°C; mPa·s)

For measurement, a cone-plate (E type) rotational viscometer made by Tokyo Keiki Inc. was used.

### (7) Viscosity (rotational viscosity; γ1; measured at 25°C; mPa·s)

Measurement was carried out according to a method described in M. Imai et al., Molecular Crystals and Liquid Crystals, Vol. 259, p. 37 (1995). A sample was put in a TN device in which a twist angle was 0 degrees and a distance (cell gap) between two glass substrates was 5 micrometers. Voltage was applied stepwise to the device in the range of 16 V to 19.5 V at an increment of 0.5 V. After a period of 0.2 second with no voltage application, voltage was repeatedly applied under conditions of only one rectangular wave (rectangular pulse; 0.2 second) and no voltage application (2 seconds) . A peak current and a peak time of transient current generated by the applied voltage were measured. A value of rotational viscosity was obtained from the measured values and calculation equation (8) on page 40 of the paper presented by M. Imai et al. A value of dielectric anisotropy required for the calculation was determined using the device by which the rotational viscosity was measured and by a method described below.

### (8) Optical anisotropy (refractive index anisotropy; measured at 25°C; Δn)

Measurement was carried out by an Abbe refractometer with a polarizing plate mounted on an ocular, using light at a wavelength of 589 nanometers. A surface of a main prism was rubbed in one direction, and then a sample was added dropwise onto the main prism. A refractive index (n∥) was measured when a direction of polarized light was parallel to a direction of rubbing. A refractive index (n⊥) was measured when the direction of polarized light was perpendicular to the direction of rubbing. A value of optical anisotropy (Δn) was calculated from an equation: Δn = n∥ - n⊥.

### (9) Dielectric anisotropy (Δε; measured at 25°C)

A sample was put in a TN device in which a distance (cell gap) between two glass substrates was 9 micrometers and a twist angle was 80 degrees. Sine waves (10 V, 1 kHz) were applied to the device, and after 2 seconds, a dielectric constant (ε∥) of liquid crystal molecules in a major axis direction was measured. Sine waves (0.5 V, 1 kHz) were applied to the device, and after 2 seconds, a dielectric constant (ε⊥) of liquid crystal molecules in a minor axis direction was measured. A value of dielectric anisotropy was calculated from an equation: Δε = ε∥ - ε⊥.

### (10) Elastic constant (K; measured at 25°C; pN)

For measurement, HP4284A LCR Meter made by Yokogawa-Hewlett-Packard Co. was used. A sample was put in a horizontal alignment device in which a distance (cell gap) between two glass substrates was 20 micrometers. An electric charge of 0 V to 20 V was applied to the device, and electrostatic capacity and applied voltage were measured. The measured values of electrostatic capacity (C) and applied voltage (V) were fitted to equation (2.98) and equation (2.101) on page 75 of "Liquid Crystal Device Handbook (Ekisho Debaisu Handobukku in Japanese; Nikkan Kogyo Shimbun, Ltd.)," and values of K₁₁ and K₃₃ were obtained from equation (2. 99) . Next, K₂₂ was calculated using the previously determined values of K₁₁ and K₃₃ in equation (3.18) on page 171. Elastic constant K was expressed in terms of a mean value of the thus determined K₁₁, K₂₂ and K₃₃.

### (11) Threshold voltage (Vth; measured at 25°C; V)

For measurement, an LCD-5100 luminance meter made by Otsuka Electronics Co., Ltd. was used. A light source was a halogen lamp. A sample was put in a normally white mode TN device in which a distance (cell gap) between two glass substrates was about 0.45/Δn (µm) and a twist angle was 80 degrees. A voltage (32 Hz, rectangular waves) to be applied to the device was stepwise increased from 0 V to 10 V at an increment of 0.02 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. A threshold voltage is expressed in terms of voltage at 90% transmittance.

### (12) Voltage holding ratio (VHR-1; measured at 25°C; %)

A TN device used for measurement had a polyimide alignment film, and a distance (cell gap) between two glass substrates was 5 micrometers. A sample was put in the device, and then the device was sealed with an ultraviolet-curable adhesive. The device was charged by applying a pulse voltage (60 microseconds at 5 V) at 25°C. A decaying voltage was measured for 16.7 milliseconds with a high-speed voltmeter, and area A between a voltage curve and a horizontal axis in a unit cycle was determined. Area B is an area without decay. A voltage holding ratio is expressed in terms of a percentage of area A to area B.

### (13) Voltage holding ratio (VHR-2; measured at 80°C; %)

A voltage holding ratio was measured according to the method described above except that the voltage holding ratio was measured at 80°C in place of 25°C. The results obtained were expressed in terms of a symbol VHR-2.

### (14) Specific resistance (ρ; measured at 25°C; Ω cm)

Into a vessel equipped with electrodes, 1.0 milliliter of sample was injected. A direct current voltage (10 V) was applied to the vessel, and a direct current after 10 seconds was measured. Specific resistance was calculated from the following equation: (specific resistance) = {(voltage) × (electric capacity of a vessel)} / {(direct current) × (dielectric constant of vacuum)}.

### (15) Response time (τ; measured at 25°C; ms)

For measurement, an LCD5100 luminance meter made by Otsuka Electronics Co., Ltd. was used. A light source was a halogen lamp. A low-pass filter was set to 5 kHz. A sample was put in a normally white mode TN device in which a distance (cell gap) between two glass substrates was 5.0 micrometers and a twist angle was 80 degrees. A voltage (rectangular waves; 60 Hz, 5 V, 0.5 second) was applied to the device. On the occasion, the device was irradiated with light from a direction perpendicular to the device, and an amount of light transmitted through the device was measured. A maximum amount of light corresponds to 100% transmittance, and a minimum amount of light corresponds to 0% transmittance. A rise time (τr; millisecond) was expressed in terms of time required for a change from 90% transmittance to 10% transmittance. A fall time (τf; millisecond) was expressed in terms of time required for a change from 10% transmittance to 90% transmittance. A response time was expressed by a sum of the rise time and the fall time thus obtained.

### Raw material

Solmix (registered trademark) A-11 is a mixture of ethanol (85.5%), methanol (13.4%) and isopropanol (1.1%), and was purchased from Japan Alcohol Trading Co., Ltd. Tetrahydrofuran may be occasionally abbreviated as THF. Tetrabutylammonium bromide may be occasionally abbreviated as TBAB. N,N-dimethylformamide may be occasionally abbreviated as DMF. Then, 2-propanol may be occasionally abbreviated as IPA. Then, 1,2-dimethoxyethane may be occasionally abbreviated as DME. Hexamethyldisilazane potassium may be occasionally abbreviated as KHMDS.

### Example 1

### Synthesis of compound (No. 43)

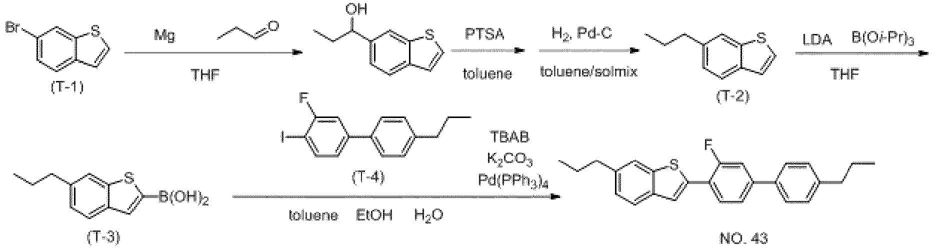

### First step

Under a nitrogen atmosphere, compound (T-1) (3 g) and THF (100 mL) were put in a reaction vessel, and the resulting mixture was cooled down to 0°C. Magnesium (0.36 g) was added thereto, and the resulting mixture was stirred for 2 hours. Propanal (0.98 g) was added thereto, and the resulting mixture was stirred for 1 hour while returning to room temperature. The resulting reaction solution was poured into water, and subjected to extraction with toluene. Combined organic layers were washed with water, a saturated aqueous solution of sodium hydrogencarbonate and water, and dried over anhydrous magnesium sulfate. Then, p-toluenesulfonic acid monohydrate (1 g) was added thereto, and the resulting mixture was refluxed for 1 hour while water was removed. The solution was returned to room temperature, and purified by silica gel column chromatography (toluene), and concentrated into a 100 mL solution. Palladium on carbon (1 g) and Solmix (100 mL) were added thereto, and the resulting mixture was stirred overnight under a hydrogen atmosphere. The resulting reaction solution was filtrated, and the resulting material was concentrated to obtain compound (T-2) (2.8 g).

### Second step

Under a nitrogen atmosphere, compound (T-2) (2.8 g) and THF (100 mL) were put in a reaction vessel, and the resulting mixture was cooled down to -60°C. Lithium diisopropylamide (1M; n-hexane solution; 19.06 mL) was added dropwise thereto, and the resulting mixture was further stirred for 2 hours. Subsequently, a THF (10 mL) solution of triisobutyl borate (4.18 g) was added dropwise thereto, and the resulting mixture was stirred for 2 hours while returning to room temperature. The resulting reaction mixture was poured into an aqueous hydrochloric acid solution, and subjected to extraction with ethyl acetate. Combined organic layers were washed with water, a saturated aqueous solution of sodium hydrogencarbonate and water, and dried over anhydrous magnesium sulfate. The solution was concentrated under reduced pressure to obtain compound (T-3) (3.3 g).

### Third step

(T-3) (3.3 g) and (T-4) (7 g) prepared according to a publicly known method were dissolved in toluene, and water, ethanol, Pd(PPh₃)₄ (1.7 g), TBAB (0.48 g) and potassium carbonate (6.2 g) were added thereto, and the resulting mixture was refluxed under heating for 6 hours . After completion of reaction, the resulting material was extracted with toluene, and washed with water, and then dried over anhydrous magnesium sulfate, and concentrated under reduced pressure to obtain a pale brown solid. The solid was formed into a solution, applied to silica gel column chromatography (heptane) and recrystallized to obtain (No. 43) (1.6 g) as a colorless crystal (ethanol).

¹H-NMR (δ ppm; CDCl₃): 7.72 (d, 1H), 7.71 (s, 1H), 7.70 (d, 1H), 7.64 (s, 1H), 7.53 (d, 2H), 7.42 (dd, 1H), 7.40 (dd, 1H), 7.27 (d, 2H), 7.19 (dd, 1H), 2.71 (t, 2H), 2.63 (t, 2H), 1.69 (sex, 2H), 1.68 (sex, 2H), 0.97 (t, 3H), 0.96 (t, 3H).

Physical properties of compound (No. 43) were as described below. Transition temperature: C 120 S 186.2 N 237.7 I.T_{NI} = 190.3°C; Δn = 0.377; Δε = 0.25; η = 60.3 mPa·s.

### Example 2

### Synthesis of compound (No. 56)

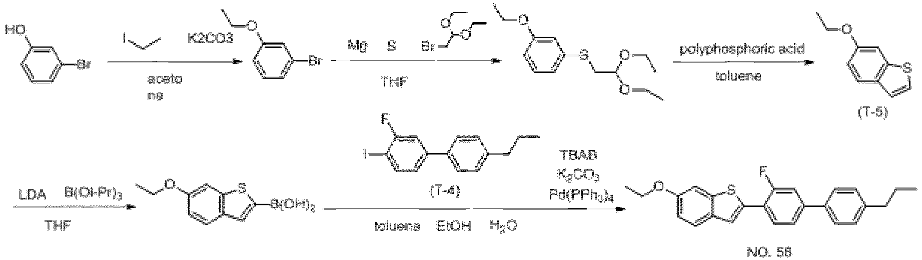

Compound (No. 56) was obtained by using compound (T-5) in place of compound (T-2) in Example 1. In addition, (T-5) was prepared according to a publicly known method as described in the scheme above.

¹H-NMR (δ ppm; CDCl₃): 7.70 (d, 1H), 7.69 (d, 1H), 7.68 (s, 1H), 7.53 (d, 2H), 7.42 (dd, 1H), 7.40 (dd, 1H), 7.30 (d, 1H), 7.27 (d, 2H), 6.99 (dd, 1H), 4.11 (q, 2H), 2.64 (t, 2H), 1.69 (sex, 2H), 1.46 (t, 2H), 0.98 (t, 3H).

Physical properties of compound (No. 56) were as described below. Transition temperature: C 168.5 N 276.2 I.T_{NI} = 227.3°C; Δn = 0.427; Δε = -0.75; η = 71.3 mPa·s.

### Example 3

### Synthesis of compound (No. 9)

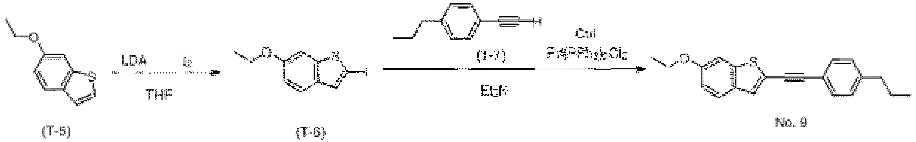

### First step

Under a nitrogen atmosphere, compound (T-5) (1.5 g) and THF (70 mL) were put in a reaction vessel, and the resulting mixture was cooled down to -74°C. Lithium diisopropylamide (1 M; n-hexane solution; 9.26 mL) was added dropwise thereto in the temperature range of -74°C to -70°C, and the resulting mixture was further stirred for 2 hours. Subsequently, iodine (2.56 g) was added thereto in the temperature range of -75°C to -70°C, and the resulting mixture was stirred for 8 hours while returning to 25°C. The resulting reaction mixture was poured into an aqueous solution of sodium hydrogensulfite, and subjected to extraction with toluene. Combined organic layers were washed with water, a saturated aqueous solution of sodium hydrogencarbonate and water, and dried over anhydrous magnesium sulfate. The solution was concentrated under reduced pressure to obtain compound (T-6) (2.6 g) .

### Second step

(T-6) (2.6 g), (T-7) (1.6 g) prepared according to a publicly known method, copper iodide (0.16 g), Pd(PPh₃)₂Cl₂ (0.6 g) and triethylamine (100 mL) were put in a vessel, and the resulting mixture was stirred overnight. The resulting reaction mixture was poured into water, and subjected to extraction with toluene, and washed with water, and then dried over anhydrous magnesium sulfate, and concentrated under reduced pressure to obtain a pale brown solid. The solid was formed into a solution, applied to silica gel column chromatography (heptane : toluene = 3 : 2 in a volume ratio) and recrystallized (ethanol) to obtain (No. 9) (1.4 g) as a colorless crystal.

¹H-NMR (δ ppm; CDCl₃): 7.61 (d, 1H), 7.45 (d, 2H), 7.38 (s, 1H), 4.09 (q, 2H), 2.60 (t, 2H), 1.65 (sex, 2H), 1.45 (t, 3H), 0.94 (t, 3H) .

Physical properties of compound (No. 9) were as described below. Transition temperature: C 73.5 N 111.3 I.T_{NI} = 104.3°C; Δn = 0.387; Δε = 0.1; η = 63.2 mPa·s.

### Example 4

### Synthesis of compound (No. 33)

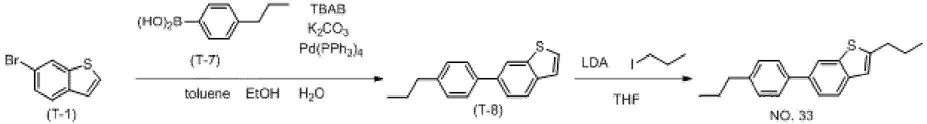

### First step

Compound (T-1) (1.5g) and compound (T-7) (1.5g) prepared according to a publicly known method were dissolved in toluene, and water, ethanol, Pd(PPh₃)₄ (0.8 g), TBAB (0.22 g) and potassium carbonate (2.9 g) were added thereto, and the resulting mixture was refluxed under heating for 6 hours. After completion of reaction, the resulting material was extracted with toluene, and washed with water, and then dried over anhydrous magnesium sulfate, and concentrated under reduced pressure to obtain a pale brown solid. The solid was formed into a solution, and applied to silica gel column chromatography (heptane) to obtain compound (T-8) (1.7 g) as a colorless crystal.

### Second step

Under a nitrogen atmosphere, compound (T-8) (1.7 g) and THF (100 mL) were put in a reaction vessel, and the resulting mixture was cooled down to -74°C. Lithium diisopropylamide (1 M; n-hexane solution; 7.4 mL) was added dropwise thereto in the temperature range of -74°C to -70°C, and the resulting mixture was further stirred for 2 hours. Subsequently, a THF (10 mL) solution of iodine propane (1.6g) was added dropwise thereto in the temperature range of -75°C to -70°C, and the resulting mixture was stirred for 8 hours while returning to 25°C. The resulting reaction mixture was poured into water, subjected to extraction with toluene, and washed with water, and then dried over anhydrous magnesium sulfate, and concentrated under reduced pressure to obtain a pale brown solid. The resulting solid was applied to silica gel column chromatography (heptane : toluene = 3 : 1 in a volume ratio) and recrystallized (ethanol) to obtain compound (No. 33) (0.7 g) as a colorless crystal.

1H-NMR (δ ppm; CDCl3):7.95 (s, 1H), 7.69 (d, 1H), 7.55 (d, 2H), 7.53 (dd, 1H), 7.25 (d, 2H), 7.00 (s, 1H), 2.88 (t, 2H), 2.63 (t, 2H), 1.78 (sex, 2H), 1.68 (sex, 2H), 1.00 (t, 3H), 0.98 (t, 3H).

Physical properties of compound (No. 33) were as described below. Transition temperature: C 27.4 N 33.4 I.T_{NI} = 45.0°C; Δn = 0.184; Δε = 6.1; η = 36.9 mPa·s.

### Comparative Example

Compound (1) was compared with a compound similar thereto from a viewpoint of physical properties.

### Comparative Example 1

Compound (S-1) in which alkyl chain length of compound (B) disclosed in Patent literature No.1 was different was prepared.

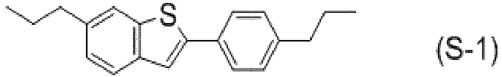

### Synthesis of compound (S-1)

(S-1) was prepared by using (T-7) described below in place of (T-4) in Example 1.

¹H-NMR (δ ppm; CDCl₃): 7.65 (d, 1H), 7.61 (s, 1H), 7.60 (d, 2H), 7.45 (s, 1H), 7.21 (d, 2H), 7.16 (dd, 1H), 2.69 (t, 2H), 2.61 (t, 2H), 1.67 (sex, 2H), 1.66 (sex, 2H), 0.97 (t, 3H), 0.96 (t, 3H).

Physical properties of compound (S-1) were as described below. Transition temperature: C 130.3 I.T_{NI} = 74.6°C; Δn = 0.277; Δε = 0.34; η = 41.4 mPa·s.

When compound (S-1) was compared with compound (No. 43) of the invention, compound (No. 43) of the invention showed suppression of large increase of the viscosity while a higher maximum temperature was showed (T_{NI} = 190.3°C; 60.3 mPa·s), and larger optical anisotropy (Δn = 0.377). Moreover, when compound (S-1) was compared with compound (No. 33) of the invention, compound (No. 33) of the invention showed smaller viscosity (η = 36.9 mPa·s).

### Comparative Example 2

Compound (S-2) in which alkyl chain length of compound (C) disclosed in Patent literature No. 1 was different was prepared.

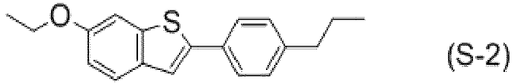

### Synthesis of compound (S-2)

(S-2) was prepared by using (T-7) described below in place of (T-4) in Example 2.

Physical properties of compound (S-2) were as described below. Transition temperature: C 148.8 I.T_{NI} = 124.3°C; Δn = 0.267; Δε = 0.54; η = 51.3 mPa·s.

When compound (S-1) was compared with compound (No. 56) of the invention, compound (No. 56) of the invention showed suppression of large increase of the viscosity while a higher maximum temperature was showed (T_{NI} = 227.3°C; 71.3 mPa·s), and larger optical anisotropy (Δn = 0.427). Moreover, when compound (S-1) was compared with compound (No. 9) of the invention, compound (No. 9) of the invention showed larger optical anisotropy (Δn = 0.387).

### Example 5

Compounds (No. 1) to (No. 200) described below can be prepared according to the synthesis method of compound (1) and synthesis procedures described in Examples 1 to 4.

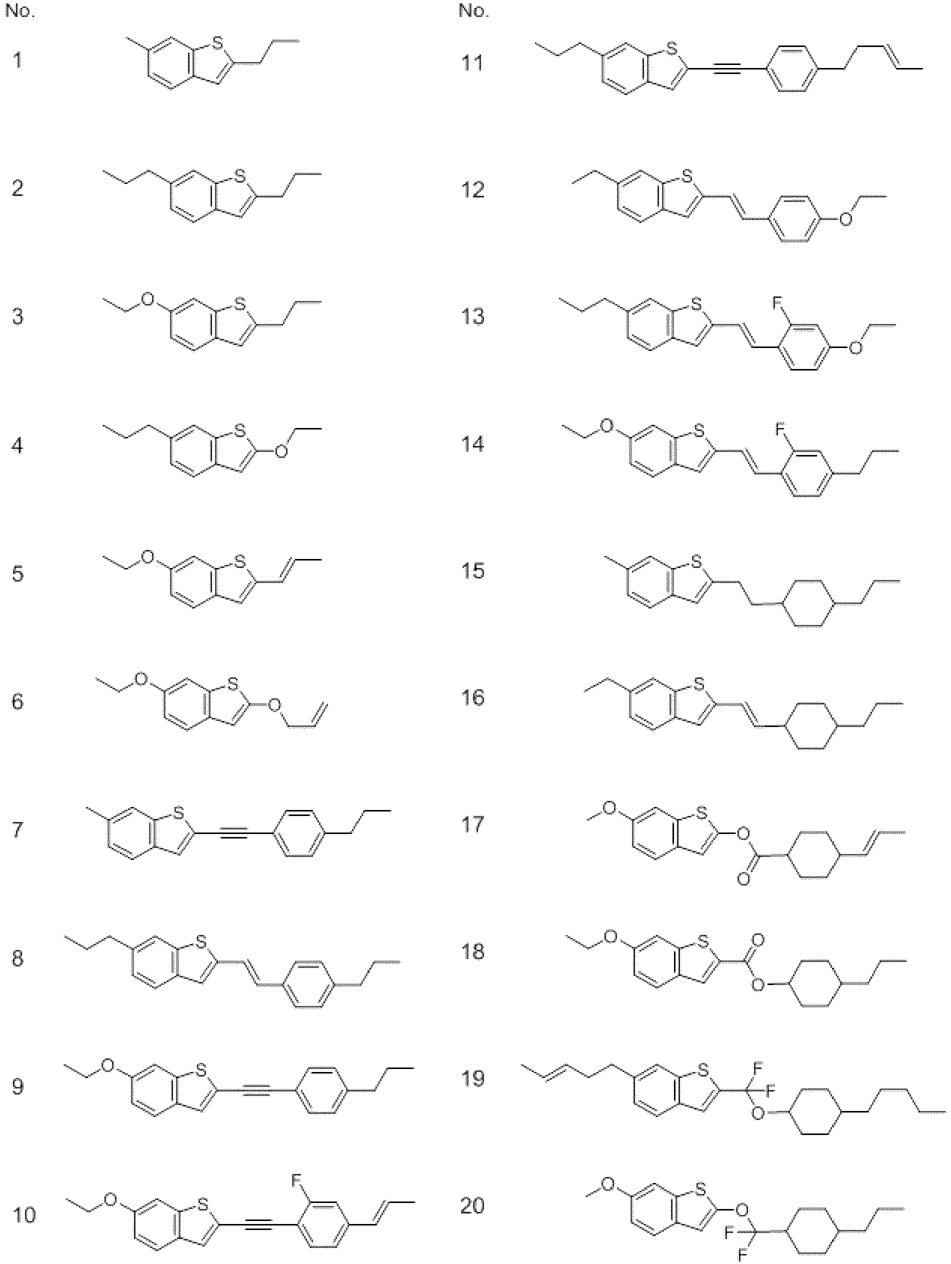

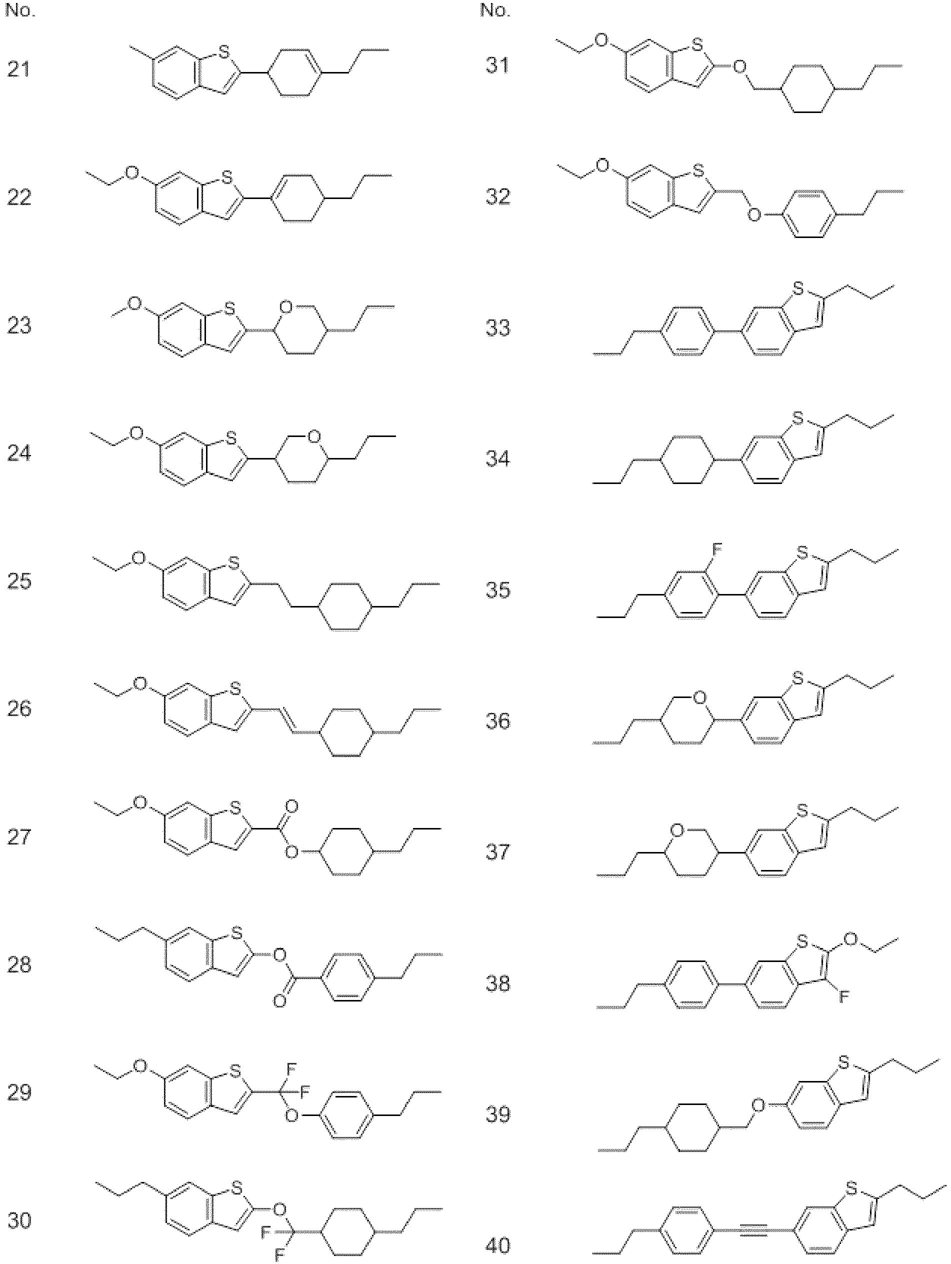

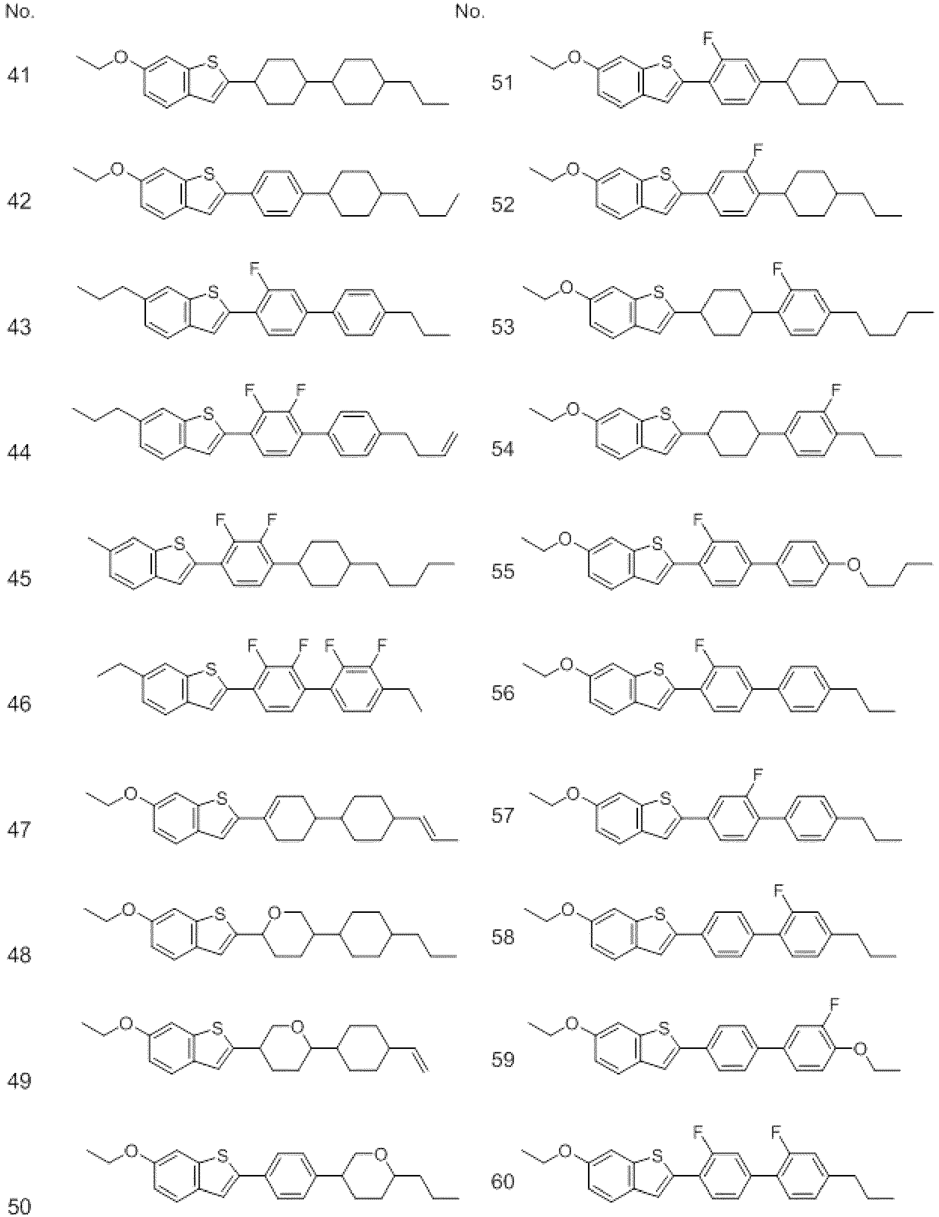

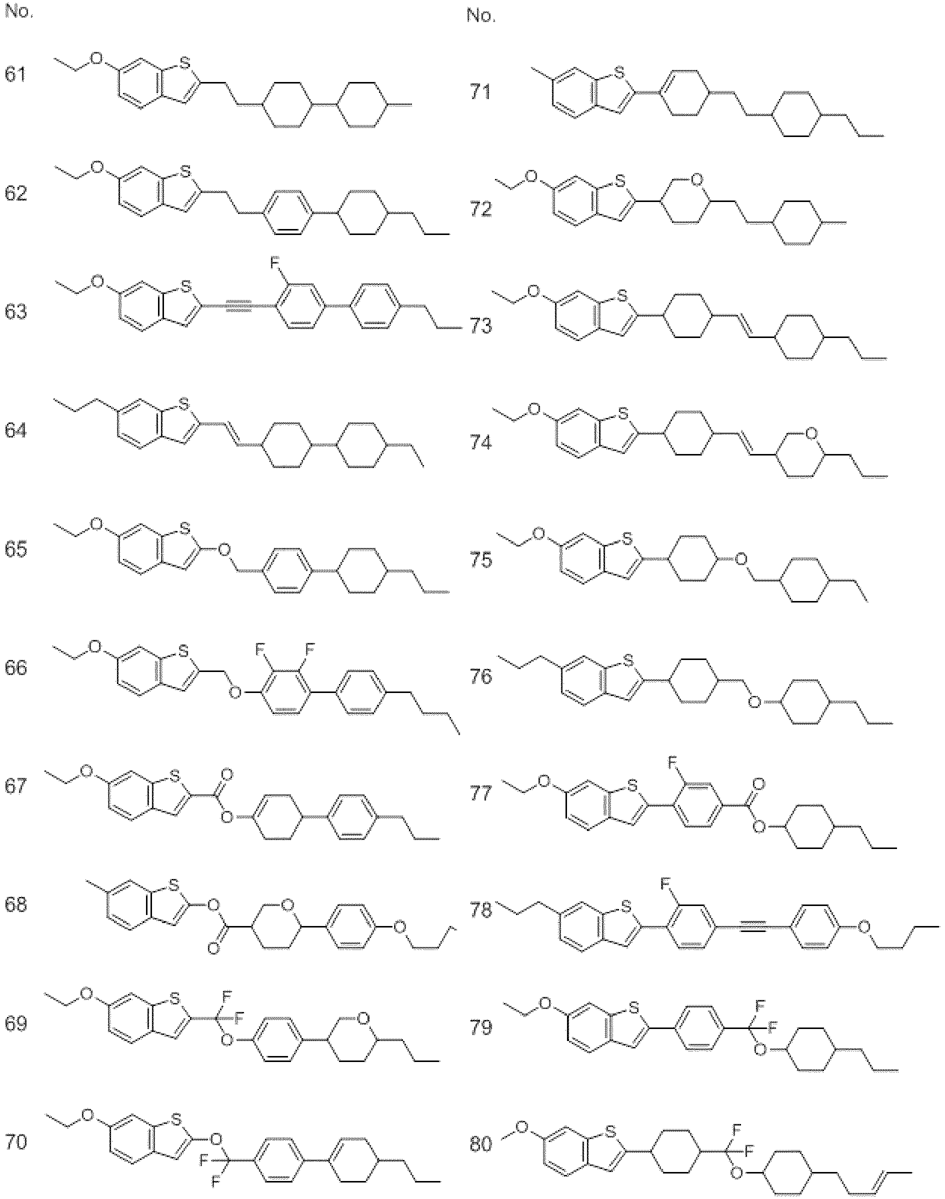

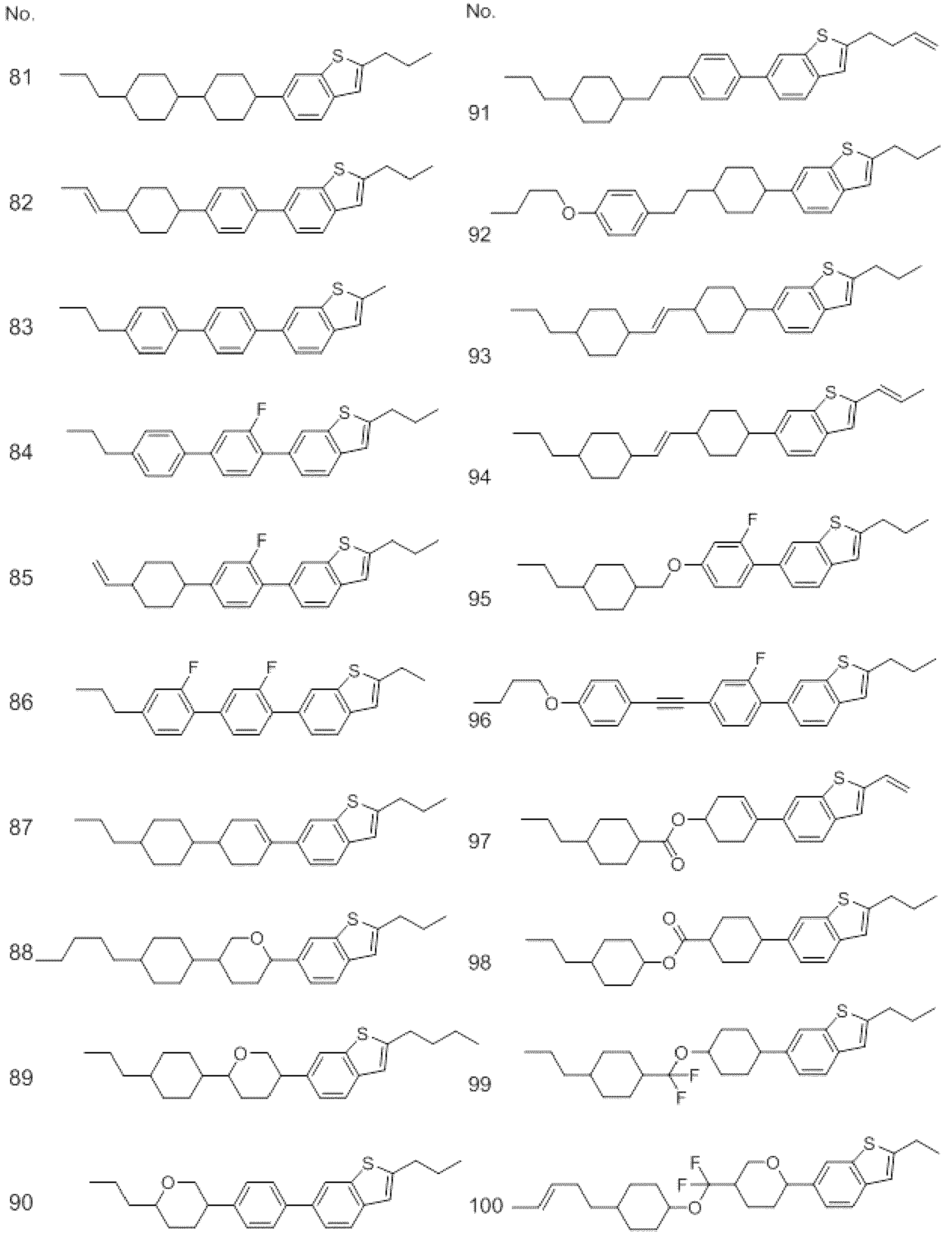

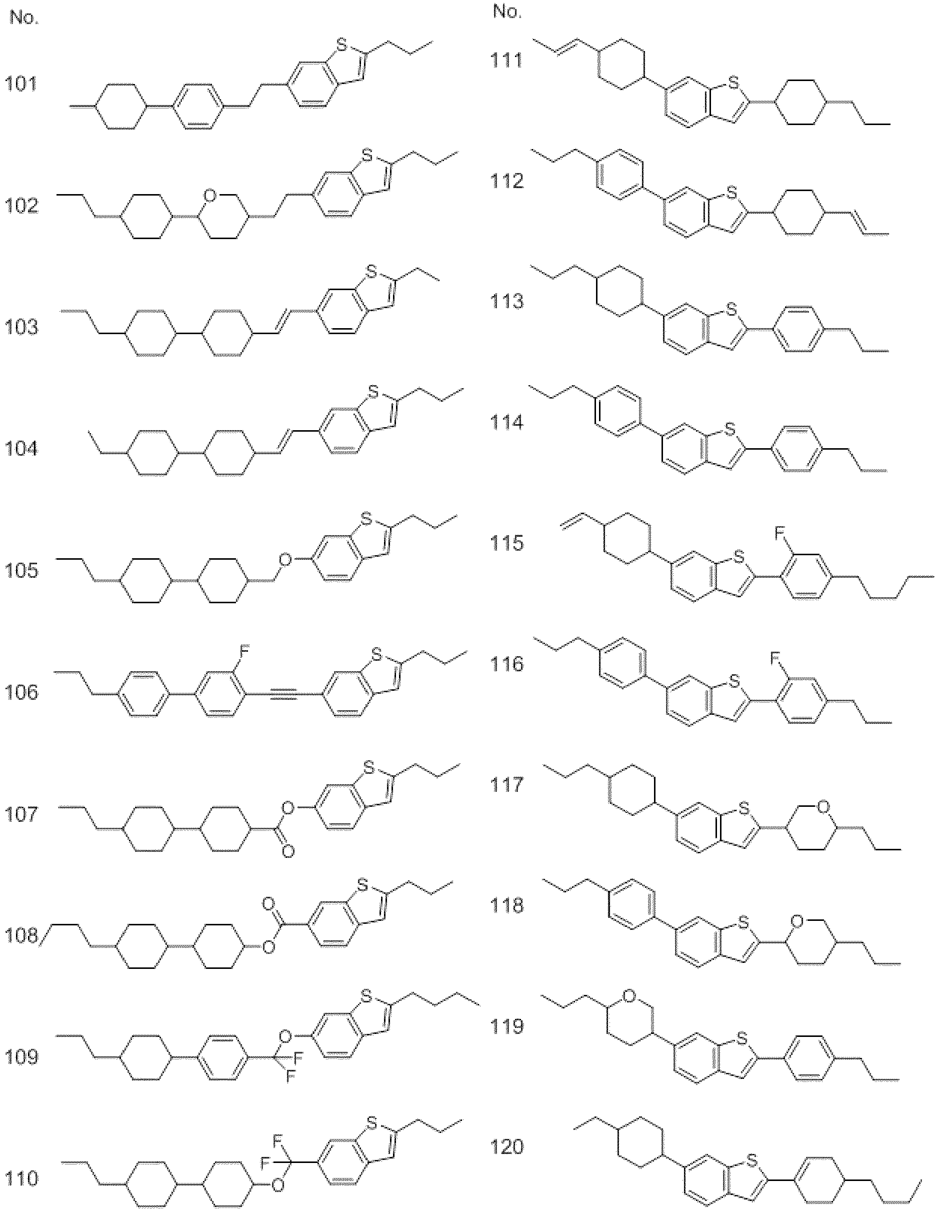

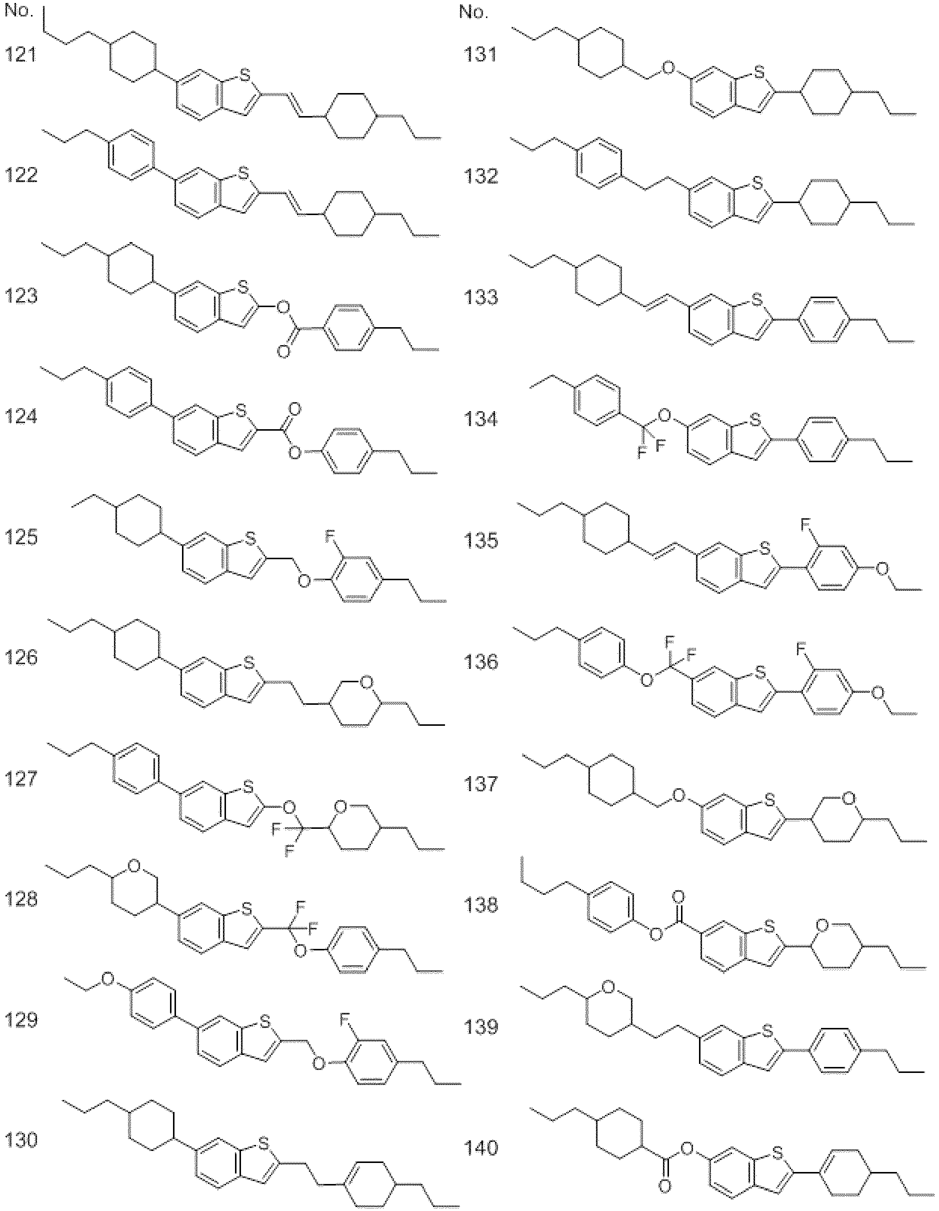

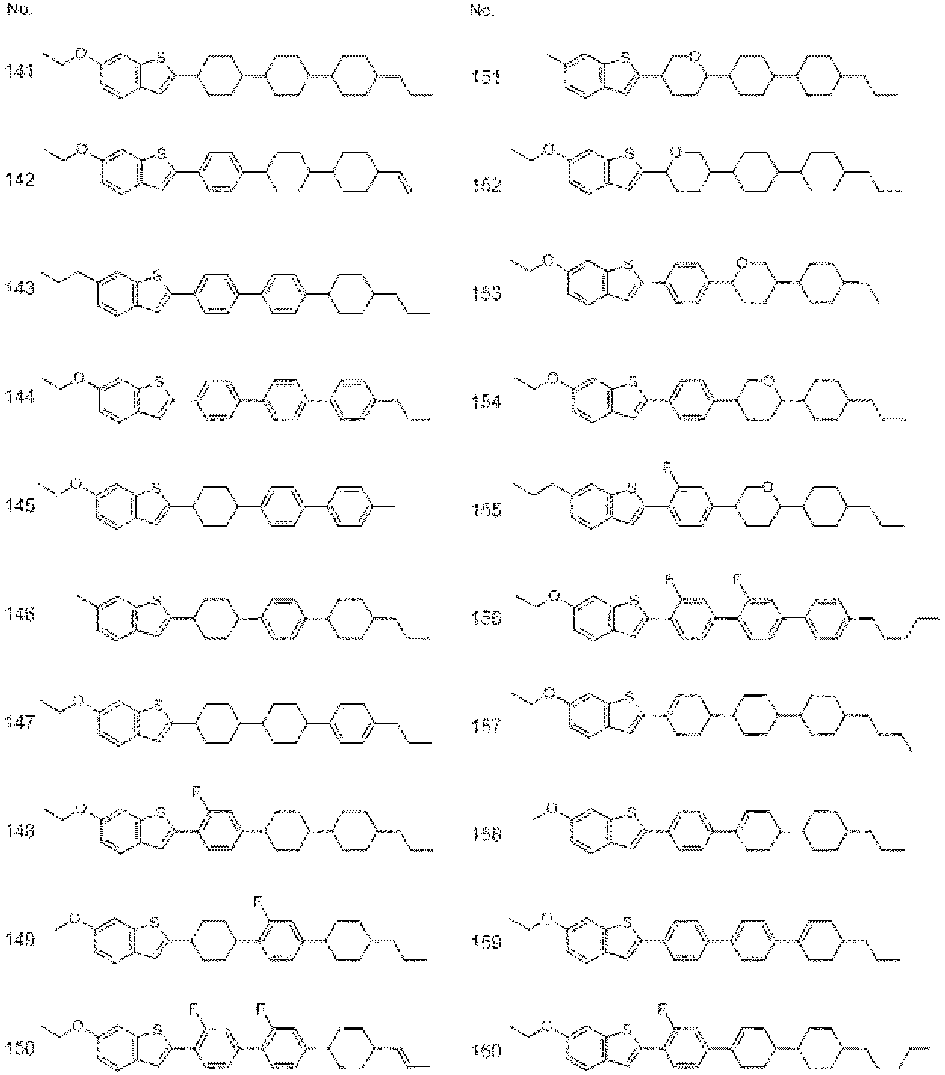

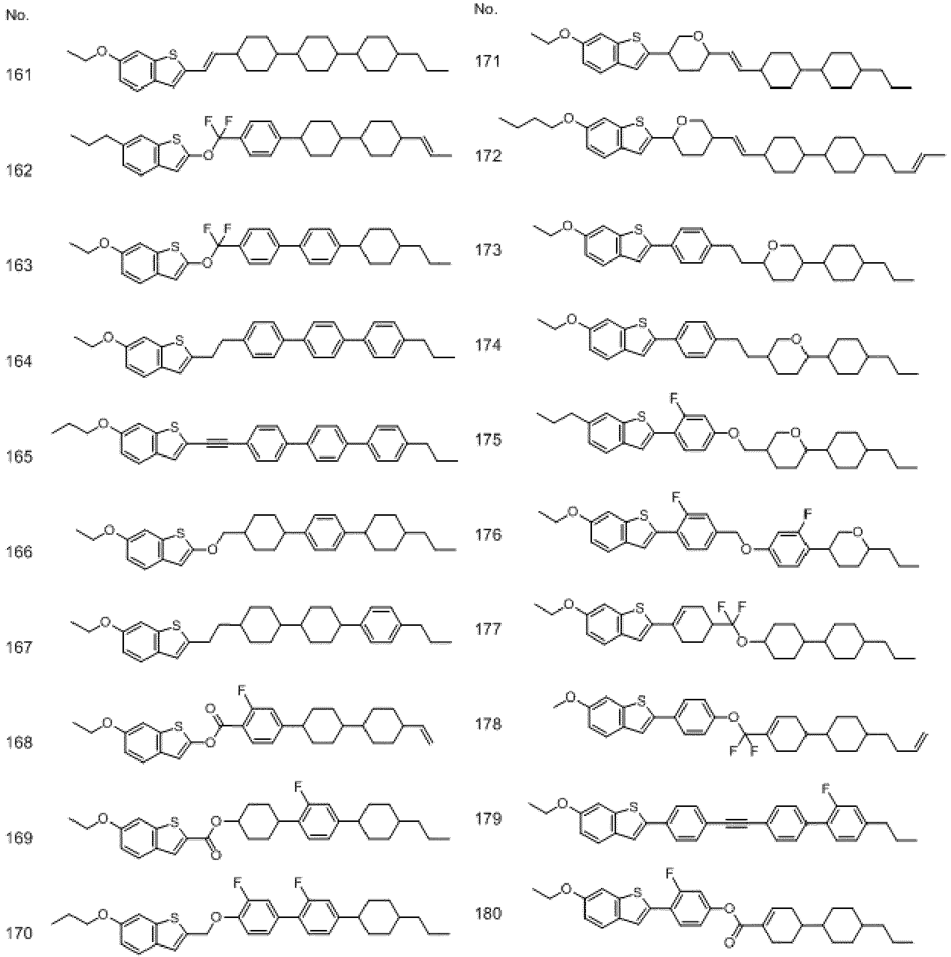

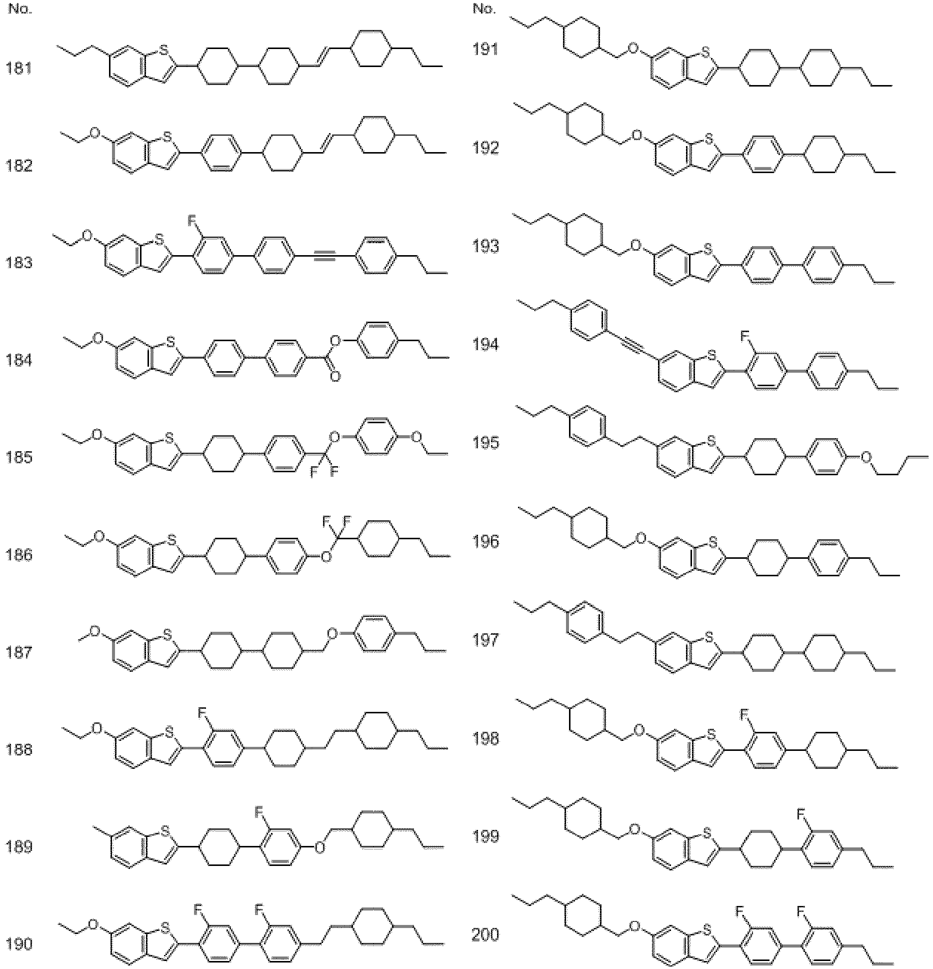

### 2. Examples of composition

The composition of the invention will be described in detail by way of Examples. The invention includes a mixture of a composition in Use Example 1 and a composition in Use Example 2. The invention also includes a mixture of at least two of compositions in Use Examples. The compounds in the Use Examples were represented using symbols according to definitions in Table 1 described below. In Table 1, the configuration of 1,4-cyclohexylene is trans. A parenthesized number next to a symbolized compound in the Use Examples corresponds to the number of the compound. A symbol (-) means any other liquid crystal compound. A proportion (percentage) of the liquid crystal compound is expressed in terms of weight percent (% by weight) based on the weight of the liquid crystal composition. Values of the physical properties of the composition are summarized in a last part. The physical properties were measured according to the methods described above, and measured values are directly described (without extrapolation).

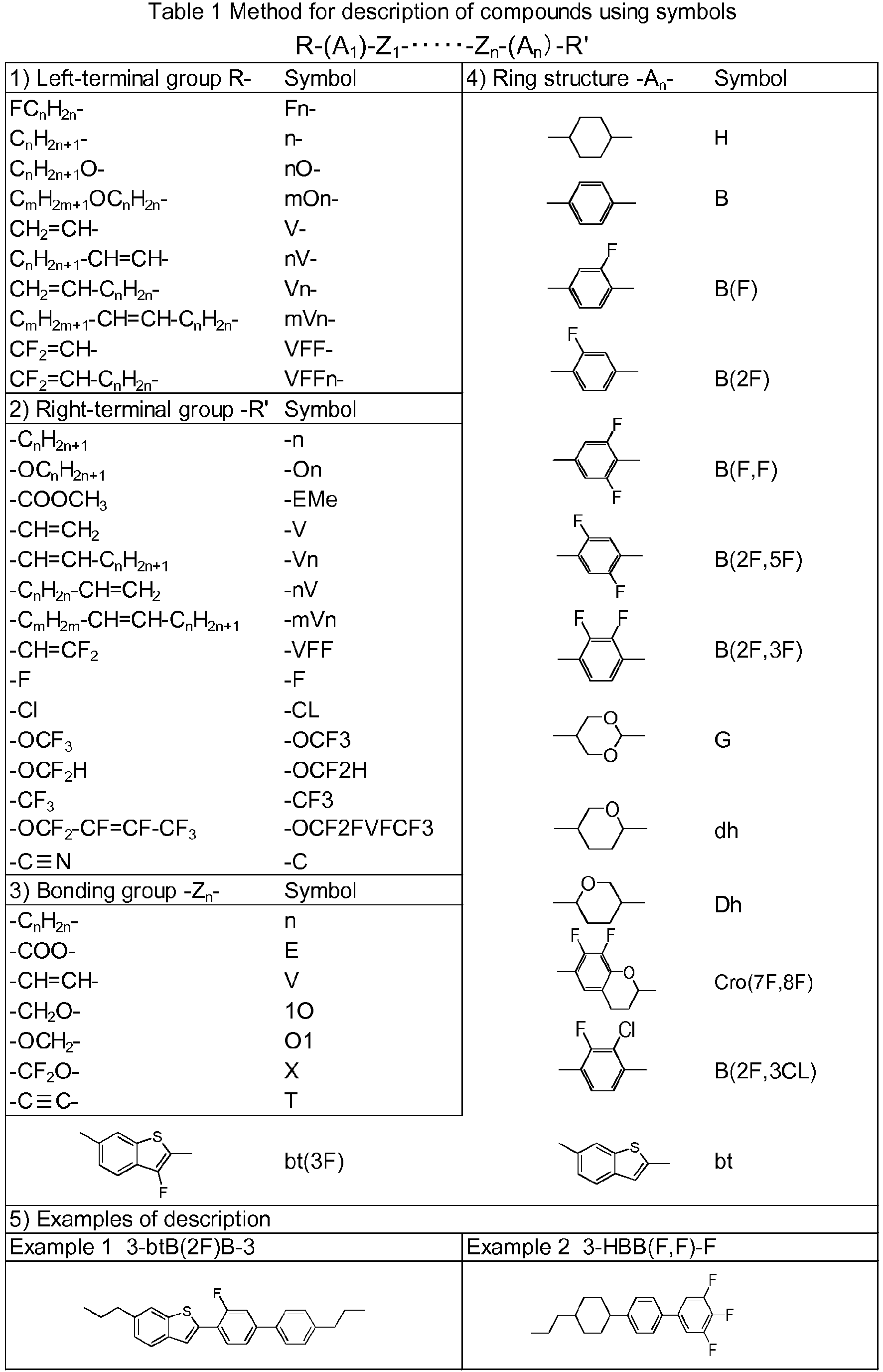

### Use Example 1

| | | |
|---|---|---|
| 3-btB(2F)B-3 | (No. 43) | 3% |
| 2O-btB(2F)B-3 | (No. 56) | 3% |
| 2-HB-C | (8-1) | 5% |
| 3-HB-C | (8-1) | 12% |
| 3-HB-O2 | (2-5) | 15% |
| 2-BTB-1 | (2-10) | 4% |
| 3-HHB-F | (6-1) | 4% |
| 3-HHB-1 | (3-1) | 5% |
| 3-HHB-O1 | (3-1) | 5% |
| 3-HHB-3 | (3-1) | 12% |
| 3-HHEB-F | (6-10) | 3% |
| 5-HHEB-F | (6-10) | 3% |
| 2-HHB(F)-F | (6-2) | 7% |
| 3-HHB(F)-F | (6-2) | 7% |
| 5-HHB(F)-F | (6-2) | 7% |
| 3-HHB(F,F)-F | (6-3) | 5% |

NI = 99.7°C; η = 23.1 mPa·s; Δn = 0.116; Δε = 4.9.

### Use Example 2

| | | |
|---|---|---|
| 2O-btTB-3 | (No. 9) | 5% |
| 3-HB-CL | (5-2) | 12% |
| 3-HH-4 | (2-1) | 12% |
| 3-HB-O2 | (2-5) | 7% |
| 3-HHB(F,F)-F | (6-3) | 3% |
| 3-HBB(F,F)-F | (6-24) | 30% |
| 5-HBB(F,F)-F | (6-24) | 23% |
| 5-HBB(F)B-2 | (4-5) | 4% |
| 5-HBB(F)B-3 | (4-5) | 4% |

NI = 70.0°C; η = 20.2 mPa·s; Δn = 0.125; Δε = 5.5.

### Use Example 3

| | | |
|---|---|---|
| 3-Bbt-3 | (No. 33) | 5% |
| 7-HB(F,F)-F | (5-4) | 3% |
| 3-HB-O2 | (2-5) | 7% |
| 2-HHB(F)-F | (6-2) | 10% |
| 3-HHB(F)-F | (6-2) | 10% |
| 5-HHB(F)-F | (6-2) | 10% |
| 2-HBB(F)-F | (6-23) | 8% |
| 3-HBB(F)-F | (6-23) | 6% |
| 5-HBB(F)-F | (6-23) | 16% |
| 2-HBB-F | (6-22) | 4% |
| 3-HBB-F | (6-22) | 4% |
| 5-HBB-F | (6-22) | 3% |
| 3-HBB(F,F)-F | (6-24) | 4% |
| 5-HBB(F,F)-F | (6-24) | 10% |

NI = 84.0°C; η = 25.0 mPa·s; Δn = 0.118; Δε = 5.6.

### Use Example 4

| | | |
|---|---|---|
| 3-btB(2F)TB-O4 | (No. 78) | 3% |
| 5-HB-CL | (5-2) | 16% |
| 3-HH-4 | (2-1) | 12% |
| 3-HH-5 | (2-1) | 4% |
| 3-HHB-F | (6-1) | 4% |
| 3-HHB-CL | (6-1) | 3% |
| 4-HHB-CL | (6-1) | 4% |
| 3-HHB(F)-F | (6-2) | 9% |
| 4-HHB(F)-F | (6-2) | 8% |
| 5-HHB(F)-F | (6-2) | 9% |
| 7-HHB(F)-F | (6-2) | 7% |
| 5-HBB(F)-F | (6-23) | 4% |
| 1O1-HBBH-5 | (4-1) | 3% |
| 3-HHBB(F,F)-F | (7-6) | 2% |
| 4-HHBB(F,F)-F | (7-6) | 3% |
| 5-HHBB(F,F)-F | (7-6) | 3% |
| 3-HH2BB(F,F)-F | (7-15) | 3% |
| 4-HH2BB(F,F)-F | (7-15) | 3% |

### Use Example 5

| | | |
|---|---|---|
| 2O-btB(2F)B-O4 | (No. 55) | 3% |
| 3-HHB(F,F)-F | (6-3) | 9% |
| 3-H2HB(F,F)-F | (6-15) | 8% |
| 4-H2HB(F,F)-F | (6-15) | 8% |
| 5-H2HB(F,F)-F | (6-15) | 8% |
| 3-HBB(F,F)-F | (6-24) | 21% |
| 5-HBB(F,F)-F | (6-24) | 17% |
| 3-H2BB(F,F)-F | (6-27) | 10% |
| 5-HHBB(F,F)-F | (7-6) | 3% |
| 5-HHEBB-F | (7-17) | 2% |
| 3-HH2BB(F,F)-F | (7-15) | 3% |
| 1O1-HBBH-4 | (4-1) | 4% |
| 1O1-HBBH-5 | (4-1) | 4% |

### Use Example 6

| | | |
|---|---|---|
| 4O-BTB(F)bt-3 | (No. 96) | 3% |
| 3-BB(F)bt-3 | (No. 84) | 3% |
| 5-HB-F | (5-2) | 10% |
| 6-HB-F | (5-2) | 9% |
| 7-HB-F | (5-2) | 6% |
| 2-HHB-OCF3 | (6-1) | 7% |
| 3-HHB-OCF3 | (6-1) | 5% |
| 4-HHB-OCF3 | (6-1) | 7% |
| 5-HHB-OCF3 | (6-1) | 5% |
| 3-HH2B-OCF3 | (6-4) | 4% |
| 5-HH2B-OCF3 | (6-4) | 3% |
| 3-HHB(F,F)-OCF2H | (6-3) | 4% |
| 3-HHB(F,F)-OCF3 | (6-3) | 5% |
| 3-HH2B(F)-F | (6-5) | 3% |
| 3-HBB(F)-F | (6-23) | 10% |
| 5-HBB(F)-F | (6-23) | 10% |
| 5-HBBH-3 | (4-1) | 3% |
| 3-HB(F)BH-3 | (4-2) | 3% |

### Use Example 7

| | | |
|---|---|---|
| 3-Bbt(3F)-O2 | (No. 38) | 5% |
| 2O-btB(2F)B(2F)B-5 | (No. 156) | 1 % |
| 5-HB-CL | (5-2) | 11% |
| 3-HH-4 | (2-1) | 7% |
| 3-HHB-1 | (3-1) | 4% |
| 3-HHB(F,F)-F | (6-3) | 7% |
| 3-HBB(F,F)-F | (6-24) | 20% |
| 5-HBB(F,F)-F | (6-24) | 14% |
| 3-HHEB(F,F)-F | (6-12) | 10% |
| 4-HHEB(F,F)-F | (6-12) | 3% |
| 5-HHEB(F,F)-F | (6-12) | 4% |
| 2-HBEB(F,F)-F | (6-39) | 3% |
| 3-HBEB(F,F)-F | (6-39) | 4% |
| 5-HBEB(F,F)-F | (6-39) | 3% |
| 3-HHBB(F,F)-F | (7-6) | 4% |

### Use Example 8

| | | |
|---|---|---|
| 3-btB(2F)B-3 | (No. 43) | 3% |
| 2O-btTB-3 | (No. 9) | 4% |
| 3-HB-CL | (5-2) | 5% |
| 5-HB-CL | (5-2) | 4% |
| 3-HHB-OCF3 | (6-1) | 4% |
| 3-H2HB-OCF3 | (6-13) | 5% |
| 5-H4HB-OCF3 | (6-19) | 15% |
| V-HHB(F)-F | (6-2) | 4% |
| 3-HHB(F)-F | (6-2) | 4% |
| 5-HHB(F)-F | (6-2) | 4% |
| 3-H4HB(F,F)-CF3 | (6-21) | 8% |
| 5-H4HB(F,F)-CF3 | (6-21) | 10% |
| 5-H2HB(F,F)-F | (6-15) | 5% |
| 5-H4HB(F,F)-F | (6-21) | 6% |
| 2-H2BB(F)-F | (6-26) | 5% |
| 3-H2BB(F)-F | (6-26) | 9% |
| 3-HBEB(F,F)-F | (6-39) | 5% |

NI = 76.3°C; η = 30.9 mPa·s; Δn = 0.117; Δε = 8.5.

### Use Example 9

| | | |
|---|---|---|
| 20-btB (2F)B-3 | (No. 56) | 3% |
| 5-HB-CL | (5-2) | 17% |
| 7-HB(F,F)-F | (5-4) | 3% |
| 3-HH-4 | (2-1) | 10% |
| 3-HH-5 | (2-1) | 5% |
| 3-HB-O2 | (2-5) | 15% |
| 3-HHB-1 | (3-1) | 8% |
| 3-HHB-O1 | (3-1) | 4% |
| 2-HHB(F)-F | (6-2) | 7% |
| 3-HHB(F)-F | (6-2) | 7% |
| 5-HHB(F)-F | (6-2) | 7% |
| 3-HHB(F,F)-F | (6-3) | 6% |
| 3-H2HB(F,F)-F | (6-15) | 4% |
| 4-H2HB(F,F)-F | (6-15) | 4% |

NI = 74.0°C; η = 16.2 mPa·s; Δn = 0.083; Δε = 2.9.

### Use Example 10

| | | |
|---|---|---|
| 3-Bbt-3 | (No. 33) | 5% |
| 3-btB(2F)TB-O4 | (No. 78) | 3% |
| 5-HB-CL | (5-2) | 3% |
| 7-HB(F)-F | (5-3) | 7% |
| 3-HH-4 | (2-1) | 9% |
| 3-HH-5 | (2-1) | 10% |
| 3-HB-O2 | (2-5) | 13% |
| 3-HHEB-F | (6-10) | 8% |
| 5-HHEB-F | (6-10) | 5% |
| 3-HHEB(F,F)-F | (6-12) | 8% |
| 4-HHEB(F,F)-F | (6-12) | 3% |
| 3-GHB(F,F)-F | (6-109) | 4% |
| 4-GHB(F,F)-F | (6-109) | 6% |
| 5-GHB(F,F)-F | (6-109) | 7% |
| 2-HHB(F,F)-F | (6-3) | 4% |
| 3-HHB(F,F)-F | (6-3) | 5% |

### Use Example 11

| | | |
|---|---|---|
| 2O-btB(2F)B-O4 | (No. 55) | 3% |
| 3-HB-O1 | (2-5) | 15% |
| 3-HH-4 | (2-1) | 5% |
| 3-HB(2F,3F)-O2 | (9-1) | 12% |
| 5-HB(2F,3F)-O2 | (9-1) | 12% |
| 2-HHB(2F,3F)-1 | (10-1) | 11% |
| 3-HHB(2F,3F)-1 | (10-1) | 10% |
| 3-HHB(2F,3F)-O2 | (10-1) | 13% |
| 5-HHB(2F,3F)-O2 | (10-1) | 13% |
| 3-HHB-1 | (3-1) | 6% |

### Use Example 12

| | | |
|---|---|---|
| 3-Bbt(3F)-O2 | (No. 38) | 5% |
| 2-HH-5 | (2-1) | 3% |
| 3-HH-4 | (2-1) | 15% |
| 3-HH-5 | (2-1) | 4% |
| 3-HB-O2 | (2-5) | 12% |
| 3-H2B(2F,3F) -O2 | (9-4) | 13% |
| 5-H2B(2F,3F)-O2 | (9-4) | 13% |
| 3-HHB(2F,3CL)-O2 | (10-12) | 5% |
| 2-HBB(2F,3F)-O2 | (10-7) | 3% |
| 3-HBB(2F,3F)-O2 | (10-7) | 9% |
| 5-HBB(2F,3F)-O2 | (10-7) | 9% |
| 3-HHB-1 | (3-1) | 3% |
| 3-HHB-3 | (3-1) | 3% |
| 3-HHB-O1 | (3-1) | 3% |

### Use Example 13

| | | |
|---|---|---|
| 3-BB(F)bt-3 | (No. 84) | 3% |
| 2-HH-3 | (2-1) | 21% |
| 3-HH-4 | (2-1) | 9% |
| 1-BB-3 | (2-8) | 9% |
| 3-HB-O2 | (2-5) | 2% |
| 3-BB(2F,3F)-O2 | (9-3) | 9% |
| 5-BB(2F,3F)-O2 | (9-3) | 5% |
| 2-HH1OB(2F,3F)-O2 | (10-5) | 12% |
| 3-HH1OB(2F,3F)-O2 | (10-5) | 21% |
| 3-HHB-1 | (3-1) | 4% |
| 3-HHB-O1 | (3-1) | 3% |
| 2-BBB(2F)-5 | (3-8) | 2% |

### Use Example 14

| | | |
|---|---|---|
| 4O-BTB(F)bt-3 | (No. 96) | 3% |
| 2O-btB(2F)BN(2F)B-5 | (No. 156) | 1 % |
| 2-HH-3 | (2-1) | 16% |
| 7-HB-1 | (2-5) | 10% |
| 5-HB-O2 | (2-5) | 8 % |
| 3-HB(2F,3F)-O2 | (9-1) | 17% |
| 5-HB(2F,3F)-O2 | (9-1) | 16% |
| 3-HHB(2F,3CL)-O2 | (10-12) | 3% |
| 4-HHB(2F,3CL)-O2 | (10-12) | 3% |
| 3-HH1OCro(7F,8F)-5 | (13-6) | 4% |
| 5-HBB(F)B-2 | (4-5) | 9% |
| 5-HBB(F)B-3 | (4-5) | 10% |

### Use Example 15

| | | |
|---|---|---|
| 3-btB(2F)B-3 | (No. 43) | 3% |
| 1-BB-3 | (2-8) | 10% |
| 3-HH-V | (2-1) | 29% |
| 3-BB(2F,3F)-O2 | (9-3) | 13% |
| 2-HH1OB(2F,3F)-O2 | (10-5) | 20% |
| 3-HH1OB(2F,3F)-O2 | (10-5) | 14% |
| 3-HHB-1 | (3-1) | 5% |
| 2-BBB(2F)-5 | (3-8) | 6% |

NI = 75.5°C; η = 16.0 mPa·s; Δn = 0.114; Δε = -3.0.

### Use Example 16

| | | |
|---|---|---|
| 2O-btB(2F)B-3 | (No. 56) | 3% |
| 2-HH-3 | (2-1) | 6% |
| 3-HH-V1 | (2-1) | 10% |
| 1V2-HH-1 | (2-1) | 8% |
| 1V2-HH-3 | (2-1) | 7% |
| 3-BB(2F,3F)-O2 | (9-3) | 8% |
| 5-BB(2F,3F)-O2 | (9-3) | 4% |
| 3-H1OB(2F,3F)-O2 | (9-5) | 7% |
| 2-HH1OB(2F,3F)-O2 | (10-5) | 8% |
| 3-HH1OB(2F,3F)-O2 | (10-5) | 18% |
| 3-HDhB(2F,3F)-O2 | (10-3) | 5% |
| 3-HHB-1 | (3-1) | 3% |
| 3-HHB-3 | (3-1) | 2% |
| 2-BB(2F,3F)B-3 | (11-1) | 11% |

NI = 87.9°C; η = 21.8 mPa·s; Δn = 0.118; Δε = -4.2.

### Use Example 17

| | | |
|---|---|---|
| 2O-btTB-3 | (No. 9) | 5% |
| 1V2-BEB(F,F)-C | (8-15) | 5% |
| 3-HB-C | (8-1) | 18% |
| 2-BTB-1 | (2-10) | 10% |
| 5-HH-VFF | (2-1) | 30% |
| 3-HHB-1 | (3-1) | 4% |
| VFF-HHB-1 | (3-1) | 8% |
| VFF2-HHB-1 | (3-1) | 10% |
| 3-H2BTB-2 | (3-17) | 4% |
| 3-H2BTB-3 | (3-17) | 3% |
| 3-H2BTB-4 | (3-17) | 3% |

NI = 79.8°C; η = 13.0 mPa·s; Δn = 0.136; Δε = 6.0.

### Use Example 18

| | | |
|---|---|---|
| 3-Bbt-3 | (No. 33) | 5% |
| 5-HB(F)B(F,F)XB(F,F)-F | (7-41) | 5% |
| 3-BB(F)B(F,F)XB(F,F)-F | (7-47) | 3% |
| 4-BB(F)B(F,F)XB(F,F)-F | (7-47) | 7% |
| 5-BB(F)B(F,F)XB(F,F)-F | (7-47) | 3% |
| 3-HH-V | (2-1) | 41% |
| 3-HH-V1 | (2-1) | 7% |
| 3-HHEH-5 | (3-13) | 3% |
| 3-HHB-1 | (3-1) | 3% |
| V-HHB-1 | (3-1) | 3% |
| V2-BB(F)B-1 | (3-6) | 5% |
| 1V2-BB-F | (5-1) | 3% |
| 3-BB(F,F)XB(F,F)-F | (6-97) | 10% |
| 3-HHBB(F,F)-F | (7-6) | 2% |

NI = 77.7°C; η = 12.2 mPa·s; Δn = 0.108; Δε = 6.1.

### Use Example 19

| | | |
|---|---|---|
| 3-btB(2F)TB-O4 | (No. 78) | 3% |
| 3-GB(F)B(F,F)XB(F,F)-F | (7-57) | 5% |
| 3-BB(F)B(F,F)XB(F,F)-F | (7-47) | 3% |
| 4-BB(F)B(F,F)XB(F,F)-F | (7-47) | 7% |
| 5-BB(F)B(F,F)XB(F,F)-F | (7-47) | 3% |
| 3-HH-V | (2-1) | 41% |
| 3-HH-V1 | (2-1) | 6% |
| 3-HHEH-5 | (3-13) | 3% |
| 3-HHB-1 | (3-1) | 4% |
| V-HHB-1 | (3-1) | 5% |
| V2-BB(F)B-1 | (3-6) | 5% |
| 1V2-BB-F | (5-1) | 3% |
| 3-BB(F,F)XB(F,F)-F | (6-97) | 5% |
| 3-GB(F,F)XB(F,F)-F | (6-113) | 4% |
| 3-HHBB(F,F)-F | (7-6) | 3% |

### Industrial Applicability

A liquid crystal compound of the invention satisfies at least one of physical properties such as high stability to heat or light, a high clearing point (or high maximum temperature), low minimum temperature of a liquid crystal phase, small viscosity, suitable optical anisotropy, large dielectric anisotropy, a suitable elastic constant and excellent compatibility with other liquid crystal compounds. The compound particularly has the excellent compatibility with other liquid crystal compounds. A liquid crystal composition contains the compound and satisfies at least one of physical properties such as high maximum temperature, low minimum temperature, small viscosity, suitable optical anisotropy, large dielectric anisotropy, large specific resistance and a suitable elastic constant. The composition has a suitable balance regarding at least two of the physical properties. A liquid crystal display device includes the composition and has a wide temperature range in which the device can be used, a short response time, a large voltage holding ratio, low threshold voltage, a large contrast ratio and a long service life. Accordingly, the composition can be widely applied to a liquid crystal display device used in a personal computer, a television and so forth.

## Claims

1. A compound, represented by formula (1): 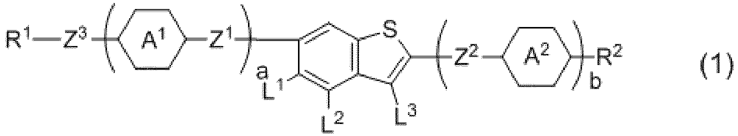 wherein, in formula (1),
R¹ and R² are independently hydrogen or alkyl having 1 to 20 carbons, and in the alkyl, at least one piece of -CH₂- may be replaced by -O- or -S-, and at least one piece of - (CH₂)₂- may be replaced by -CH=CH-, and in the groups, at least one hydrogen may be replaced by halogen;
ring A¹ and ring A² are independently 1,4-cyclohexylene, 1,4-cyclohexenylene, 1,4-phenylene, tetrahydropyran-2,5-diyl, 1,3-dioxane-2,5-diyl, pyrimidine-2,5-diyl, 2,6,7-trioxabicyclo[2.2.2]octane-1,4-diyl, naphthalene-2,6-diyl or pyridine-2,5-diyl, and at least one hydrogen on the rings may be replaced by halogen;
a and b are independently 0, 1, 2, 3 or 4, and a sum of a and b is 4 or less, and when a or b is 2 or more, two of ring A¹, two of ring A², two pieces of Z¹ or two pieces of Z² may be identical or different;
Z¹ and Z² are independently a single bond or alkylene having 1 to 4 carbons, and in the alkylene, at least one piece of -CH₂- may be replaced by -O- or -COO-, and at least one piece of -(CH₂)₂- may be replaced by -CH=CH- or -C=C-, and in the groups, at least one hydrogen may be replaced by halogen, and a case where Z¹ and Z² are simultaneously either -CH=CH- or -C=C- is excluded, and when a is 0 and b is 1, Z² is alkylene having 1 to 4 carbons, and in the alkylene, at least one piece of -CH₂- may be replaced by -O- or -COO-, at least one piece of - (CH₂)₂- may be replaced by -CH=CH- or -C=C-, and at least one hydrogen may be replaced by halogen;
Z³ is -O- or a single bond; and
L¹, L² and L³ are independently hydrogen, fluorine or chlorine.

2. The compound according to claim 1, wherein, in formula (1),
R¹ and R² are independently hydrogen, alkyl having 1 to 10 carbons, alkoxy having 1 to 9 carbons, alkenyl having 2 to 10 carbons or fluorinated alkyl having 1 to 10 carbons;
ring A¹ and ring A² are independently 1,4-cyclohexylene, 1,4-cyclohexenylene, 1,4-phenylene in which at least one hydrogen may be replaced by halogen, or tetrahydropyran-2,5-diyl;
Z¹ and Z² are independently a single bond, - (CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or -(CH₂)₂-CH=CH-, and when a is 0 and b is 1, Z² is -(CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or - (CH₂)₂-CH=CH-;
a and b are independently 0, 1, 2, 3 or 4, and a sum of a and b is 4 or less; and
L¹, L² and L³ are independently hydrogen, fluorine or chlorine.

3. The compound according to claim 1, wherein, in formula (1),
a sum of a and b is 0, 1, 2 or 3;
R¹ and R² are independently hydrogen, alkyl having 1 to 10 carbons, alkoxy having 1 to 9 carbons, alkenyl having 2 to 10 carbons, or alkyl having 1 to 10 carbons in which at least one hydrogen is replaced by fluorine;
ring A¹ and ring A² are independently 1,4-cyclohexylene, 1,4-cyclohexenylene, 1,4-phenylene in which at least one hydrogen may be replaced by halogen, or tetrahydropyran-2,5-diyl;
Z¹ and Z² are independently a single bond, - (CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or -(CH₂)₂-CH=CH-, and when a is 0 and b is 1, Z² is -(CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or - (CH₂)₂-CH=CH-; and
L¹, L² and L³ are independently hydrogen, fluorine or chlorine.

4. The compound according to any one of claims 1 to 3, represented by any one of formulas (1-1) to (1-10): 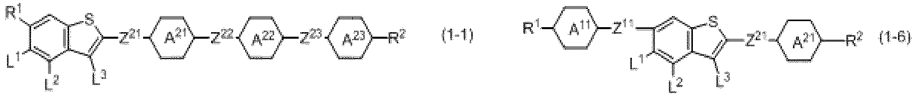 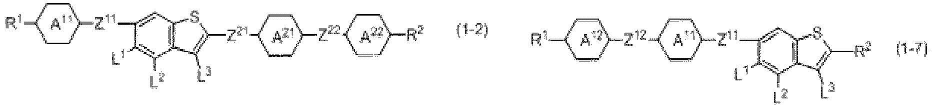 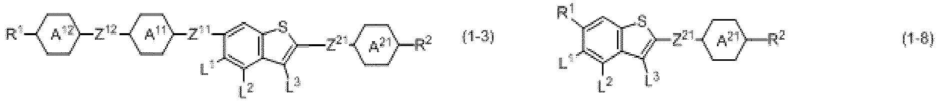 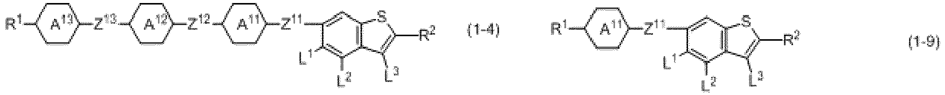 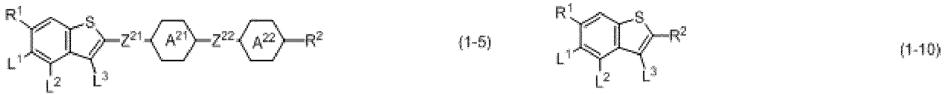 wherein, in formulas (1-1) to (1-7) and (1-9) to (1-10),
R¹ and R² are independently hydrogen, alkyl having 1 to 10 carbons, alkoxy having 1 to 9 carbons, alkenyl having 2 to 10 carbons or fluorinated alkyl having 1 to 10 carbons;
ring A¹¹, ring A¹², ring A¹³, ring A²¹, ring A²² and ring A²³ are independently 1,4-cyclohexylene, 1,4-cyclohexenylene, 1,4-phenylene, 1,4-phenylene in which at least one hydrogen is replaced by halogen, or tetrahydropyran-2,5-diyl;
Z¹¹, Z¹², Z¹³, Z²¹, Z²² and Z²³ are independently a single bond, -(CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or -(CH₂)₂-CH=CH-; and
L¹, L² and L³ are independently hydrogen, fluorine or chlorine; and in formula (1-8),
R¹ and R² are independently hydrogen, alkyl having 1 to 10 carbons, alkoxy having 1 to 9 carbons, alkenyl having 2 to 10 carbons, or alkyl having 1 to 10 carbons in which at least one hydrogen is replaced by fluorine;
ring A²¹ is 1,4-cyclohexylene, 1,4-cyclohexenylene, 1,4-phenylene, 1,4-phenylene in which at least one hydrogen is replaced by halogen, or tetrahydropyran-2,5-diyl;
Z²¹ is independently -(CH₂)₂-, -CH=CH-, -CF=CF-, -C≡C-, -COO-, -OCO-, -CF₂O-, -OCF₂-, -CH₂O-, -OCH₂-, -(CH₂)₄-, -(CH₂)₂CF₂O-, -(CH₂)₂OCF₂-, -CF₂O(CH₂)₂-, -OCF₂(CH₂)₂-, -CH=CH-(CH₂)₂- or -(CH₂)₂-CH=CH-; and
L¹, L² and L³ are independently hydrogen, fluorine or chlorine.

5. The compound according to claim 4, represented by any one of formulas (1-1) to (1-10), wherein, in formulas (1-1) to (1-7) and (1-9) to (1-10),
R¹ and R² are independently hydrogen, fluorine, alkyl having 1 to 10 carbons, alkoxy having 1 to 9 carbons, alkenyl having 2 to 10 carbons, or alkyl having 1 to 10 carbons in which at least one hydrogen is replaced by fluorine;
ring A¹¹, ring A¹², ring A¹³, ring A²¹, ring A²² and ring A²³ are independently 1,4-cyclohexylene, 1,4-cyclohexenylene, 1,4-phenylene in which at least one hydrogen may be replaced by halogen, or tetrahydropyran-2,5-diyl;
Z¹¹, Z¹², Z¹³, Z²¹, Z²² and Z²³ are independently a single bond, -(CH₂)₂-, -CH=CH-, -C≡C-, -CF₂O-, -OCF₂-, -CH₂O- or -OCH₂-; and
L¹, L² and L³ are independently hydrogen, fluorine or chlorine; and in formula (1-8),
R¹ and R² are independently hydrogen, fluorine, alkyl having 1 to 10 carbons, alkoxy having 1 to 9 carbons, alkenyl having 2 to 10 carbons or fluorinated alkyl having 1 to 10 carbons;
ring A¹¹, ring A¹², ring A¹³, ring A²¹, ring A²² and ring A²³ are independently 1,4-cyclohexylene, 1,4-cyclohexenylene, 1,4-phenylene in which at least one hydrogen may be replaced by halogen, or tetrahydropyran-2,5-diyl;
Z¹¹, Z¹², Z¹³, Z²¹, Z²² and Z²³ are independently -(CH₂)₂-, -CH=CH-, -C≡C-, -CF₂O-, -OCF₂-, -CH₂O- or -OCH₂-; and
L¹, L² and L³ are independently hydrogen, fluorine or chlorine.

6. The compound according to claim 5, represented by any one of formulas (1-1), (1-5) and (1-10) : 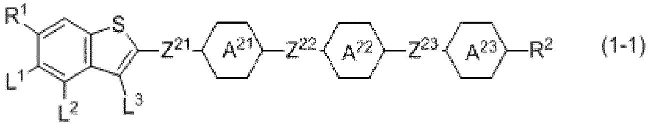 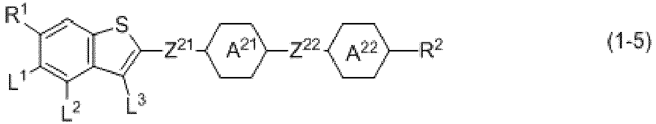 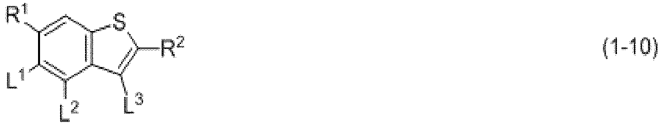 wherein, in formulas (1-1), (1-5) and (1-10),
R¹ and R² are independently hydrogen, fluorine, alkyl having 1 to 10 carbons, alkoxy having 1 to 9 carbons, alkenyl having 2 to 10 carbons, or fluorinated alkyl having 1 to 10 carbons in which at least one hydrogen is by fluorine;
ring A²¹, ring A²² and ring A²³ are independently 1, 4-cyclohexylene, or 1,4-phenylene in which at least one hydrogen may be replaced by halogen;
Z²¹, Z²² and Z²³ are independently a single bond, - (CH₂)₂-, -CH=CH-, -C≡C-, -CH₂O- or -OCH₂-; and
L¹, L² and L³ are independently hydrogen or fluorine.

7. The compound according to claim 5, represented by formula (1-8) : 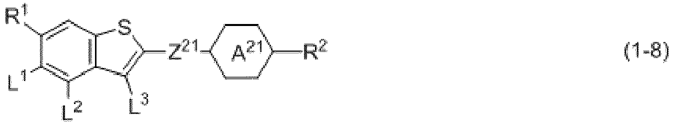 wherein, in formula (1-8),
R¹ and R² are independently hydrogen, fluorine, alkyl having 1 to 10 carbons, alkoxy having 1 to 9 carbons, alkenyl having 2 to 10 carbons or fluorinated alkyl having 1 to 10 carbons;
ring A²¹ is 1, 4-cyclohexylene, or 1, 4-phenylene in which at least one hydrogen may be replaced by halogen;
Z²¹ is a single bond, -(CH₂)₂-, -CH=CH-, -C≡C-, -CH₂O- or -OCH₂-; and
L¹, L² and L³ are independently hydrogen or fluorine.

8. The compound according to claim 5, represented by any one of formulas (1-4), (1-7) and (1-9): 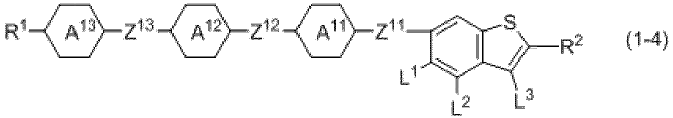 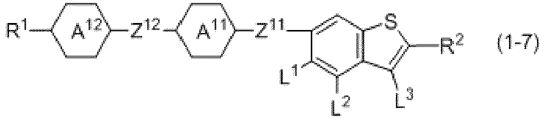 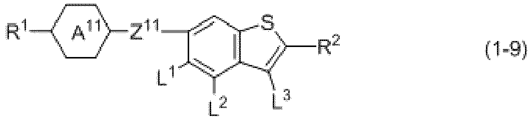 wherein, in formulas (1-4), (1-7) and (1-9),
R¹ and R² are independently hydrogen, fluorine, alkyl having 1 to 10 carbons, alkoxy having 1 to 9 carbons, alkenyl having 2 to 10 carbons or fluorinated alkyl having 1 to 10 carbons;
ring A¹¹, ring A¹² and ring A¹³ are independently 1, 4-cyclohexylene, or 1,4-phenylene in which at least one hydrogen may be replaced by halogen;
Z¹¹, Z¹² and Z¹³ are independently a single bond, -(CH₂)₂-, -CH=CH-, -C≡C-, -CH₂O- or -OCH₂-; and
L¹, L² and L³ are independently hydrogen or fluorine.

9. A liquid crystal composition, containing at least one compound according to any one of claims 1 to 8.

10. The liquid crystal composition according to claim 9, further containing at least one compound selected from the group of compounds represented by formulas (2) to (4): 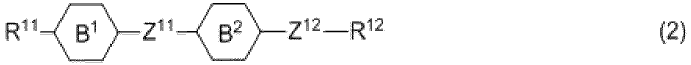 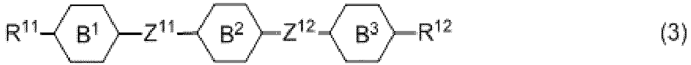 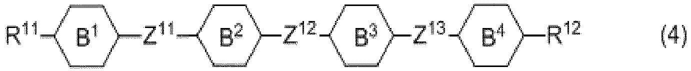 wherein, in formulas (2) to (4),
R¹¹ and R¹² are independently alkyl having 1 to 10 carbons or alkenyl having 2 to 10 carbons, and in the alkyl or the alkenyl, at least one piece of -CH₂- may be replaced by -O-, and at least one hydrogen may be replaced by fluorine;
ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
Z¹¹, Z¹² and Z¹³ are independently a single bond, -CH₂CH₂-, -CH=CH-, -C≡C- or -COO-.

11. The liquid crystal composition according to claim 9 or 10, further containing at least one compound selected from the group of compounds represented by formulas (5) to (7): 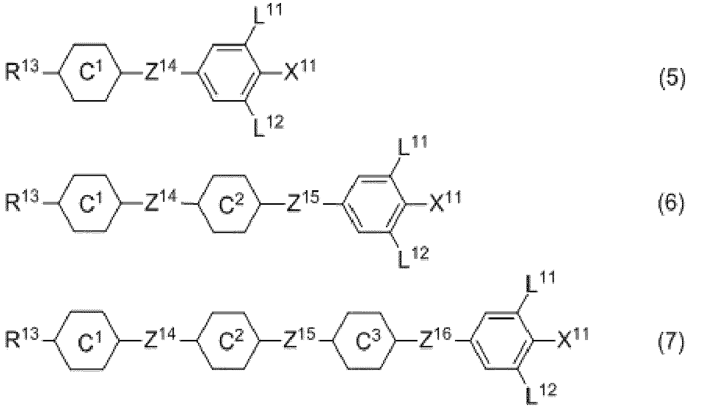 wherein, in formulas (5) to (7),
R¹³ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine;
X¹¹ is fluorine, chlorine, -OCF₃, -OCHF₂, -CF₃, -CHF₂, -CH₂F, -OCF₂CHF₂ or -OCF₂CHFCF₃;
ring C¹, ring C² and ring C³ are independently 1,4-cyclohexylene, 1, 4-phenylene in which at least one hydrogen may be replaced by fluorine, tetrahydropyran-2,5-diyl, 1,3-dioxane-2,5-diyl, or pyrimidine-2,5-diyl;
Z¹⁴, Z¹⁵ and Z¹⁶ are independently a single bond, -CH₂CH₂-, -CH=CH-, -C=C-, -COO-, -CF₂O-, -OCF₂-, -CH₂O- or -(CH₂)₄-; and
L¹¹ and L¹² are independently hydrogen or fluorine.

12. The liquid crystal composition according to any one of claims 9 to 11, further containing at least one compound selected from the group of compounds represented by formula (8): 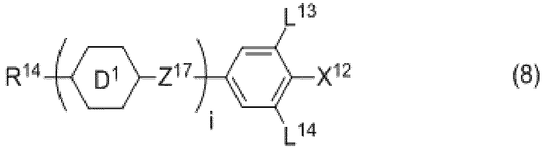 wherein, in formula (8),
R¹⁴ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine;
X¹² is -C≡N or -C≡C-C≡N;
ring D¹ is 1,4-cyclohexylene, 1,4-phenylene in which at least one hydrogen may be replaced by fluorine, tetrahydropyran-2,5-diyl, 1,3-dioxane-2,5-diyl, or pyrimidine-2,5-diyl;
Z¹⁷ is a single bond, -CH₂CH₂-, -C≡C-, -COO-, -CF₂O-, -OCF₂- or -CH₂O- ;
L¹³ and L¹⁴ are independently hydrogen or fluorine; and
i is 1, 2, 3 or 4.

13. The liquid crystal composition according to any one of claims 9 to 12, further containing at least one compound selected from the group of compounds represented by formulas (9) to (15): 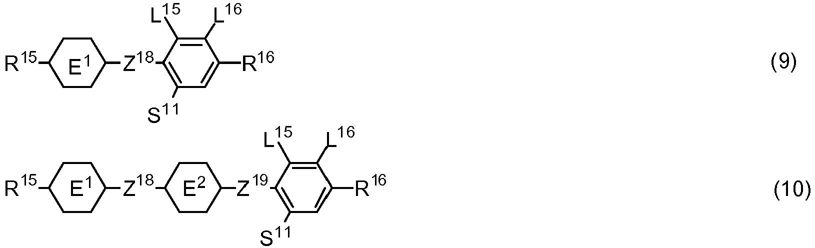 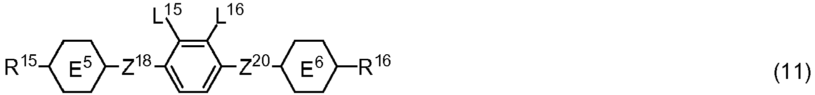 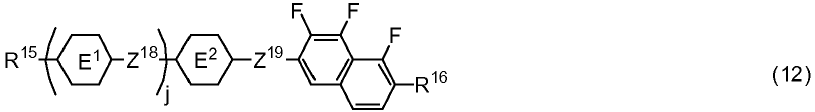 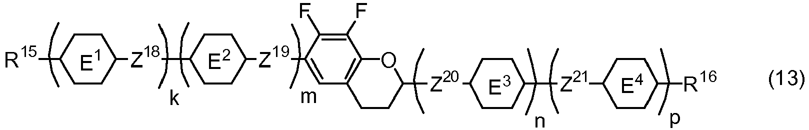 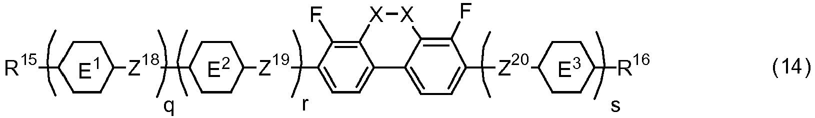 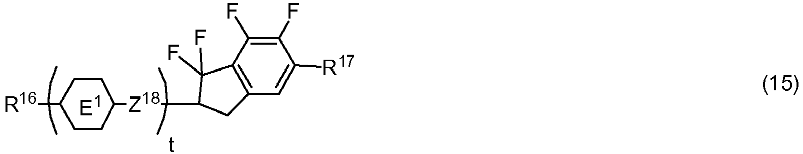 wherein, in formulas (9) to (15),
R¹⁵ and R¹⁶ are independently alkyl having 1 to 10 carbons or alkenyl having 2 to 10 carbons, and in the alkyl and the alkenyl, at least one piece of -CH₂- may be replaced by -O-, and at least one hydrogen may be replaced by fluorine;
R¹⁷ is hydrogen, fluorine, alkyl having 1 to 10 carbons or alkenyl having 2 to 10 carbons, and in the alkyl and the alkenyl, at least one piece of -CH₂- may be replaced by -O-, and at least one hydrogen may be replaced by fluorine;
ring E¹, ring E², ring E³ and ring E⁴ are independently 1,4-cyclohexylene, 1,4-cyclohexenylene, 1,4-phenylene in which at least one hydrogen may be replaced by fluorine, tetrahydropyran-2,5-diyl, or decahydronaphthalene-2,6-diyl, and ring E⁵ and ring E⁶ are independently 1,4-cyclohexylene, 1,4-cyclohexenylene, 1,4-phenylene, tetrahydropyran-2,5-diyl or decahydronaphthalene-2,6-diyl;
Z¹⁸, Z¹⁹, Z²⁰ and Z²¹ are independently a single bond, -CH₂CH₂-, -COO-, -CH₂O-, -OCF₂- or -OCF₂CH₂CH₂-;
L¹⁵ and L¹⁶ are independently fluorine or chlorine;
S¹¹ is hydrogen or methyl;
X is -CHF- or -CF₂-; and
j, k, m, n, p, q, r and s are independently 0 or 1, a sum of k, m, n and p is 1 or 2, a sum of q, r and s is 0, 1, 2 or 3, and t is 1, 2 or 3.

14. The liquid crystal composition according to any one of claims 9 to 13, further containing at least one of a polymerizable compound, a polymerization initiator, a polymerization inhibitor, an optically active compound, an antioxidant, an ultraviolet light absorber, a light stabilizer, a heat stabilizer, a dye and an antifoaming agent.

15. A liquid crystal display device, including the liquid crystal composition according to any one of claims 9 to 14.

16. The liquid crystal display device according to claim 15, wherein the liquid crystal composition according to any one of claims 9 to 14 is encapsulated.

17. The liquid crystal display device according to claim 15, wherein the liquid crystal composition according to any one of claims 9 to 14 is used for switching between 2D and 3D.
